# EUROPEAN PATENT SPECIFICATION

(11) **EP 0 876 491 B1**
(45) Date of publication and mention of the grant of the patent: **08.04.2009**
(21) Application number: 96946233.2
(22) Date of filing: 20.12.1996
(51) Int. Cl.: C12N 15/63, C12N 5/00, C12N 15/00, A01N 43/04, A61K 31/70

(54) **A LONG QT SYNDROME GENE WHICH ENCODES KVLQT1 AND ITS ASSOCIATION WITH minK**
EIN GEN DES "LANGEN QT SYNDROMS" (LONG QT SYNDROM), WELCHES KVLQT1 KODIERT UND SEINE WECHSELWIRKUNG MIT MINK
GENE DU SYNDROME DU Q-T LONG CODANT KVLQT1 ET SON ASSOCIATION AVEC minK

(30) Priority: 22.12.1995 US 19014 P; 29.10.1996 US 739383
(43) Date of publication of application: 11.11.1998
(73) Proprietor: University of Utah Research Foundation, Salt Lake City, Utah 84108 (US); Genzyme Genetics, Framingham, MA 01701 (US)
(72) Inventor: KEATING, Mark, F., Salt Lake City, UT 84103 (US); CURRAN, Mark, E., Hopkinton, MA 01748 (US); LANDES, Gregory, M., Northboro, MA 01532 (US); CONNORS, Timothy, D., Hopkinton, MA 01748 (US); BURN, Timothy, C., Hockessin, DE 19707 (US); WANG, Qing, Shaker Heights, Ohio 44120 (US)
(74) Representative: Dörries, Hans Ulrich
(86) International application number: PCT/US1996/019917
(87) International publication number: WO 1997/023632

(56) References cited:
- US-A- 5 599 673
- BARHANIN J ET AL: "KVLQT1 AND ISK (MINK) PROTEINS ASSOCIATE TO FORM TH IKS CARDIAC POTASSIUM CURRENT" NATURE, MACMILLAN JOURNALS LTD. LONDON, GB, vol. 384, 7 November 1996 (1996-11-07), pages 78-80, XP002910425 ISSN: 0028-0836
- NATURE, 07 November 1996, Vol. 384, SANGUINETTI et al., "Coassembly of K(V)LQT1 and MinK (IsK) Proteins to Form Cardiac I(Ks) Potassium Channel", pages 80-83, XP002916128
- NATURE GENETICS, January 1996, Vol. 12, WANG et al., "Positional Cloning of a Novel Potassium Channel Gene: KVLQT1 Mutations Cause Cardiac Arrhythmias", pages 17-23, XP002916129
- NATURE, 07 November 1996, Vol. 384, ATTALI, "Ion Channels. A New Wave for Heart Rhythms", pages 24-25, XP002916130
- JOURNAL SUISSE DE MEDECINE, 12 October 1996, Vol. 126, No. 41, PRIORI et al., "The Long QT Syndrome: New Diagnostic and Therapeutic Approach in the Era of Molecular Biology. Schweizerische Medizinisch Wochenschrift", pages 1727-1731, XP000865562

## Description

### BACKGROUND OF THE INVENTION

The present invention describes a gene and gene products associated with long QT syndrome (LQT) and is directed to a process for the assessment of the risk for LQT. LQT is diagnosed by analyzing the DNA sequence of the *KVLQT1* gene of an individual to be tested and comparing the respective DNA sequence to the known DNA sequence of a normal *KVLQT1* gene. Alternatively, the *KVLQT1* gene of an individual to be tested can be screened for mutations which cause LQT. Prediction of LQT will enable practitioners to prevent this disorder using existing medical therapy. Further described is the discovery that the KVLQT1 and minK proteins coassemble to form a cardiac I_{Ks} potassium channel. This knowledge can be used to coexpress these two proteins in a cell and such a transformed cell can be used for screening for drugs which will be useful in treating or preventing LQT.

Cardiac arrhythmias are a common cause of morbidity and mortality, accounting for approximately 11% of all natural deaths (Kannel, 1987; Willich et al., 1987). In general, presymptomatic diagnosis and treatment of individuals with life-threatening ventricular tachyarrhythmias is poor, and in some cases medical management actually increases the risk of arrhythmia and death (New Engl. J. Med. 327, 227 (1992)). These factors make early detection of individuals at risk for cardiac arrhythmias and arrhythmia prevention high priorities.

Both genetic and acquired factors contribute to the risk of developing cardiac arrhythmias. Long QT syndrome (LQT) is an inherited cardiac arrhythmia that causes abrupt loss of consciousness, syncope, seizures and sudden death from ventricular tachyarrhythmias, specifically *torsade de pointes* and ventricular fibrillation (Ward, 1964; Romano, 1965; Schwartz et al., 1975; Moss et al., 1991). This disorder usually occurs in young, otherwise healthy individuals (Ward, 1964; Romano, 1965; Schwartz, 1975). Most LQT gene carriers manifest prolongation of the QT interval on electrocardiograms, a sign of abnormal cardiac repolarization (Vincent et al., 1992). The clinical features of LQT result from episodic cardiac arrhythmias, specifically repolarization-related ventricular tachyarrhythmias like *torsade de pointes,* named for the characteristic undulating nature of the electrocardiogram in this arrhythmia and ventricular fibrillation (Schwartz et al., 1975; Moss and McDonald, 1970). *Torsade de pointes* may degenerate into ventricular fibrillation, a particularly lethal arrhythmia. Although LQT is not a common diagnosis, ventricular arrhythmias are very common; more than 300,000 United States citizens die suddenly every year (Kannel, et al., 1987; Willich et al., 1987) and, in many cases, the underlying mechanism may be aberrant cardiac repolarization. LQT, therefore, provides a unique opportunity to study life-threatening cardiac arrhythmias at the molecular level.

Both inherited and acquired forms of LQT have been defined. Acquired LQT and secondary arrhythmias can result from cardiac ischemia, bradycardia and metabolic abnormalities such as low serum potassium or calcium concentration (Zipes, 1987). LQT can also result from treatment with certain medications, including antibiotics, antihistamines, general anesthetics, and, most commonly, antiarrhythmic medications (Zipes, 1987). Inherited forms of LQT can result from mutations in at least three different genes. In previous studies, LQT loci were mapped to chromosome 11p15.5 (*LQT1*) (Keating et al., 1991a; Keating et al., 1991b), 7q35-36 (*LQT2*) and 3p21-24 (*LQT3*) (Jiang et al., 1994). Of these, the most common cause of inherited LQT is *LQT1*. Our data indicate that mutations in this gene are responsible for more than 50% of inherited LQT (Q. Wang, unpublished results). Recently, a fourth LQT locus (*LQT4*) was mapped to 4q25-27 (Schott et al., 1995). The present work indicates that *minK,* a gene located on chromosome 21, is also involved in LQT.

Autosomal dominant and autosomal recessive forms of this disorder have been reported. Autosomal recessive LQT (also known as Jervell-Lange-Nielson syndrome) has been associated with congenital neural deafness; this form of LQT is rare (Jervell and Lange-Nielson, 1957). Autosomal dominant LQT (Romano-Ward syndrome) is more common, and is not associated with other phenotypic abnormalities. A disorder very similar to inherited LQT can also be acquired, usually as a result of pharmacologic therapy (Schwartz et al., 1975; Zipes, 1987).

The data have implications for the mechanism of arrhythmias in LQT. Two hypotheses for LQT have previously been proposed (Schwartz et al., 1994). One suggests that a predominance of left autonomic innervation causes abnormal cardiac repolarization and arrhythmias. This hypothesis is supported by the finding that arrhythmias can be induced in dogs by removal of the right stellate ganglion. In addition, anecdotal evidence suggests that some LQT patients are effectively treated by β-adrenergic blocking agents and by left stellate ganglionectomy (Schwartz et al., 1994). The second hypothesis for LQT-related arrhythmias suggests that mutations in cardiac-specific ion channel genes, or genes that modulate cardiac ion channels, cause delayed myocellular repolarization. Delayed myocellular repolarization could promote reactivation of L-type calcium channels, resulting in secondary depolarizations (January and Riddle, 1989). These secondary depolarizations are the likely cellular mechanism of *torsade de pointes* arrhythmias (Surawicz, 1989). This hypothesis is supported by the observation that pharmacologic block of potassium channels can induce QT prolongation and repolarization-related arrhythmias in humans and animal models (Antzelevitch and Sicouri, 1994). The discovery that one form of LQT results from mutations in a cardiac potassium channel gene supports the myocellular hypothesis.

In 1991, the complete linkage between autosomal dominant LQT and a polymorphism at HRAS was reported (Keating et al., 1991a; Keating et al., 1991b). This discovery localized *LQT1* to chromosome 11p15.5 and made presymptomatic diagnosis possible in some families. Autosomal dominant LQT was previously thought to be genetically homogeneous, and the first seven families that were studied were linked to 11p15.5 (Keating et al., 1991b). In 1993, it was found that there was locus heterogeneity for LQT (Benhorin et al., 1993; Curran et al., 1993; Towbin et al., 1994). Two additional LQT loci were subsequently identified, *LQT2* on chromosome 7q35-36 (nine families) and *LQT3* on 3p21-24 (three families) (Jiang et al., 1994). Several families remain unlinked to the known loci, indicating additional locus heterogeneity for LQT. This degree of heterogeneity suggests that distinct LQT genes may encode proteins that interact to modulate cardiac repolarization and arrhythmia risk.

Although little is known about the physiology of LQT, the disorder is associated with prolongation of the QT interval on electrocardiograms, a sign of abnormal cardiac repolarization. This association suggests that genes encoding ion channels, or their modulators, are reasonable candidates for LQT. HRAS, which was localized to chromosome 11p15.5, was excluded as a candidate for *LQT1* based on direct DNA sequence analyses (unpublished observations) and by linkage analyses (Roy et al., 1994). A neuroendocrine calcium channel gene (*CACNL1A2 ;* Chin et al., 1991; Seino et al., 1992) and a gene encoding a GTP-binding protein that modulates potassium channels (*GNAI2;* Weinstein et al., 1988; Magovcevic et al., 1992) became candidates for *LQT3* based on their chromosomal location. Subsequent linkage analyses, however, have excluded these genes (Wang and Keating, unpublished data). A skeletal muscle chloride channel (*CLCN1* ; Koch et al., 1992) and a cardiac muscarinic-acetylcholine receptor (*CHRM2*; Bonner et al., 1987) became candidates for *LQT2* based on their chromosome 7q35-36 location, but subsequent linkage analyses have excluded these genes (Wang et al., submitted).

In theory, mutations in a cardiac sodium channel gene could cause LQT. Voltage-gated sodium channels mediate rapid depolarization in ventricular myocytes, and also conduct a small current during the plateau phase of the action potential (Attwell et al., 1979). Subtle abnormalities of sodium channel function (e.g., delayed sodium channel inactivation or altered voltage-dependence of channel inactivation) could delay cardiac repolarization, leading to QT prolongation and arrhythmias. In 1992, Gellens and colleagues cloned and characterized a cardiac sodium channel gene, *SCN5A* (Gellens et al., 1992). The structure of this gene was similar to other, previously characterized sodium channels, encoding a large protein of 2016 amino acids. These channel proteins contain four homologous domains (DI-DIV), each of which contains six putative membrane spanning segments (S1-S6). *SCN5A* was recently mapped to chromosome 3p21, making it an excellent candidate gene for *LQT3* (George et al., 1995), and this gene was then proved to be associated with LQT3 (Wang et al., 1995a).

In 1994, Warmke and Ganetzky identified a novel human cDNA, human ether a-go-go related gene (*HERG,* Warmke and Ganetzky, 1994). *HERG* was localized to human chromosome 7 by PCR analysis of a somatic cell hybrid panel (Warmke and Ganetzky, 1994) making it a candidate for LQT2. The function of the protein encoded by *HERG* is not known, but it has predicted amino acid sequence homology to potassium channels. *HERG* was isolated from a hippocampal cDNA library by homology to the *Drosophila* ether a-go-go gene (*eag*), which encodes a calcium-modulated potassium channel (Bruggeman et al., 1993). *HERG* is not the human homolog of *eag*, however, sharing only ∼50% amino acid sequence homology. *HERG* has been shown to be associated with LQT2 (Curran et al., 1995).

A novel potassium channel gene has now been discovered which is named *KVLQT1*. Evidence is presented here indicating that *KVLQT1* is *LQT1*. Sixteen families with mutations in *KVLQT1* were identified and characterized and it was shown that in all sixteen families there was complete linkage between *LQT1* and *KVLQT1. KVLQT1* was mapped to chromosome 11p15.5 making it a candidate gene for *LQT1. KVLQT1* encodes a protein with structural characteristics of potassium channels, and expression of the gene as measured by Northern blot analysis demonstrated that *KVLQT1* is most strongly expressed in the heart. One intragenic deletion and ten different missense mutations which cause LQT were identified in *KVLQT1*. These data define KVLQT1 as a novel cardiac potassium channel gene and show that mutations in this gene cause susceptibility to ventricular tachyarrhythmias and sudden death.

It was known that that one component of the I_{Ks} channel is minK, a 130 amino acid protein with a single putative transmembrane domain (Takumi et al., 1988; Goldstein and Miller, 1991; Hausdorff et al., 1991; Takumi et al., 1991; Busch et al., 1992; Wang and Goldstein, 1995; Wang et al., 1996). The size and structure of this protein made it unlikely that minK alone forms functional channels (Attali et al., 1993; Lesage et al., 1993). Evidence is presented that KVLQT1 and minK coassemble to form the cardiac I_{Ks} potassium channel. I_{Ks} dysfunction is a cause of cardiac arrhythmia.

### SUMMARY OF THE INVENTION

The present invention relates to a method for assessing the risk in a human subject for long OT syndrom according to Claims 1-4. The molecular basis of long QT syndrome is demonstrated. More specifically, the present invention has determined that molecular variants of the *KVLQT1* gene cause or are involved in the pathogenesis of LQT. Genotypic analyses show that *KVLQT1* is completely linked to *LQT1* in sixteen unrelated families. Analysis of the *KVLQT1* gene will provide an early diagnosis of subjects with LQT. The diagnostic method comprises analyzing the DNA sequence of the *KVLQT1* gene of an individual to be tested and comparing it with the DNA sequence of the native, non-variant gene. In a second embodiment, the *KVLQT1* gene of an individual to be tested is screened for mutations which cause LQT. The ability to predict LQT will enable physicians to prevent the disease with medical therapy such as beta blocking agents.

It is further demonstrated that KVLQT1 and minK coassemble to form a cardiac I_{Ks} potassium channel. I_{Ks} dysfunction is a cause of cardiac arrhythmia. The knowledge that these two proteins coassemble to form the I_{Ks} channel is useful for developing an assay to screen for drugs which are useful in treating or preventing LQT1. By coexpressing both genes in a cell such as an oocyte it is possible to screen for drugs which have an effect on the I_{Ks} channel, both in its wild-type and in its mutated forms. This knowledge is also useful for the analysis of the *minK* gene for an early diagnosis of subjects with LQT. The diagnostic methods are performed as noted above for *KVLQT1*.

### BRIEF DESCRIPTION OF THE FIGURES

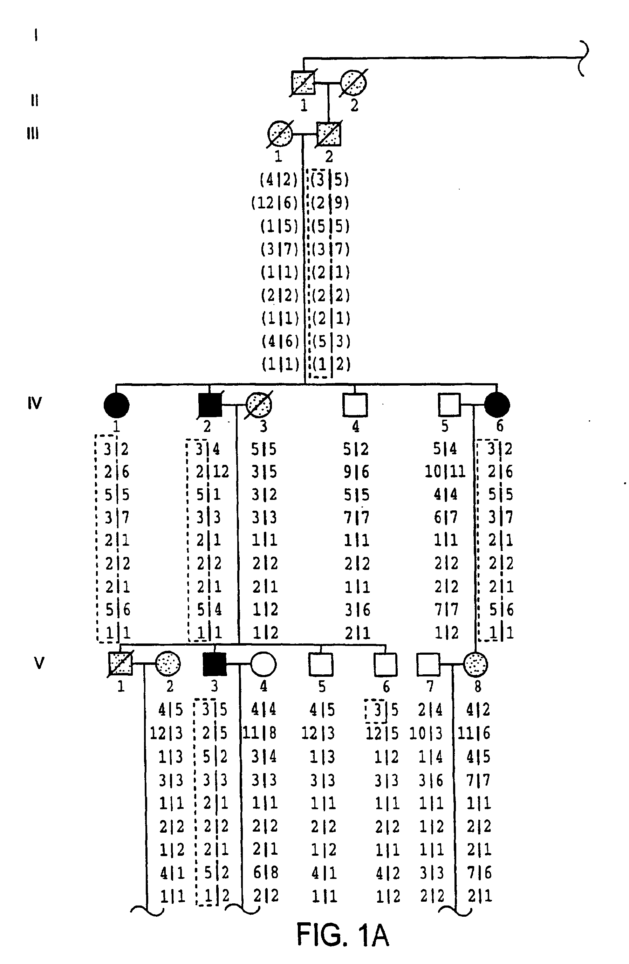

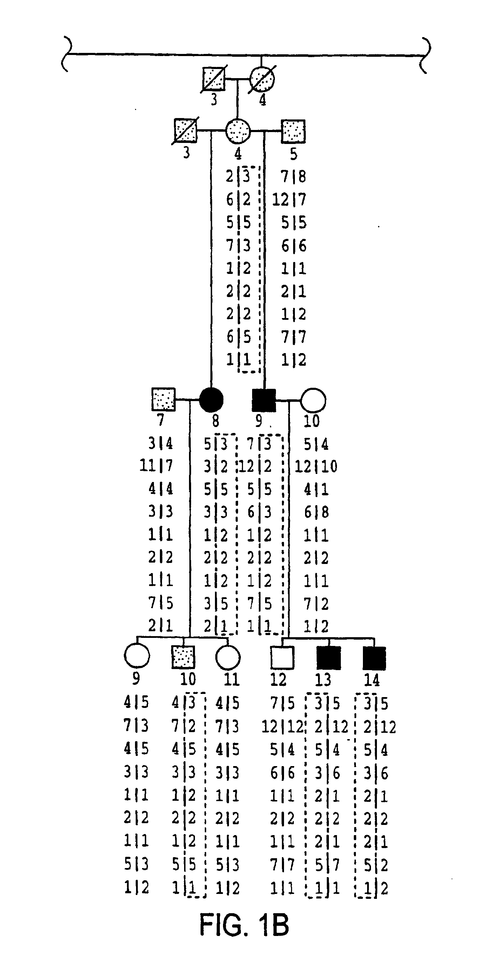

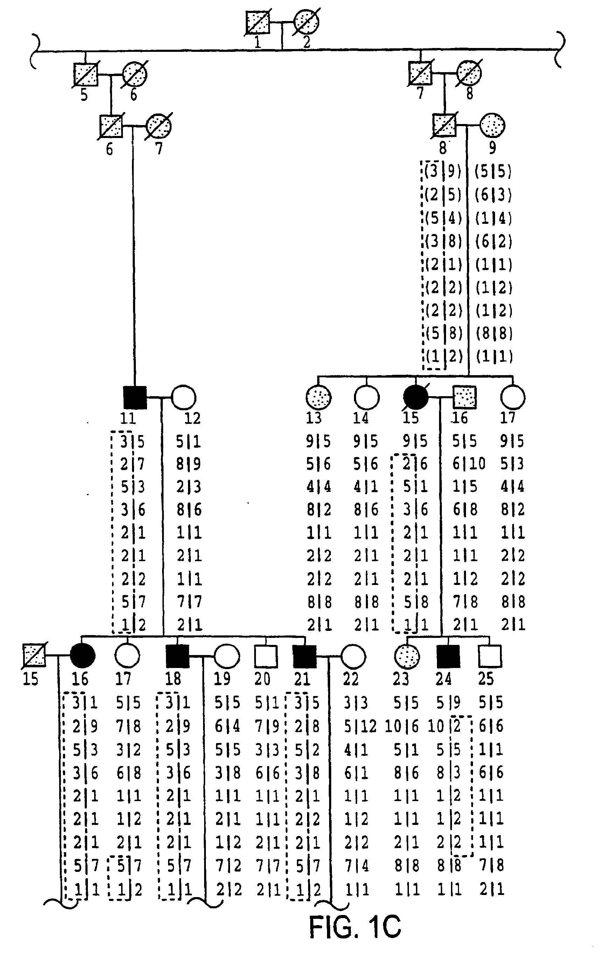

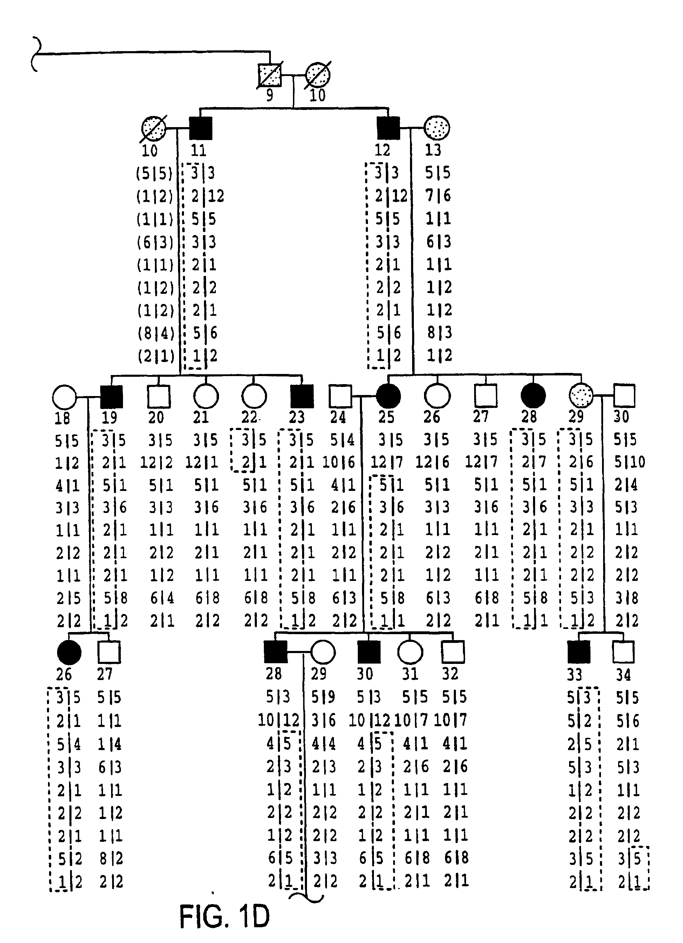

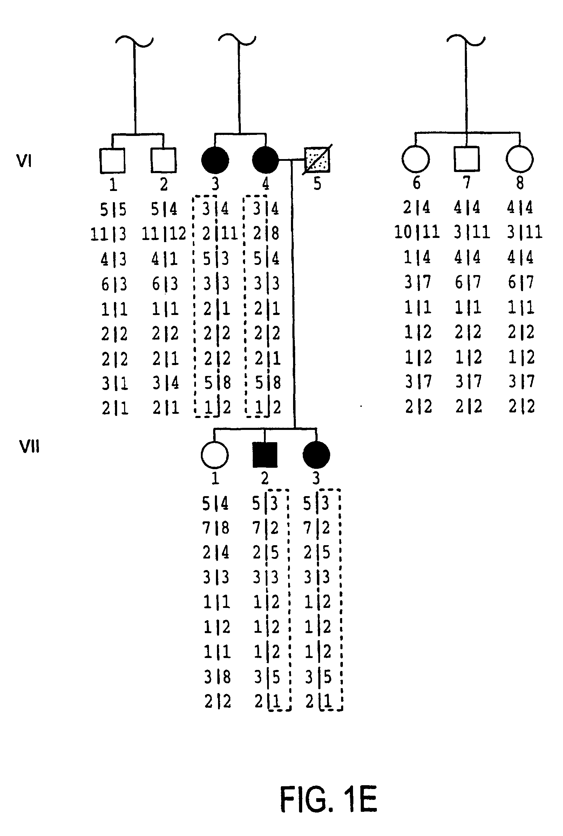

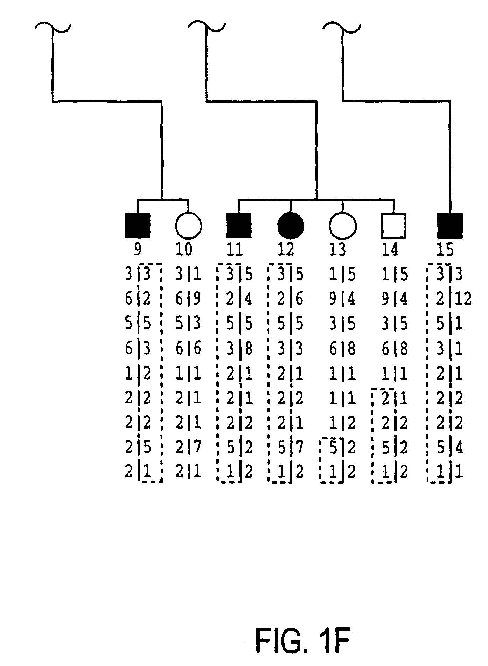

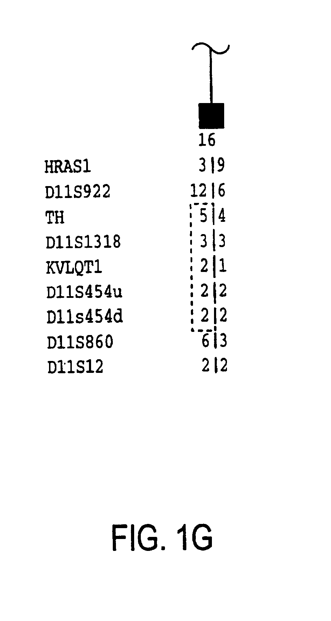

Figure 1. Pedigree structure for a portion of LQT kindred 1532. Affected individuals are shown as filled circles (females) or squares (males), unaffected individuals as empty symbols and individuals with equivocal phenotypes are stippled. Genotypes for chromosome 11 markers are indicated beneath each symbol and are shown as haplotypes. Marker order (top to bottom) is: *Tel-HRAS-D11S922-TH-D11S1318-D11S454-D11S860-D11S12*-Cen. The accuracy of haplotypes was ensured using genotypes from additional chromosome 11p15.5 markers (Q. Wang, unpublished results). Inferred genotypes are shown in brackets. Disease chromosomes are indicated by boxes and recombination events are indicated with solid horizontal lines. Recombination events affecting disease chromosomes occur in individuals: IV-22, IV-25, V-6, V-17, V-24, V-34, VI-13, VI-14 and VI-16. Recombination events occurring in non-disease chromosomes are not indicated. *KVLQT1* is an SSCP conformer within *KVLQT1* identified by primers 5 and 6; this conformer was only identified in K1532 and represents a disease-associated mutation (allele 2 is the mutant allele). Haplotype analyses indicate that *KVLQT1* is located between flanking markers *D11S922* and *D11S454*.

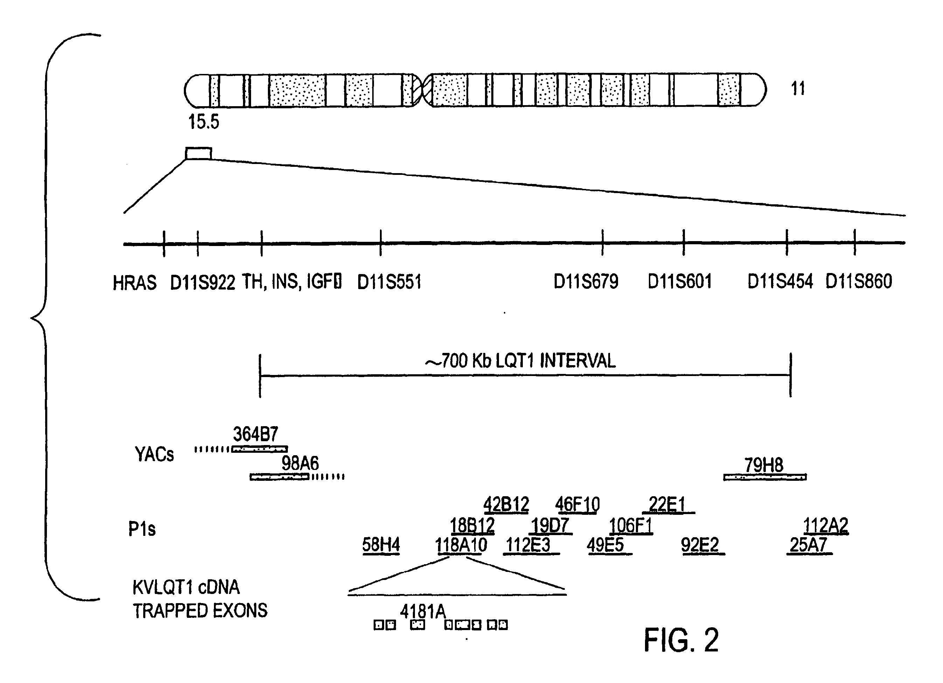

Figure 2. Physical map of the *LQT1* region. Ideogram of chromosome 11 indicates the approximate location of *LQT1* (11p15.5). The location of polymorphic markers and some cosmids are indicated by vertical lines on the map. Refined genetic mapping places *LQT1* between *TH* and *D11S454*. The distance between *TH* and *D11S454* was estimated by pulsed field gel analyses as <700 kb. A physical map of the minimal set of overlapping YAC and P1 clones is shown. The locations of the *KVLQT1* cDNA and trapped exons are indicated. Dashed lines in YACs indicate chimerism.

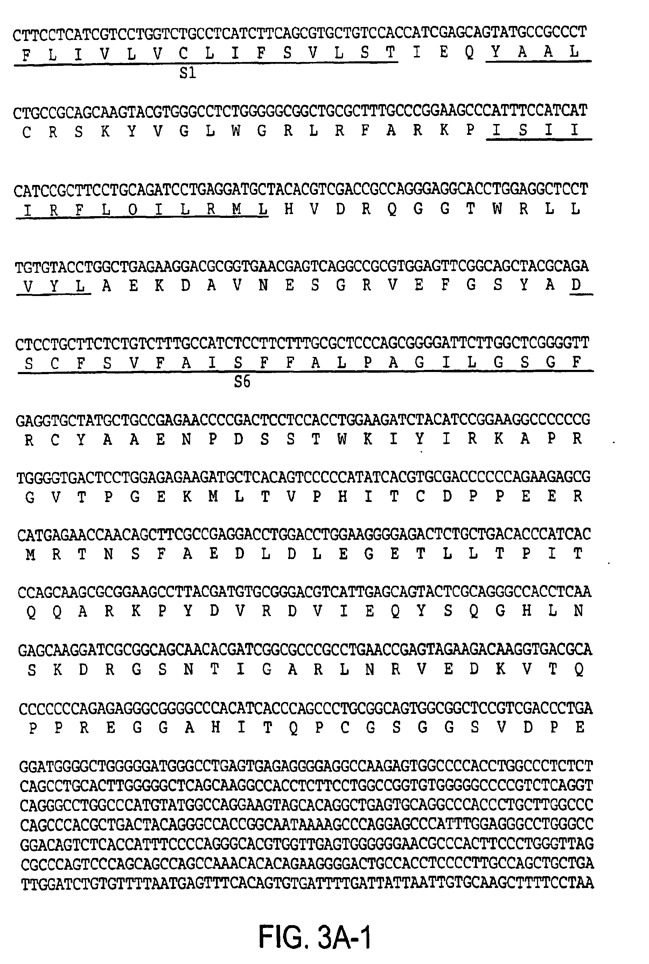

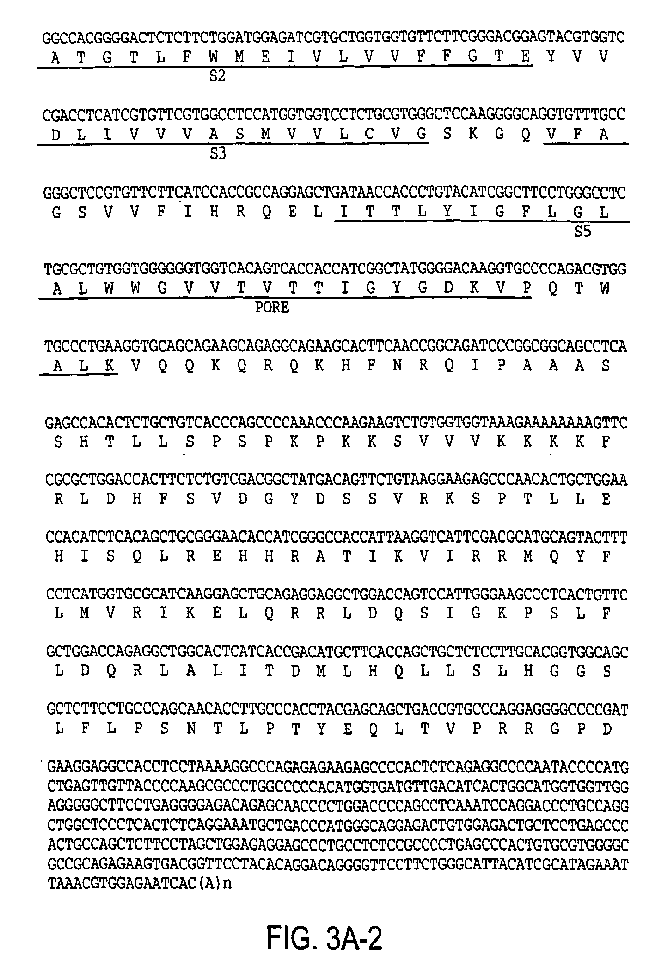

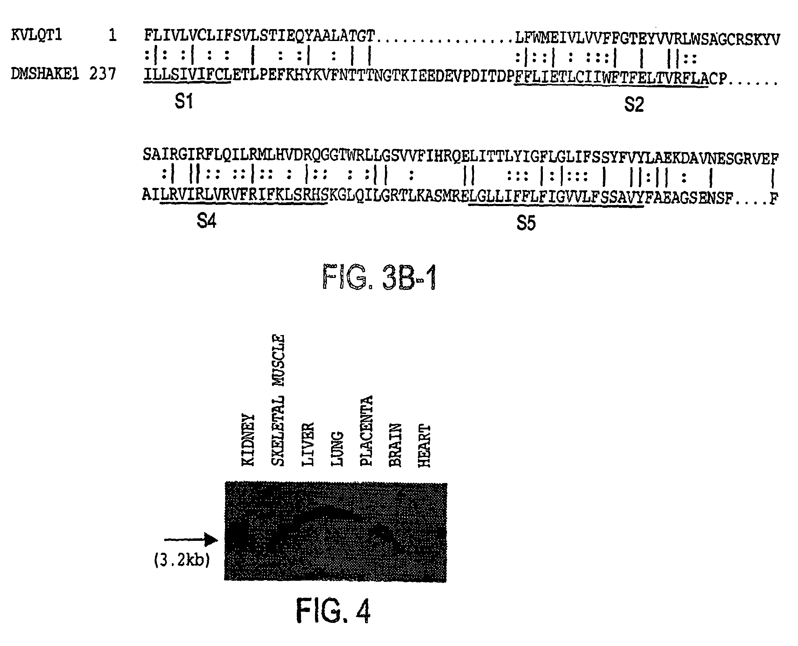

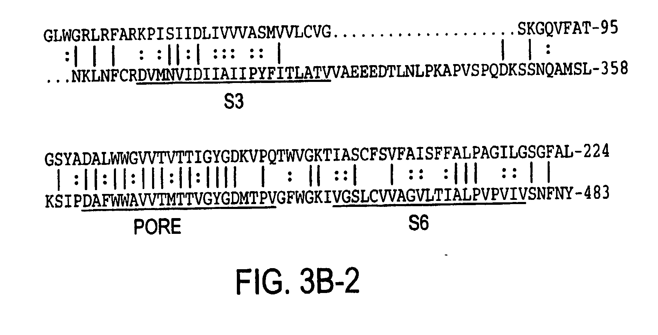

Figures 3A and 3B. Nucleotide and deduced amino acid sequences of *KVLQT1* (not including the region encoding the first 34 amino acids). (A) The composite sequence of *KVLQT1* is shown. The nucleotide sequence is SEQ ID NO:15. The amino acid sequence is SEQ ID NO:16. Six putative transmembrane segments (S 1 to S6) and a putative pore region (Pore) are indicated. A potential glycosylation site (N160) is italicized. Two consensus polyadenylation signals are indicated in the 3' untranslated region in bold. Composite cDNA sequences for *KVLQT1* were obtained by end sequencing of overlapping cDNA clones and by primer walking. *KVLQT1* sequences have been assigned GenBank accession number U40990. (B) Alignment of the S1-S6 region of KVLQT1 with *Drosophila* Shaker potassium channel, DMSHAKE1 (SHA) (Pongs et al., 1988). Identity (I) and similarity (:) are indicated. The 3 separate fragments of KVLQT1 are in order: SEQ ID NO:17, SEQ ID NO:18 and SEQ ID NO:19. The 3 separate fragments of DMSHAKE1 are in order: SEQ ID NO:20, SEQ ID NO:21 and SEQ ID NO:22.
Figure 4. Tissue expression pattern of *KVLQT1*. Northern analyses revealed a 3.2 kb *KVLQT1* mRNA in human kidney, lung, placenta, and heart, with highest levels in the heart.

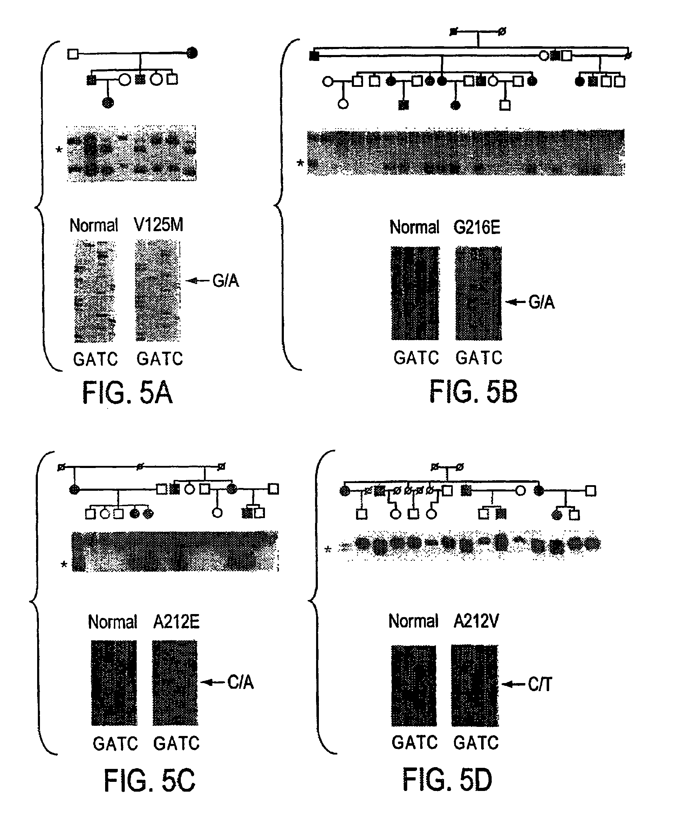

Figures 5A-5D. *KVLQT1* missense mutations cosegregate with LQT in kindreds K1532 (Figure 5A), K2605 (Figure 5B), K1723 (Figure 5C) and K1807 (Figure 5D). The results of SSCP analyses with primer pair 5-6 (K1532), primer pair 9-10 (K1723, K1807), and primer pair 11-12 (K2605) are shown below each pedigree. Aberrant SSCP conformers (indicated by *) cosegregate with LQT in each kindred. For K1532, only eight of the 217 individuals are shown; the results of SSCP analyses in additional members of K1532 are shown in Figure 1 (*KVLQT1* allele 2). Because aberrant SSCP conformers cosegregating with LQT in K161 and K162 were identical to the aberrant conformer defined in K1807, results for these kindreds are not shown. Results of DNA sequence analyses of the normal (left) and aberrant conformers (right) are shown below each pedigree.

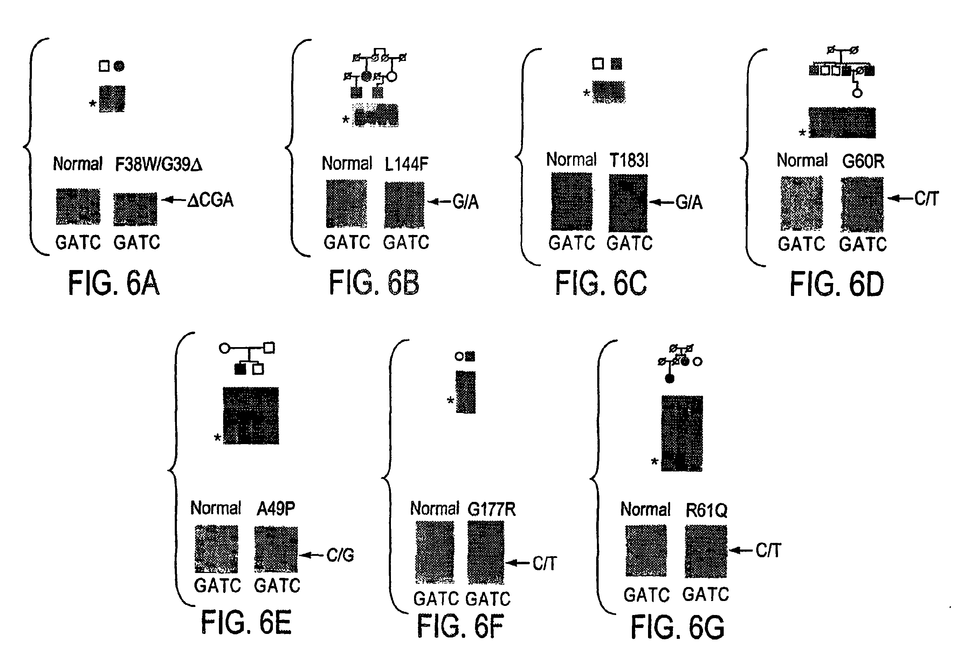

Figures 6A-6G. *KVLQT1* intragenic deletions and missense mutations associated with LQT in kindreds K13216 (Figure 6A), K1777 (Figure 6B), K20925 (Figure 6C), K2557 (Figure 6D), K13119 (Figure 6E), K20926 (Figure 6F) and K15019 (Figure 6G). The results of SSCP analyses with primer pair 1-2 (K13216, K2557, K13119, K15019), primer pair 7-8 (K1777, K20926), and primer pair 9-10 (K20925) are shown below each pedigree. Because aberrant SSCP conformers cosegregating with LQT in K2050, K163 and K164 were identical to the aberrant conformers defined in K1723 and K1807, results for these kindreds are not shown. Results of DNA sequence analyses of the normal (left) and aberrant (right) conformers are shown below each pedigree. Sequences shown are on the antisense strand.

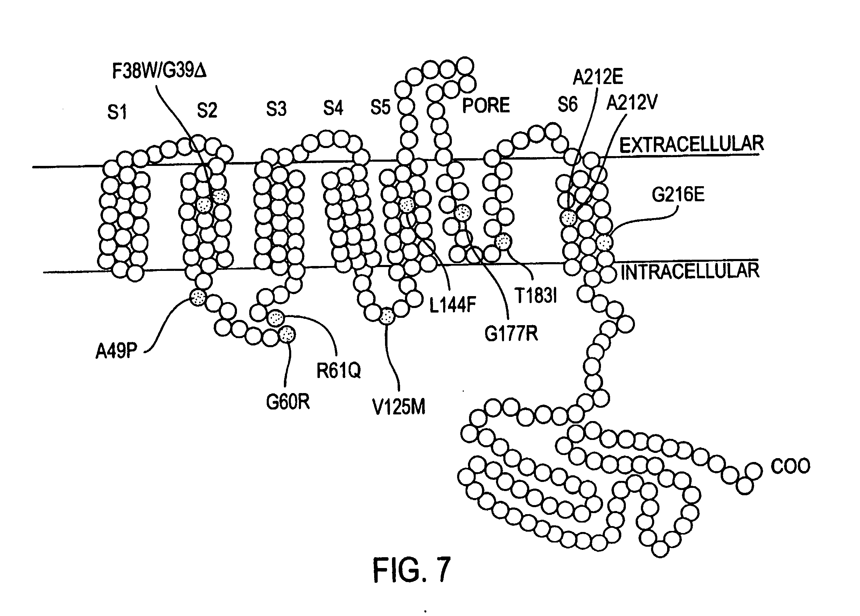

Figure 7. Schematic representation of the predicted topology of KVLQT1 protein and location of *KVLQT1* mutations.

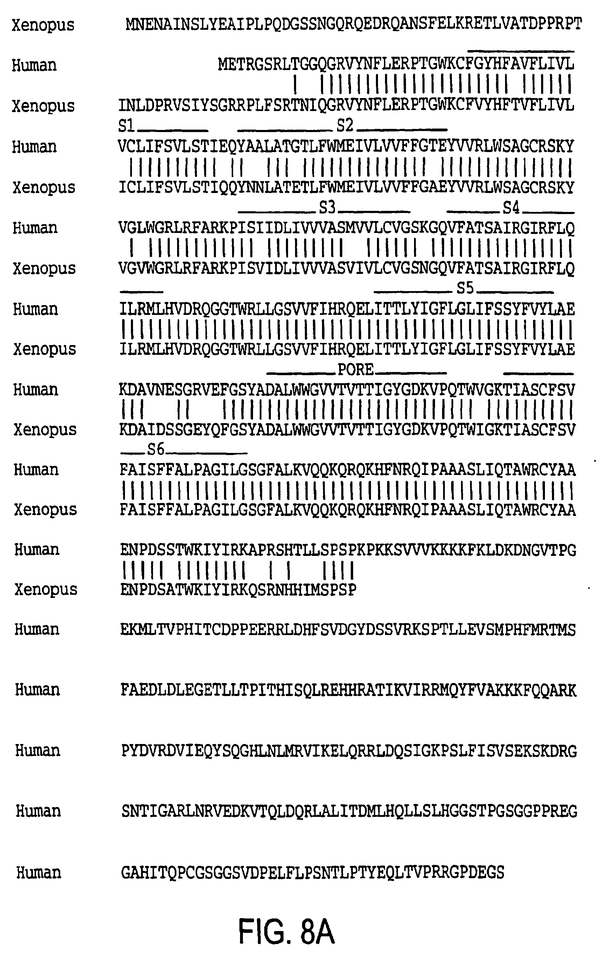

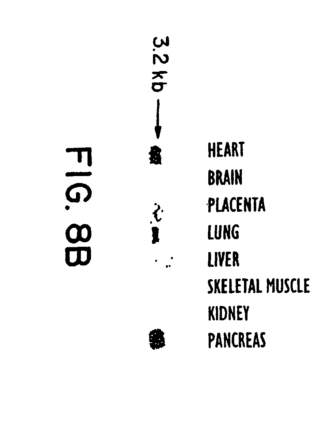

Figures 8A and 8B. Structure of human and *Xenopus* KVLQT1 and tissue-expression pattern of human *KVLQT1*. A) Comparison of human and a partial *Xenopus* KVLQT1 amino acid sequence. Vertical lines indicate identical residues. The Xenopus amino acid sequence is SEQ ID NO:23 and the human amino acid sequence is SEQ ID NO:24. B) Northern analysis indicating expression of *KVLQT1* in human heart, placenta, lung, kidney and pancreas.

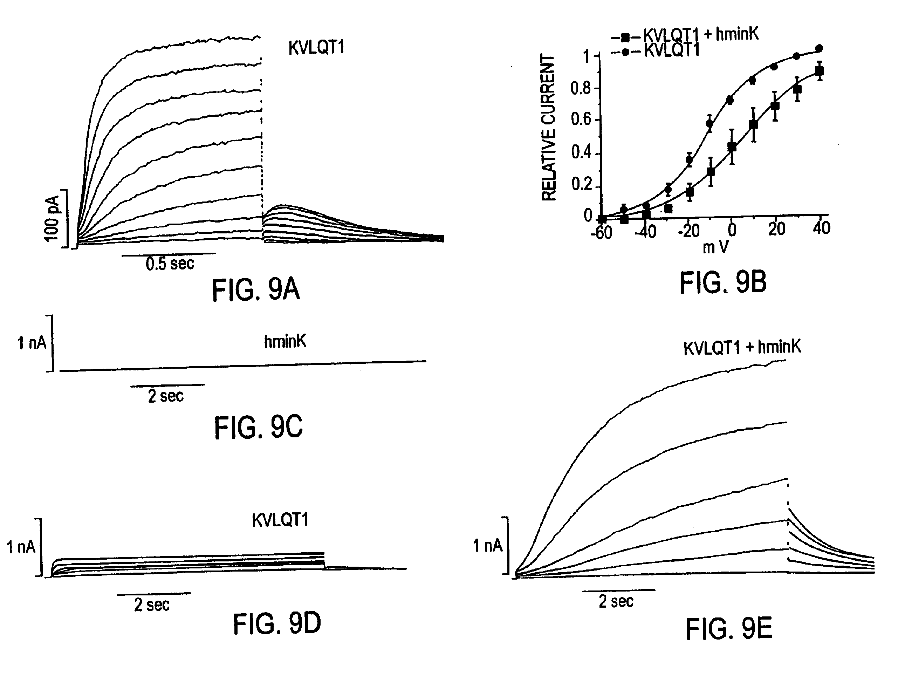

Figures 9A-9E. KVLQT1 and hminK coexpression in CHO cells induces a current nearly identical to cardiac I_{Ks}. A) KVLQT1 currents recorded during 1 sec depolarizing pulses to membrane potentials of -50 to +40 mV, applied from a holding potential of -80 mV. Tail currents were measured at -70 mV. B) Normalized isochronal activation curves for cells transfected with *KVLQT1* (n = 6; 1 sec pulses) or *KVLQT1* and *hminK* (n = 7; 7.5 sec pulses). C-E) Currents recorded during 7.5 sec pulses to -40, -20, -10, 0, +20 and +40 mV in cells transfected with *hminK* (C), *KVLQT1* (D) or *KVLQT1* and *hminK* (E). Tail currents were measured at -70 mV in D, and at -50 mV in C and E. The amplitude of steady state KVLQT1 current at +40 mV was 0.37 ± 0.14 nA (n = 6). In cells cotransfected with *KVLQT1* and *hminK*, time-dependent current during a 7.5-s pulse to +40 mV was 1.62 ± 0.39 nA (n = 7).

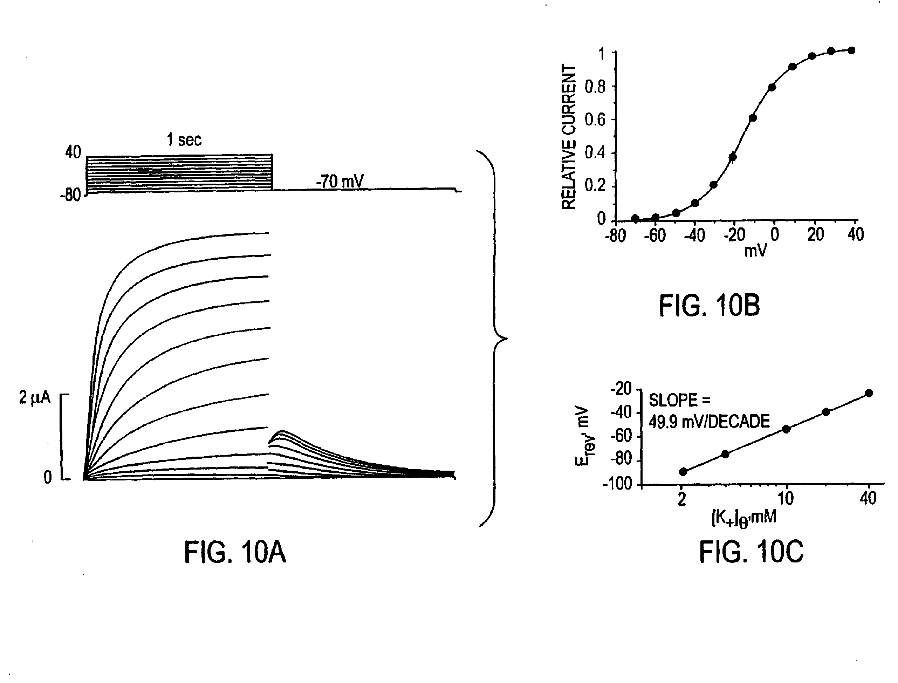

Figures 10A-10C. Expression of KVLQT1 in *Xenopus* oocytes. A) Currents recorded in an oocyte injected with 12.5 ng *KVLQT1* cRNA. Pulses were applied in 10 mV increments from -70 to +40 mV. B) Isochronal (1s) activation curve for KVLQT1 current. The V_{½} was -14.0 ± 0.2 mV and the slope factor was 11.2 ± 0.2 mV (n = 9). C) The relationship of Eᵣₑᵥ versus log[K⁺]ₑ was fit with a linear function and had a slope of 49.9 ± 0.4 mV (n = 6-7 oocytes per point). Tail currents were measured at several voltages after 1.6 sec prepulses to +10 mV.

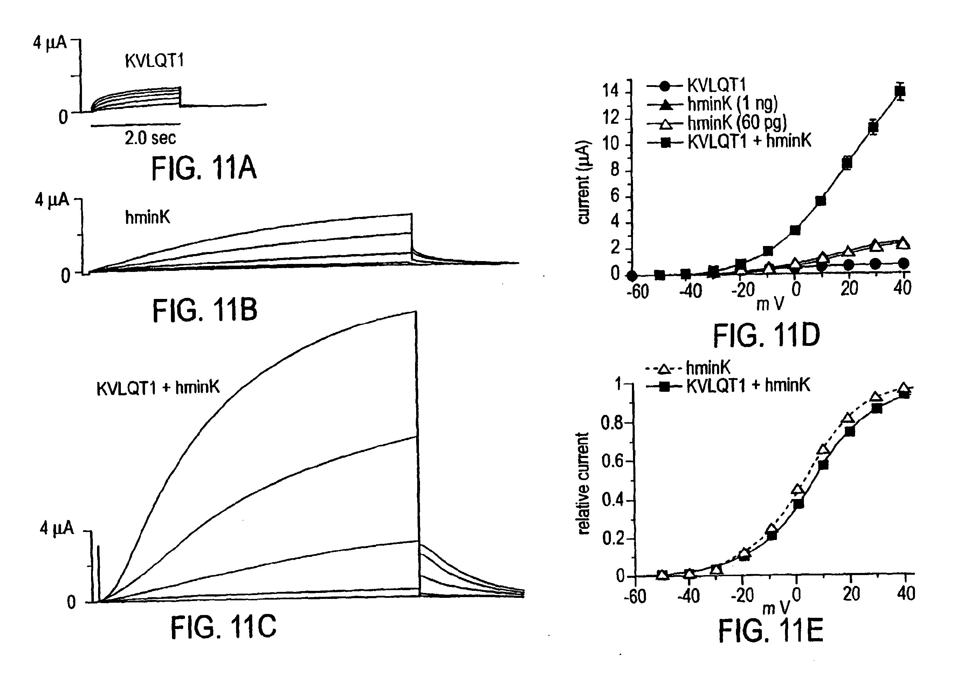

Figures 11A-11E. Coexpression of KVLQT1 and hminK suggests the presence of a KVLQT1 homologue in *Xenopus* oocytes. Currents were recorded at -40, -20, 0, +20 and +40 mV in oocytes injected with either 5.8 ng *KVLQT1* (Figure 11A), 1 ng *hminK* (Figure 11B), or co-injected with both cRNAs (Figure 11C). Figure 11D shows current-voltage relationships measured using 2 sec pulses for KVLQT1, and 7.5 sec pulses for hminK, or KVLQT1 and hminK (n = 20 cells for each condition). For oocytes injected with 60 pg or 1 ng of *hminK* cRNA, I_{sK} at +40 mV was 2.11 ± 0.12 µA and 2.20 ± 0.18 µA. Figure 11E shows normalized isochronal activation curves for oocytes injected with *hminK* (V½ = 2.4 ± 0.3 mV; slope = 11.4 ± 0.3 mV; n = 16) or co-injected with *KVLQT1* and *hminK* cRNA (V½ = 6.2 ± 0.3 mV; slope = 12.3 ± 0.2 mV; n = 20).

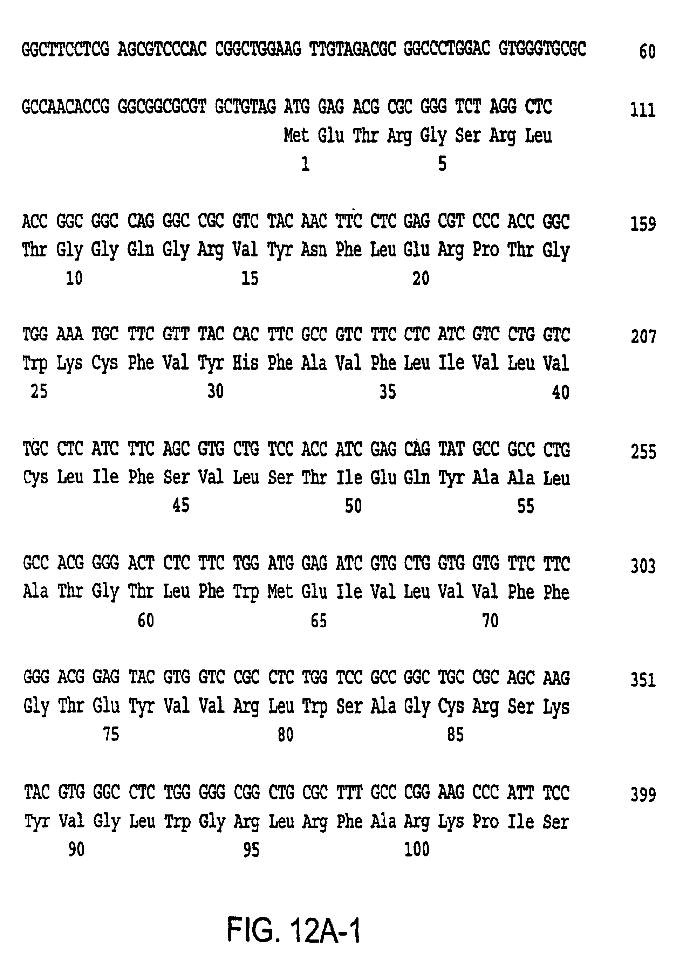

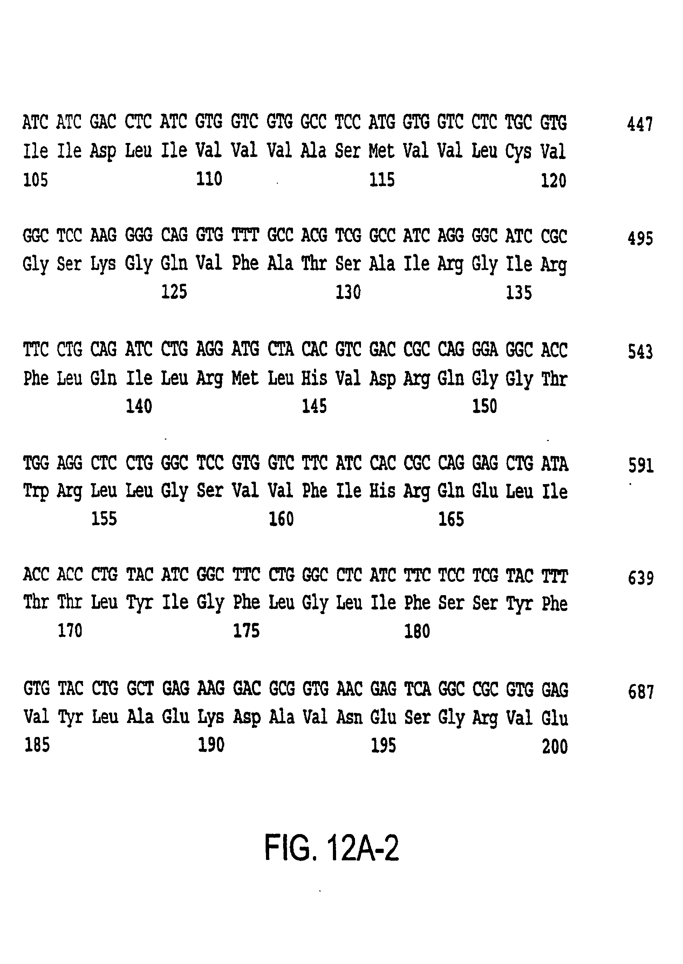

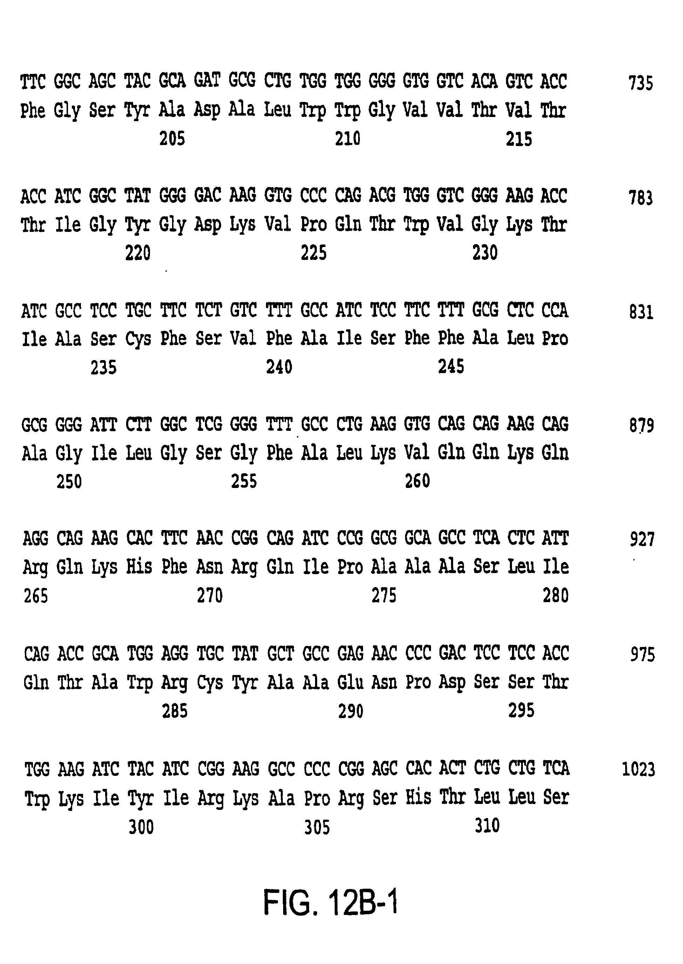

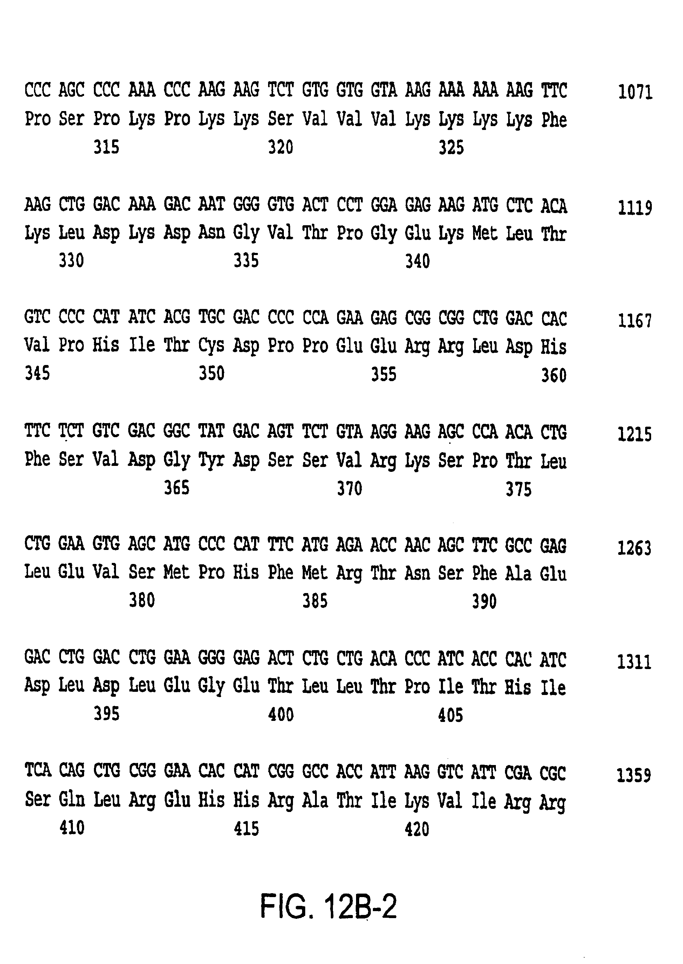

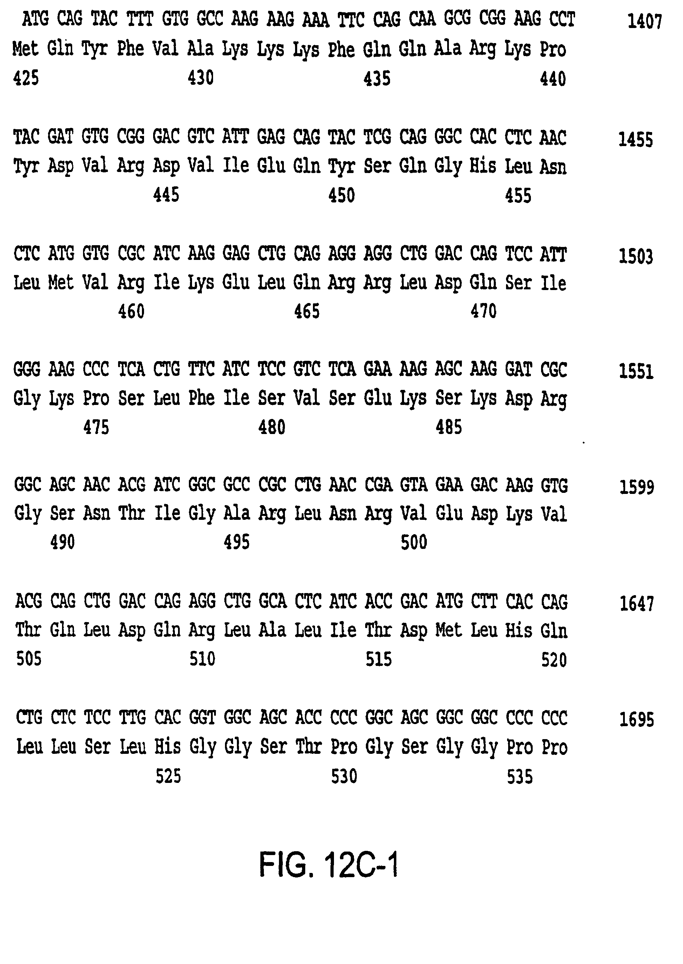

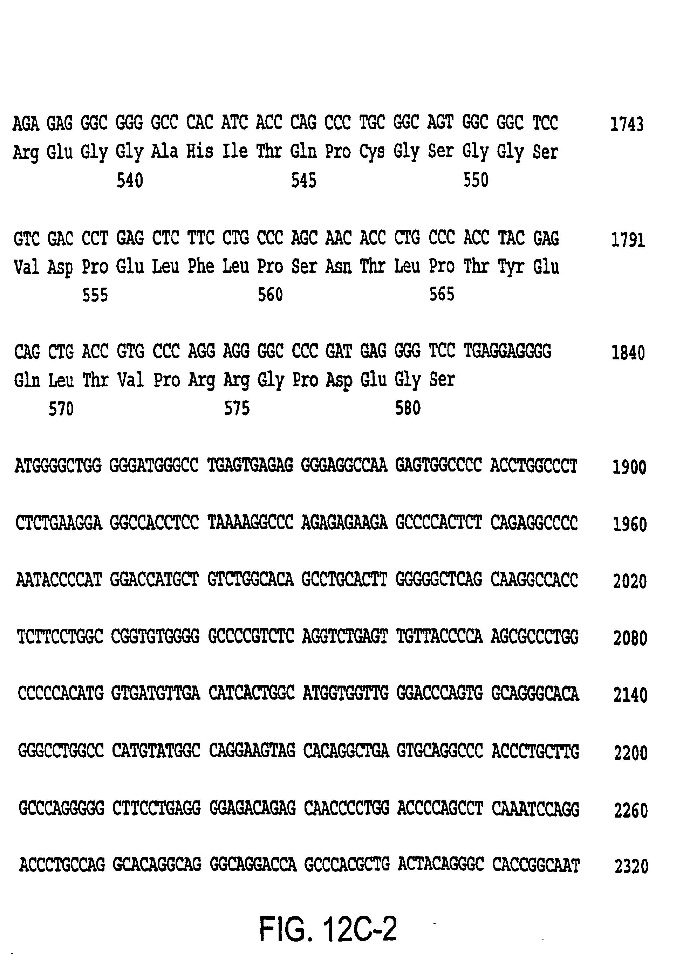

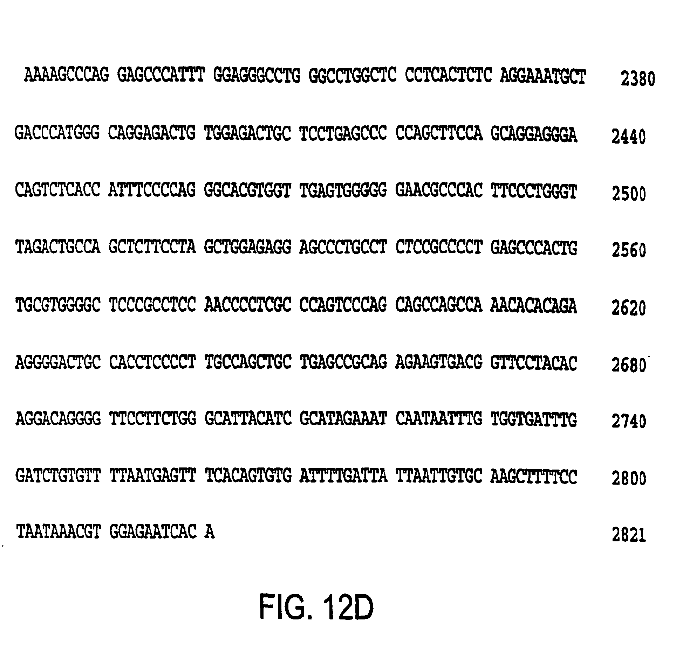

Figures 12A-12D. The nucleotide sequence for *KVLQT1* cDNA and its translation product are shown.

### DETAILED DESCRIPTION OF THE INVENTION

The present invention is directed to the determination that LQT maps to the *KVLQT1* gene and that molecular variants of this gene according to seq ID No. 15 cause or are involved in the pathogenesis of LQT. Described is the determination that KVLQT1 and minK coassemble to form cardiac I_{Ks} potassium channels. More specifically, the present invention relates to mutations in the *KVLQT1* gene and their use in the diagnosis of LQT. The present invention is further directed to methods of screening humans for the presence of *KVLQT1* gene variants which cause LQT. Since LQT can now be detected earlier (i.e., before symptoms appear) and more definitively, better treatment options will be available in those individuals identified as having LQT. Also described are methods for screening for drugs useful in treating or preventing LQT1.

The present invention provides methods of screening the *KVLQT1* gene to identify mutations. Such methods may further comprise the step of amplifying a portion of the *KVLQT1* gene, and may further include a step of providing a set of polynucleotides which are primers for amplification of said portion of the *KVLQT1* gene. The method is useful for identifying mutations for use in prognosis of LQT.

Long QT syndrome is an inherited disorder that causes sudden death from cardiac arrhythmias, specifically *torsade de pointes* and ventricular fibrillation. LQT was previously mapped to three loci: *LQT1* on chromosome 11p15.5, *LQT2* on 7q35-36 and *LQT3* on 3p21-24. It is a discovery of the present invention that there is a genetic linkage between *LQT1* and polymorphisms within *KVLQT1*, a cardiac potassium channel gene.

Demonstrated is that *minK* on chromosome 21 is also involved in LQT. The minK protein and KVLQT1 coassemble to form a K⁺ channel. Thus, described are methods of screening the *minK* gene to identify mutations. Such methods may further comprise the step of amplifying a portion of the *minK* gene, and may further include a step of providing a set of polynucleotides which are primers for amplification of said portion of the *minK* gene. The method is useful for identifying mutations for use in either diagnosis of LQT or prognosis of LQT.

Finally, described is a method for screening drug candidates to identify drugs useful for treating or preventing LQT. Drug screening is performed by coexpressing mutant *KVLQT1* and/or *minK* genes in cells, such as oocytes, mammalian cells or transgenic animals, and assaying the effect of a drug candidate on the I_{Ks} channel. The effect is compared to the I_{Ks} channel activity of the wild-type *KVLQT1* and *minK* genes.

Proof that the *KVLQT1* gene is involved in causing LQT is obtained by finding sequences in DNA extracted from affected kindred members which create abnormal *KVLQT1* gene products or abnormal levels of the gene products. Such LQT susceptibility alleles will cosegregate with the disease in large kindreds. They will also be present at a much higher frequency in non-kindred individuals with LQT than in individuals in the general population. The key is to find mutations which are serious enough to cause obvious disruption to the normal function of the gene product. These mutations can take a number of forms. The most severe forms would be frame shift mutations or large deletions which would cause the gene to code for an abnormal protein or one which would significantly alter protein expression. Less severe disruptive mutations would include small in-frame deletions and nonconservative base pair substitutions which would have a significant effect on the protein produced, such as changes to or from a cysteine residue, from a basic to an acidic amino acid or vice versa, from a hydrophobic to hydrophilic amino acid or vice versa, or other mutations which would affect secondary or tertiary protein structure. Silent mutations or those resulting in conservative amino acid substitutions would not generally be expected to disrupt protein function.

According to the prognostic method of the present invention, alteration of the wild-type *KVLQT1* gene is detected. In addition, the method can be performed by detecting the wild-type *KVLQT1* gene and confirming the lack of a cause of LQT as a result of this locus. "Alteration of a wild-type gene" encompasses all forms of mutations including deletions, insertions and point mutations in the coding and noncoding regions. Deletions may be of the entire gene or of only a portion of the gene. Point mutations may result in stop codons, frameshift mutations or amino acid substitutions. Somatic mutations are those which occur only in certain tissues and are not inherited in the germline. Germline mutations can be found in any of a body's tissues and are inherited. Point mutational events may occur in regulatory regions, such as in the promoter of the gene, leading to loss or diminution of expression of the mRNA. Point mutations may also abolish proper RNA processing, leading to loss of expression of the *KVLQT1* gene product, or to a decrease in mRNA stability or translation efficiency.

The presence of LQT may be ascertained by testing any tissue of a human for mutations of the *KVLQT1* gene or the *minK* gene. For convenience of reference, the following description will be directed to the *KVLQT1* gene. However, the description is equally applicable for the *minK* gene for testing for mutations. For example, a person who has inherited a germline *KVLQT1* mutation would be prone to develop LQT. This can be determined by testing DNA from any tissue of the person's body. Most simply, blood can be drawn and DNA extracted from the cells of the blood. In addition, prenatal diagnosis can be accomplished by testing fetal cells, placental cells or amniotic cells for mutations of the *KVLQT1* gene. Alteration of a wild-type *KVLQT1* allele, whether, for example, by point mutation or deletion, can be detected by any of the means discussed herein.

There are several methods that can be used to detect DNA sequence variation. Direct DNA sequencing, either manual sequencing or automated fluorescent sequencing can detect sequence variation. Another approach is the single-stranded conformation polymorphism assay (SSCP) (Orita et al., 1989). This method does not detect all sequence changes, especially if the DNA fragment size is greater than 200 bp, but can be optimized to detect most DNA sequence variation. The reduced detection sensitivity is a disadvantage, but the increased throughput possible with SSCP makes it an attractive, viable alternative to direct sequencing for mutation detection on a research basis. The fragments which have shifted mobility on SSCP gels are then sequenced to determine the exact nature of the DNA sequence variation. Other approaches based on the detection of mismatches between the two complementary DNA strands include clamped denaturing gel electrophoresis (CDGE) (Sheffield et al., 1991), heteroduplex analysis (HA) (White et al., 1992) and chemical mismatch cleavage (CMC) (Grompe et al., 1989). None of the methods described above will detect large deletions, duplications or insertions, nor will they detect a regulatory mutation which affects transcription or translation of the protein. Other methods which might detect these classes of mutations such as a protein truncation assay or the asymmetric assay, detect only specific types of mutations and would not detect missense mutations. A review of currently available methods of detecting DNA sequence variation can be found in a recent review by Grompe (1993). Once a mutation is known, an allele specific detection approach such as allele specific oligonucleotide (ASO) hybridization can be utilized to rapidly screen large numbers of other samples for that same mutation.

A rapid preliminary analysis to detect polymorphisms in DNA sequences can be performed by looking at a series of Southern blots of DNA cut with one or more restriction enzymes, preferably with a large number of restriction enzymes. Each blot contains a series of normal individuals and a series of LQT cases. Southern blots displaying hybridizing fragments (differing in length from control DNA when probed with sequences near or including the *KVLQT1* locus) indicate a possible mutation. If restriction enzymes which produce very large restriction fragments are used, then pulsed field gel electrophoresis (PFGE) is employed.

Detection of point mutations may be accomplished by molecular cloning of the *KVLQT1* allele and sequencing the allele using techniques well known in the art.

There are six well known methods for a more complete, yet still indirect, test for confirming the presence of a susceptibility allele: 1) single stranded conformation analysis (SSCP) (Orita et al., 1989); 2) denaturing gradient gel electrophoresis (DGGE) (Wartell et al., 1990; Sheffield et al., 1989); 3) RNase protection assays (Finkelstein et al., 1990; Kinszler et al., 1991); 4) allele-specific oligonucleotides (ASOs) (Conner et al., 1983); 5) the use of proteins which recognize nucleotide mismatches, such as the *E. coli* mutS protein (Modrich, 1991); and 6) allele-specific PCR (Rano and Kidd, 1989). For allele-specific PCR, primers are used which hybridize at their 3' ends to a particular *KVLQT1* mutation. If the particular mutation is not present, an amplification product is not observed. Amplification Refractory Mutation System (ARMS) can also be used, as disclosed in European Patent Application Publication No. 0332435 and in Newton et al., 1989. Insertions and deletions of genes can also be detected by cloning, sequencing and amplification. In addition, restriction fragment length polymorphism (RFLP) probes for the gene or surrounding marker genes can be used to score alteration of an allele or an insertion in a polymorphic fragment. Such a method is particularly useful for screening relatives of an affected individual for the presence of the mutation found in that individual. Other techniques for detecting insertions and deletions as known in the art can be used.

In the first three methods (SSCP, DGGE and RNase protection assay), a new electrophoretic band appears. SSCP detects a band which migrates differentially because the sequence change causes a difference in single-strand, intramolecular base pairing. RNase protection involves cleavage of the mutant polynucleotide into two or more smaller fragments. DGGE detects differences in migration rates of mutant sequences compared to wild-type sequences, using a denaturing gradient gel. In an allele-specific oligonucleotide assay, an oligonucleotide is designed which detects a specific sequence, and the assay is performed by detecting the presence or absence of a hybridization signal. In the mutS assay, the protein binds only to sequences that contain a nucleotide mismatch in a heteroduplex between mutant and wild-type sequences.

Mismatches, according to the present invention, are hybridized nucleic acid duplexes in which the two strands are not 100% complementary. Lack of total homology may be due to deletions, insertions, inversions or substitutions. Mismatch detection can be used to detect point mutations in the gene or in its mRNA product. While these techniques are less sensitive than sequencing, they are simpler to perform on a large number of samples. An example of a mismatch cleavage technique is the RNase protection method. In the practice of the present invention, the method involves the use of a labeled riboprobe which is complementary to the human wild-type *KVLQT1* gene coding sequence. The riboprobe and either mRNA or DNA isolated from the person are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full length duplex RNA for the riboprobe and the mRNA or DNA. The riboprobe need not be the full length of the mRNA or gene but can be a segment of either. If the riboprobe comprises only a segment of the mRNA or gene, it will be desirable to use a number of these probes to screen the whole mRNA sequence for mismatches.

In similar fashion, DNA probes can be used to detect mismatches, through enzymatic or chemical cleavage. See, e.g., Cotton *et al*., 1988; Shenk *et al*., 1975; Novack *et al*., 1986. Alternatively, mismatches can be detected by shifts in the electrophoretic mobility of mismatched duplexes relative to matched duplexes. See, e.g., Cariello, 1988. With either riboprobes or DNA probes, the cellular mRNA or DNA which might contain a mutation can be amplified using PCR (see below) before hybridization. Changes in DNA of the *KVLQT1* gene can also be detected using Southern hybridization, especially if the changes are gross rearrangements, such as deletions and insertions.

DNA sequences of the *KVLQT1* gene which have been amplified by use of PCR may also be screened using allele-specific probes. These probes are nucleic acid oligomers, each of which contains a region of the gene sequence harboring a known mutation. For example, one oligomer may be about 30 nucleotides in length, corresponding to a portion of the gene sequence. By use of a battery of such allele-specific probes, PCR amplification products can be screened to identify the presence of a previously identified mutation in the gene. Hybridization of allele-specific probes with amplified *KVLQT1* sequences can be performed, for example, on a nylon filter. Hybridization to a particular probe under stringent hybridization conditions indicates the presence of the same mutation in the tissue as in the allele-specific probe.

The most definitive test for mutations in a candidate locus is to directly compare genomic *KVLQT1* sequences from patients with those from a control population. Alternatively, one could sequence messenger RNA after amplification, e.g., by PCR, thereby eliminating the necessity of determining the exon structure of the candidate gene.

Mutations from patients falling outside the coding region of *KVLQT1* can be detected by examining the non-coding regions, such as introns and regulatory sequences near or within the genes. An early indication that mutations in noncoding regions are important may come from Northern blot experiments that reveal messenger RNA molecules of abnormal size or abundance in patients as compared to control individuals.

Alteration of *KVLQT1* mRNA expression can be detected by any techniques known in the art. These include Northern blot analysis, PCR amplification and RNase protection. Diminished mRNA expression indicates an alteration of the wild-type gene. Alteration of wild-type genes can also be detected by screening for alteration of wild-type *KVLQT1* protein. For example, monoclonal antibodies immunoreactive with *KVLQT1* can be used to screen a tissue. Lack of cognate antigen would indicate a mutation. Antibodies specific for products of mutant alleles could also be used to detect mutant gene product. Such immunological assays can be done in any convenient formats known in the art. These include Western blots, immunohistochemical assays and ELISA assays. Any means for detecting an altered *KVLQT1* protein can be used to detect alteration of the wild-type *KVLQT1* gene. Functional assays, such as protein binding determinations, can be used. In addition, assays can be used which detect *KVLQT1* biochemical function. Finding a mutant *KVLQT1* gene product indicates alteration of a wild-type *KVLQT1* gene.

A mutant *KVLQT1* gene or gene product can also be detected in other human body samples, such as serum, stool, urine and sputum. The same techniques discussed above for detection of mutant genes or gene products in tissues can be applied to other body samples. By screening such body samples, a simple early diagnosis can be achieved for LQT.

The primer pairs described herein are useful for determination of the nucleotide sequence of a particular *KVLQT1* or *minK* allele using PCR. The pairs of single-stranded DNA primers for *KYLQT1* can be annealed to sequences within or surrounding the *KVLQT1* gene on chromosome 11 in order to prime amplifying DNA synthesis of the gene itself. The pairs of single-stranded DNA primers for *minK* can be annealed to sequences within or surrounding the *minK* gene on chromosome 21 in order to prime amplifying DNA synthesis of the gene itself. A complete set of these primers allows synthesis of all of the nucleotides of the gene coding sequences, i.e., the exons. The set of primers preferably allows synthesis of both intron and exon sequences. Allele-specific primers can also be used. Such primers anneal only to particular *KVLQT1* mutant alleles, and thus will only amplify a product in the presence of the mutant allele as a template.

In order to facilitate subsequent cloning of amplified sequences, primers may have restriction enzyme site sequences appended to their 5' ends. Thus, all nucleotides of the primers are derived from *KVLQT1* sequence or sequences adjacent to *KVLQT1,* except for the few nucleotides necessary to form a restriction enzyme site. Such enzymes and sites are well known in the art. The primers themselves can be synthesized using techniques which are well known in the art. Generally, the primers can be made using oligonucleotide synthesizing machines which are commercially available. Given the sequence of *KVLQT1,* design of particular primers is well within the skill of the art.

The nucleic acid probes provided herein are useful for a number of purposes. They can be used in Southern hybridization to genomic DNA and in the RNase protection method for detecting point mutations already discussed above. The probes can be used to detect PCR amplification products. They may also be used to detect mismatches with the *KVLQT1* gene or mRNA using other techniques.

It has been discovered that individuals with the wild-type *KVLQT1* gene do not have LQT. However, mutations which interfere with the function of the *KVLQT1* gene product are involved in the pathogenesis of LQT. Thus, the presence of an altered (or a mutant) *KVLQT1* gene which produces a protein having a loss of function, or altered function, directly causes LQT which increases the risk of cardiac arrhythmias. In order to detect a *KVLQT1* gene mutation, a biological sample is prepared and analyzed for a difference between the sequence of the allele being analyzed and the sequence of the wild-type allele. Mutant *KVLQT1* alleles can be initially identified by any of the techniques described above. The mutant alleles are then sequenced to identify the specific mutation of the particular mutant allele. Alternatively, mutant alleles can be initially identified by identifying mutant (altered) proteins, using conventional techniques. The mutant alleles are then sequenced to identify the specific mutation for each allele. The mutations, especially those which lead to an altered function of the protein, are then used for the prognostic methods of the present invention.

It has also been discovered that the KVLQT1 protein coassembles with the minK protein. Thus, mutations in *minK* which interfere in the function of the *minK* gene product are involved in the pathogenesis of LQT. Thus, the presence of an altered (or a mutant) *minK* gene which produces a protein having a loss of function, or altered function, directly causes LQT which increases the risk of cardiac arrhythmias. In order to detect a *minK* gene mutation, a biological sample is prepared and analyzed for a difference between the sequence of the allele being analyzed and the sequence of the wild-type allele. Mutant *minK* alleles can be initially identified by any of the techniques described above. The mutant alleles are then sequenced to identify the specific mutation of the particular mutant (altered) proteins, using conventional techniques. The mutant alleles are then sequenced to identify the specific mutation for each allele. The mutations, especially those which lead to an altered function of the protein, are then used for the prognostic methods of the present invention.

### Definitions

The present invention employs the following definitions:

"Probes". Polynucleotide polymorphisms associated with *KVLQT1* alleles which predispose to LQT are detected by hybridization with a polynucleotide probe which forms a stable hybrid with that of the target sequence, under stringent to moderately stringent hybridization and wash conditions. If it is expected that the probes will be perfectly complementary to the target sequence, stringent conditions will be used. Hybridization stringency may be lessened if some mismatching is expected, for example, if variants are expected with the result that the probe will not be completely complementary. Conditions are chosen which rule out nonspecific/adventitious bindings, that is, which minimize noise. Since such indications identify neutral DNA polymorphisms as well as mutations, these indications need further analysis to demonstrate detection of a *KVLQT1* susceptibility allele.

Probes for *KVLQT1* alleles may be derived from the sequences of the *KVLQT1* region or its cDNA. The probes may be of any suitable length, which span all or a portion of the *KVLQT1* region, and which allow specific hybridization to the region. If the target sequence contains a sequence identical to that of the probe, the probes may be short, e.g., in the range of about 8-30 base pairs, since the hybrid will be relatively stable under even stringent conditions. If some degree of mismatch is expected with the probe, i.e., if it is suspected that the probe will hybridize to a variant region, a longer probe may be employed which hybridizes to the target sequence with the requisite specificity.

The probes will include an isolated polynucleotide attached to a label or reporter molecule and may be used to isolate other polynucleotide sequences, having sequence similarity by standard methods. For techniques for preparing and labeling probes see, e.g., Sambrook et al., 1989 or Ausubel et al., 1992. Other similar polynucleotides may be selected by using homologous polynucleotides. Alternatively, polynucleotides encoding these or similar polypeptides may be synthesized or selected by use of the redundancy in the genetic code. Various codon substitutions may be introduced, e.g., by silent changes (thereby producing various restriction sites) or to optimize expression for a particular system. Mutations may be introduced to modify the properties of the polypeptide, perhaps to change the polypeptide degradation or turnover rate.

Probes comprising synthetic oligonucleotides or other polynucleotides described herein may be derived from naturally occurring or recombinant single- or double-stranded polynucleotides, or be chemically synthesized. Probes may also be labeled by nick translation, Klenow fill-in reaction, or other methods known in the art.

Portions of the polynucleotide sequence having at least about eight nucleotides, usually at least about 15 nucleotides, and fewer than about 6 kb, usually fewer than about 1.0 kb, from a polynucleotide sequence encoding *KVLQT1* are preferred as probes. The probes may also be used to determine whether mRNA encoding *KVLQT1* is present in a cell or tissue.

**"Regulatory sequences"** refers to those sequences normally within 100 kb of the coding region of a locus, but they may also be more distant from the coding region, .which affect the expression of the gene (including transcription of the gene, and translation, splicing, stability or the like of the messenger RNA).

**"Substantial homology or similarity".** A nucleic acid or fragment thereof is "substantially homologous" ("or substantially similar") to another if, when optimally aligned (with appropriate nucleotide insertions or deletions) with the other nucleic acid (or its complementary strand), there is nucleotide sequence identity in at least about 60% of the nucleotide bases, usually at least about 70%, more usually at least about 80%, preferably at least about 90%, and more preferably at least about 95-98% of the nucleotide bases.

Alternatively, substantial homology or (similarity) exists when a nucleic acid or fragment thereof will hybridize to another nucleic acid (or a complementary strand thereof) under selective hybridization conditions, to a strand, or to its complement. Selectivity of hybridization exists when hybridization which is substantially more selective than total lack of specificity occurs. Typically, selective hybridization will occur when there is at least about 55% homology over a stretch of at least about 14 nucleotides, preferably at least about 65%, more preferably at least about 75%, and most preferably at least about 90%. See, Kanehisa, 1984. The length of homology comparison, as described, may be over longer stretches, and in certain embodiments will often be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides.

Nucleic acid hybridization will be affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringent temperature conditions will generally include temperatures in excess of 30°C, typically in excess of 37°C, and preferably in excess of 45°C. Stringent salt conditions will ordinarily be less than 1000 mM, typically less than 500 mM, and preferably less than 200 mM. However, the combination of parameters is much more important than the measure of any single parameter. See, e.g., Wetmur & Davidson, 1968.

Probe sequences may also hybridize specifically to duplex DNA under certain conditions to form triplex or other higher order DNA complexes. The preparation of such probes and suitable hybridization conditions are well known in the art.

### Preparation of recombinant or chemically synthesized nucleic acids; vectors, transformation, host cells

Large amounts of the polynucleotides described herein may be produced by replication in a suitable host cell. Natural or synthetic polynucleotide fragments coding for a desired fragment will be incorporated into recombinant polynucleotide constructs, usually DNA constructs, capable of introduction into and replication in a prokaryotic or eukaryotic cell. Usually the polynucleotide constructs will be suitable for replication in a unicellular host, such as yeast or bacteria, but may also be intended for introduction to (with and without integration within the genome) cultured mammalian or plant or other eukaryotic cell lines. The purification of nucleic acids produced by the methods described herein are described, e.g., in Sambrook et al., 1989 or Ausubel et al., 1992.

The polynucleotides may also be produced by chemical synthesis, e.g., by the phosphoramidite method described by Beaucage & Carruthers, 1981 or the triester method according to Matteucci and Caruthers, 1981, and may be performed on commercial, automated oligonucleotide synthesizers. A double-stranded fragment may be obtained from the single-stranded product of chemical synthesis either by synthesizing the complementary strand and annealing the strand together under appropriate conditions or by adding the complementary strand using DNA polymerase with an appropriate primer sequence.

Polynucleotide constructs prepared for introduction into a prokaryotic or eukaryotic host may comprise a replication system recognized by the host, including the intended polynucleotide fragment encoding the desired polypeptide, and will preferably also include transcription and translational initiation regulatory sequences operably linked to the polypeptide encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Such vectors may be prepared by means of standard recombinant techniques well known in the art and discussed, for example, in Sambrook et al., 1989 or Ausubel et al., 1992.

An appropriate promoter and other necessary vector sequences will be selected so as to be functional in the host, and may include, when appropriate, those naturally associated with the *KVLQT1* or *minK* gene. Examples of workable combinations of cell lines and expression vectors are described in Sambrook et al., 1989 or Ausubel et al., 1992; see also, e.g., Metzger et al., 1988. Many useful vectors are known in the art and may be obtained from such vendors as Stratagene, New England Biolabs, Promega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization, and others. Vectors and promoters suitable for use in yeast expression are further described in Hitzeman et al., EP 73,675A. Appropriate non-native mammalian promoters might include the early and late promoters from SV40 (Fiers et al., 1978) or promoters derived from murine Molony leukemia virus, mouse tumor virus, avian sarcoma viruses, adenovirus II, bovine papilloma virus or polyoma. In addition, the construct may be joined to an amplifiable gene (e.g., DHFR) so that multiple copies of the gene may be made. For appropriate enhancer and other expression control sequences, see also Enhancers and Eukaryotic Gene Expression, Cold Spring Harbor Press, Cold Spring Harbor, New York (1983).

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells which express the inserts. Typical selection genes encode proteins that a) confer resistance to antibiotics or other toxic substances, e.g. ampicillin, neomycin, methotrexate, etc., b) complement auxotrophic deficiencies, or c) supply critical nutrients not available from complex media, e.g., the gene encoding D-alanine racemase for *Bacilli.* The choice of the proper selectable marker will depend on the host cell, and appropriate markers for different hosts are well known in the art.

The vectors containing the nucleic acids of interest can be transcribed *in vitro*, and the resulting RNA introduced into the host cell by well-known methods, e.g., by injection (see, Kubo et al., 1988), or the vectors can be introduced directly into host cells by methods well known in the art, which vary depending on the type of cellular host, including electroporation; transfection employing calcium chloride, rubidium chloride calcium phosphate, DEAE-dextran, or other substances; microprojectile bombardment; lipofection; infection (where the vector is an infectious agent, such as a retroviral genome); and other methods. See generally, Sambrook et al., 1989 and Ausubel et al., 1992. The introduction of the polynucleotides into the host cell by any method known in the art, including, *inter alia*, those described above, will be referred to herein as "transformation." The cells into which have been introduced nucleic acids described above are meant to also include the progeny of such cells.

Large quantities of the nucleic acids and polypeptides may be prepared by expressing the *KVLQT1* or minK nucleic acid or portions thereof in vectors or other expression vehicles in compatible prokaryotic or eukaryotic host cells. The most commonly used prokaryotic hosts are strains of *Escherichia coli,* although other prokaryotes, such as *Bacillus subtilis* or *Pseudomonas* may also be used.

Mammalian or other eukaryotic host cells, such as those of yeast, filamentous fungi, plant, insect, or amphibian or avian species, may also be useful for production of the proteins described herein. Propagation of mammalian cells in culture is *per se* well known. See, Jakoby and Pastan (eds.), 1979. Examples of commonly used mammalian host cell lines are VERO and HeLa cells, Chinese hamster ovary (CHO) cells, and WI38, BHK, and COS cell lines, although it will be appreciated by the skilled practitioner that other cell lines may be appropriate, e.g., to provide higher expression, desirable glycosylation patterns, or other features.

Clones are selected by using markers depending on the mode of the vector construction. The marker may be on the same or a different DNA molecule, preferably the same DNA molecule. In prokaryotic hosts, the transformant may be selected, e.g., by resistance to ampicillin, tetracycline or other antibiotics. Production of a particular product based on temperature sensitivity may also serve as an appropriate marker.

Prokaryotic or eukaryotic cells transformed with the polynucleotides will be useful not only for the production of the nucleic acids and polypeptides described herein, but also, for example, in studying the characteristics of KVLQT1 or minK polypeptide.

The probes and primers based on the *KVLQT1* gene sequence disclosed herein are used to identify homologous *KVLQT1* gene sequences and proteins in other species. These gene sequences and proteins are used in the diagnostic/prognostic, therapeutic and drug screening methods described herein for the species from which they have been isolated.

### Methods of Use: Drug Screening

The disclosure herein is particularly useful for screening compounds by using KVLQT1 and minK proteins in transformed cells, transfected oocytes or transgenic animals. Since mutations in either the KVLQT1 or minK protein can alter the functioning of the cardiac I_{Ks} potassium channel, candidate drugs are screened for effects on the channel using cells containing either a normal KVLQT1 or minK protein and a mutant minK or KVLQT1 protein, respectively or a mutant KVLQT1 and a mutant minK protein. The drug is added to the cells in culture or administered to a transgenic animal and the effect on the induced current of the I_{Ks} potassium channel is compared to the induced current of a cell or animal containing the wild-type KVLQT1 and minK. Drug candidates which alter the induced current to a more normal level are useful for treating or preventing LQT.

### Methods of Use: Nucleic Acid Diagnosis and Diagnostic Kits

In order to detect the presence of a *KVLQT1* or *minK* allele predisposing an individual to LQT, a biological sample such as blood is prepared and analyzed for the presence or absence of susceptibility alleles of *KVLQT1* or *minK*. In order to detect the presence of LQT or as a prognostic indicator, a biological sample is prepared and analyzed for the presence or absence of mutant alleles of *KVLQT1* or *minK*. Results of these tests and interpretive information are returned to the health care provider for communication to the tested individual. Such diagnoses may be performed by diagnostic laboratories, or, alternatively, diagnostic kits are manufactured and sold to health care providers or to private individuals for self-diagnosis.

Initially, the screening method involves amplification of the relevant *KVLQT1* or *minK* sequences. In another preferred embodiment of the invention, the screening method involves a non-PCR based strategy. Such screening methods include two-step label amplification methodologies that are well known in the art. Both PCR and non-PCR based screening strategies can detect target sequences with a high level of sensitivity.

The most popular method used today is target amplification. Here, the target nucleic acid sequence is amplified with polymerases. One particularly preferred method using polymerase-driven amplification is the polymerase chain reaction (PCR). The polymerase chain reaction and other polymerase-driven amplification assays can achieve over a million-fold increase in copy number through the use of polymerase-driven amplification cycles. Once amplified, the resulting nucleic acid can be sequenced or used as a substrate for DNA probes.

When the probes are used to detect the presence of the target sequences the biological sample to be analyzed, such as blood or serum, may be treated, if desired, to extract the nucleic acids. The sample nucleic acid may be prepared in various ways to facilitate detection of the target sequence, e.g. denaturation, restriction digestion, electrophoresis or dot blotting. The targeted region of the analyte nucleic acid usually must be at least partially single-stranded to form hybrids with the targeting sequence of the probe. If the sequence is naturally single-stranded, denaturation will not be required. However, if the sequence is double-stranded, the sequence will probably need to be denatured. Denaturation can be carried out by various techniques known in the art.

Analyte nucleic acid and probe are incubated under conditions which promote stable hybrid formation of the target sequence in the probe with the putative targeted sequence in the analyte. The region of the probes which is used to bind to the analyte can be made completely complementary to the targeted region of human chromosome 11 for *KVLQT1*. Therefore, high stringency conditions are desirable in order to prevent false positives. However, conditions of high stringency are used only if the probes are complementary to regions of the chromosome which are unique in the genome. The stringency of hybridization is determined by a number of factors during hybridization and during the washing procedure, including temperature, ionic strength, base composition, probe length, and concentration of formamide. These factors are outlined in, for example, Maniatis et al., 1982 and Sambrook et al., 1989. Under certain circumstances, the formation of higher order hybrids, such as triplexes, quadraplexes, etc., may be desired to provide the means of detecting target sequences.

Detection, if any, of the resulting hybrid is usually accomplished by the use of labeled probes. Alternatively, the probe may be unlabeled, but may be detectable by specific binding with a ligand which is labeled, either directly or indirectly. Suitable labels, and methods for labeling probes and ligands are known in the art, and include, for example, radioactive labels which may be incorporated by known methods (e.g., nick translation, random priming or kinasing), biotin, fluorescent groups, chemiluminescent groups (e.g., dioxetanes, particularly triggered dioxetanes), enzymes, antibodies and the like. Variations of this basic scheme are known in the art, and include those variations that facilitate separation of the hybrids to be detected from extraneous materials and/or that amplify the signal from the labeled moiety. A number of these variations are reviewed in, e.g., Matthews & Kricka, 1988; Landegren et al., 1988; U.S. Patent 4,868,105; and in EPO Publication No. 225,807.

As noted above, non-PCR based screening assays are also contemplated in this invention. This procedure hybridizes a nucleic acid probe (or an analog such as a methyl phosphonate backbone replacing the normal phosphodiester), to the low level DNA target. This probe may have an enzyme covalently linked to the probe, such that the covalent linkage does not interfere with the specificity of the hybridization. This enzyme-probe-conjugate-target nucleic acid complex can then be isolated away from the free probe enzyme conjugate and a substrate is added for enzyme detection. Enzymatic activity is observed as a change in color development or luminescent output resulting in a 10³-10⁶ increase in sensitivity. For an example relating to the preparation of oligodeoxynucleotide-alkaline phosphatase conjugates and their use as hybridization probes, see Jablonski et al., 1986.

Two-step label amplification methodologies are known in the art. These assays work on the principle that a small ligand (such as digoxigenin, biotin, or the like) is attached to a nucleic acid probe capable of specifically binding *KVLQT1*. Allele specific probes are also contemplated within the scope of this example and exemplary allele specific probes include probes encompassing the predisposing mutations of this patent application.

In one example, the small ligand attached to the nucleic acid probe is specifically recognized by an antibody-enzyme conjugate. In one embodiment of this example, digoxigenin is attached to the nucleic acid probe. Hybridization is detected by an antibody-alkaline phosphatase conjugate which turns over a chemiluminescent substrate. For methods for labeling nucleic acid probes according to this embodiment see Martin et al., 1990. In a second example, the small ligand is recognized by a second ligand-enzyme conjugate that is capable of specifically complexing to the first ligand. A well known embodiment of this example is the biotin-avidin type of interactions. For methods for labeling nucleic acid probes and their use in biotin-avidin based assays see Rigby et al., 1977 and Nguyen et al., 1992.

The nucleic acid probe assays will employ a cocktail of nucleic acid probes capable of detecting *KVLQT1* or *minK*. Thus, in one example to detect the presence of *KVLQT1* in a cell sample, more than one probe complementary to the gene is employed and in particular the number of different probes is alternatively two, three, or five different nucleic acid probe sequences. In another example, to detect the presence of mutations in the *KVLQT1* gene sequence in a patient, more than one probe complementary to these genes is employed where the cocktail includes probes capable of binding to the allele-specific mutations identified in populations of patients with alterations in *KVLQT1*. Any number of probes can be used, and will preferably include probes corresponding to the major gene mutations identified as predisposing an individual to LQT.

### Methods of Use: Peptide Diagnosis and Diagnostic Kits

The presence of LQT can also be detected on the basis of the alteration of wild-type *KVLQT1* according to Seq ID.No 15 or *minK* polypeptide. Such alterations can be determined by sequence analysis in accordance with conventional techniques. More preferably, antibodies (polyclonal or monoclonal) are used to detect differences in, or the absence of *KVLQT1* or *minK* peptides. Techniques for raising and purifying antibodies are well known in the art and any such techniques may be chosen to achieve the preparations described herein. In a preferred embodiment of the invention, antibodies will immunoprecipitate *KVLQT1* or *minK* proteins from solution as well as react with these proteins on Western or immunoblots of polyacrylamide gels. In another preferred embodiment, antibodies will detect *KVLQT1* or *minK* proteins in paraffin or frozen tissue sections, using immunocytochemical techniques.

Preferred embodiments relating to methods for detecting *KVLQT1* or *minK* or their mutations include enzyme linked immunosorbent assays (ELISA), radioimmunoassays (RIA), immunoradiometric assays (IRMA) and immunoenzymatic assays (IEMA), including sandwich assays using monoclonal and/or polyclonal antibodies. Exemplary sandwich assays are described by David et al., in U.S. Patent Nos. 4,376,110 and 4,486,530.

### EXAMPLE 1

### Methods for Phenotypic Evaluation

For these studies, six large LQT kindreds (K1532, K1723, K2605, K1807, K161 and K162) as well as some small kindreds and sporadic cases were studied. LQT patients were identified from medical clinics throughout North America and Europe. Two factors were considered for phenotyping: 1) historical data (the presence of syncope, the number of syncopal episodes, the presence of seizures, the age of onset of symptoms, and the occurrence of sudden death); and 2) the QT interval on electrocardiograms corrected for heart rate (QT_{c}) (Bazzett, 1920). To avoid misclassifying individuals, the same conservative approach to phenotypic assignment that was successful in previous studies was used (Keating et al., 1991 a; Keating et al., 1991b; Jiang et al., 1994). Informed consent was obtained from each individual, or their guardians, in accordance with local institutional review board guidelines. Phenotypic data were interpreted without knowledge of genotype. Symptomatic individuals with a corrected QT interval (QT_{c}) of 0.45 seconds or greater and asymptomatic individuals with a QT_{c} of 0.47 seconds or greater were classified as affected. Asymptomatic individuals with a QT_{c} of 0.41 seconds or less were classified as unaffected. Asymptomatic individuals with QT_{c} between 0.41 and 0.47 seconds and symptomatic individuals with QT_{c} of 0.44 seconds or less were classified as uncertain.

### EXAMPLE 2

### Genotyping and Linkage Analysis

Genomic DNA was prepared from peripheral blood lymphocytes or cell lines derived from Epstein-Barr virus transformed lymphocytes using standard procedures (Anderson and Gusella, 1984). For genotypic analyses, four small tandem repeat (STR) polymorphisms were used that were previously mapped to chromosome 11p15.5: *D11S922, TH, D11S1318* and *D11S860* (Gyapay et al., 1994). Genotyping of RFLP markers (*HRAS1, D11S454* and *D11S12*) was performed as previously described (Keating et al., 1991a).

Pairwise linkage analysis was performed using MLINK in LINKAGE v5.1 (Lathrop et al., 1985). Assumed values of 0.90 for penetrance and 0.001 for LQT gene frequency were used. Gene frequency was assumed to be equal between males and females. Male and female recombination frequencies were considered to be equal. STR allele frequencies were 1/n where n=number of observed alleles. Although the maximum LOD score for *D11S454* was identified at a recombination fraction of 0, the presence of one non-obligate recombinant (individual VI-14, Figure 1) places this LQT gene telomeric of *D11S454*.

### EXAMPLES 3

### Physical Mapping

Primers were designed based on sequences from TH-INS-IGFII and D11S454 loci and used to identify and isolate clones from CEPH YAC libraries using the PCR based technique (Green and Olson, 1990; Kwiatowski et al., 1990). YAC terminal sequences were determined by inverse PCR as described (Ochman et al., 1988) and used as STSs.

P1 clones were isolated using single copy probes from previously identified cosmids cosQW22 (this study), cCI11-469 (D11S679), cCI11-385 (D11S551), cCI11-565 (D11S601), cCI11-237 (D11S454) (Tanigami et al., 1992; Tokino et al., 1991; Sternberg, 1990). Newly isolated P1s were mapped to chromosome 11p15 by FISH or Southern analyses. End-specific riboprobes were generated from newly isolated P1s and used to identify additional adjacent clones (Riboprobe Gemini Core System Kit; Promega). DNA for P1 and cosmid clones was prepared using alkaline lysis plasmid isolation and purified by equilibrium centrifugation in CsCl-ethidium bromide gradients as described (Sambrook et al., 1989). P1 insert end sequences were determined by cycle sequencing as described (Wang and Keating, 1994). STSs were generated based on these insert end sequences. Overlap between P1s and cosmids was calculated by summing the restriction fragments in common.

### EXAMPLE 4

### Isolation and Characterization of KVLOT1 Clones

An adult human cardiac cDNA library (Stratagene) was plated, and 1 x 10⁶ plaques were screened using trapped exon 4181 A as the probe. Sequences of trapped exon 4181 A were used to design oligonucleotide probes for cDNA library screening. The GENETRAPPER^{™} cDNA Positive Selection System was used to screen 1 x 10¹¹ clones from a human heart cDNA library (Life Technologies, Inc.). The sequences of the capture and repair oligonucleotides were 5'-CAGATCCTGAGGATGCT-3' (SEQ ID NO:1) and 5'-GTACCTGGCTGAGAAGG-3' (SEQ ID NO:2).

Composite cDNA sequences for KVLQT1 were obtained by end sequencing of overlapping cDNA clones and by primer walking. Sequencing was performed either automatically, using Pharmacia A.L.F. automated sequencers, or manually, using a Sequenase Version 2.0 DNA Sequencing Kit (United States Biochemical, Inc.). Database analyses and sequence analyses were carried out using the GCG software package, IG software package, and the BLAST network service from the National Center for Biotechnology Information.

The partial genomic structure (from transmembrane domain S2 to S6) of KVLQT1 was determined by cycle sequencing of P1 18B12 as described (Wang and Keating, 1994). Primers were designed based on KVLQT1 cDNA sequence and used for cycle sequencing.

### EXAMPLE 5

### Mutation Analyses

SSCP was carried out as previously described (Wang et al., 1995a; Wang et al., 1995b). Normal and aberrant SSCP products were isolated sequenced directly as described (Wang and Keating, 1994) or subcloned into pBluescript (SK+; Stratagene) using the T-vector method (Marchuk et al., 1990). When the latter method was used, several clones were sequenced by the dideoxy chain termination method using Sequenase^{™} Version 2.0 (United States Biochemicals, Inc.).

### EXAMPLE 6

### Northern Analyses

A multiple tissue Northern filter (Human MTN blot 1, Clontech) was probed with a ³²P-labeled KVLQT1 cDNA probe as previously described (Curran et al., 1995).

### EXAMPLE 7

### Refined Genetic and Physical Localization of LQT1

The precise location of *LQT1* was determined by genotypic analyses in kindred 1532 (K 1532), a large Utah family of northern European descent (Figure 1). This kindred had been used in the initial study linking the first LQT gene, *LQT1*, to chromosome 11p15.5 (Keating et al., 1991 a; Keating et al., 1991b). Additional family members were identified and phenotyped for a total sample size of 217 individuals. Phenotypic determination was performed as previously described (Keating et al., 1991a; Keating et al., 1991b; Jiang et al., 1994). Preliminary genotypic analyses using markers at *HRAS, TH, D11S454*, and *D11S12* included all ascertained members of K1532. These experiments identified informative branches of this family. Additional genotypic analyses were performed using three highly polymorphic markers from chromosome 11p15.5: *D11S922*, *D11S1318*, and *D11S860* (Gyapay et al., 1994). Genotypes and pairwise LOD scores for each marker are shown in Figure 1 and Table 1. Of these markers, *TH* and *D11S1318* were completely linked. Recombination was identified with all other markers tested, including HRAS, but in each case a statistically significant positive LOD score (+3 or greater) was identified. These data indicate that *LQT1* is completely linked to *TH and D11S1318* in this kindred and that the disease gene is located centromeric of *HRAS*.

To refine localization of *LQT1,* haplotype analyses of K1532 were performed (see Figure 1). Nine chromosomes bearing informative recombination events were identified. Telomeric recombination events were observed in unaffected individual IV-22 (between *D11S922* and *TH*), affected individual IV-25 (between *D11S922* and *TH*), unaffected individual V-6 (between *HRAS* and *DIIS922*), and affected individual V-24 (between *HRAS* and *D11S922*). Centromeric recombination events were identified in unaffected individual V-17 (between *D11S860* and *D11S454*), affected individual V-24 (between *D11S860* and *DIIS454*), unaffected individual V-34 (between *D11S860* and *D11S454*), unaffected individual VI-13 (between *D11S860* and *D11S454*), unaffected individual VI-14 (between *D11S454* and *D11S1318*), and affected individual VI-16 (between *D11S860* and *D11S454*). These data indicate that *LQT1* is located between *D11S922* and *D11S454*. Together with recent studies placing *LQT1* centromeric of *TH* (Russell et al., 1995), these data place *LQT1* in the interval between *TH* and *D11S454*.

The size of the region containing *LQT1* was estimated using pulsed-field gel analyses with genomic probes from chromosome 11P15.5. Probes from *TH, D11S551* and *D11S454* hybridized to a 700 kb Mlu I restriction fragment (Figure 2). These data suggested that the region containing *LQT1* is less than 700 kb. Physical representation of this region was achieved by screening yeast artificial chromosome (YAC) and P1 libraries with probes from the region (Tanigami et al., 1992; Tokino et al., 1991). The order of these clones was confirmed using fluorescent *in situ* hybridization (FISH) analyses as: telomere-*TH-D11S551-D11S679-D11S601-D11S454*-centromere. The clones identified in initial experiments were then used for identification of adjacent, overlapping clones. The minimum set of clones from the *LQT1* interval is shown in Figure 2.

**Table 1 Pairwise LOD scores between LQT1 and 11p15.5 markers**

| Recombination fraction (θ) | | | | | | | | |
|---|---|---|---|---|---|---|---|---|
| | 0.0 | 0.001 | 0.01 | 0.05 | 0.1 | 0.2 | Zₘₐₓ* | θₘₐₓ† |
| *HRAS* | 9.67 | 9.94 | 10.50 | 10.38 | 9.62 | 7.57 | 10.59 | 0.021 |
| *D11S922* | 10.05 | 13.05 | 13.85 | 13.59 | 12.59 | 10.01 | 13.92 | 0.019 |
| *TH* | 11.01 | 10.99 | 10.82 | 10.06 | 9.07 | 6.96 | 11.01 | 0.0 |
| *D11S1318* | 10.30 | 10.29 | 10.13 | 9.40 | 8.47 | 6.50 | 10.30 | 0.0 |
| *KVLQT1* | 14.19 | 14.17 | 13.94 | 12.89 | 11.54 | 8.68 | 14.19 | 0.0 |
| *D11S454* | 11.06 | 11.05 | 10.89 | 10.16 | 9.17 | 7.01 | 11.06 | 0.0 |
| *D11S860* | 5.77 | 6.92 | 8.32 | 9.14 | 8.92 | 7.46 | 9.15 | 0.058 |
| *D11S12* | 1.50 | 2.26 | 3.12 | 3.46 | 3.27 | 2.49 | 3.46 | 0.047 |

| | | | | | | | | |
|---|---|---|---|---|---|---|---|---|
| LOD scores were computed with the assumption of 90% penetrance and gene frequency of 0.001 (ref 31). *Zₘₐₓ indicates maximum LOD score. †θₘₐₓ indicates estimated recombination fraction at Zₘₐₓ. | | | | | | | | |

### EXAMPLE 8

### Identification and Characterization of KVLQT1

Exon amplification with clones from the physical map was performed to identify candidate genes for *LQT1*. Exon trapping was performed using pSPL3B (Burn et al., 1995) on genomic P1 clones as previously described (Buckler et al., 1991; Church et al., 1994). A minimum of 128 trapped exons from each P1 clone were initially characterized by sizing the PCR products. From these, 400 clones were further analyzed by dideoxy sequencing using an A.L.F. automated sequencer (Pharmacia). DNA sequence and database analyses revealed eight possible exons with predicted amino acid sequence similarity to ion channels. The highest similarity was obtained for a 238 base pair trapped exon (4181A), with 53% similarity to potassium channel proteins from multiple species, including similarity to a portion of a putative pore region. PCR analyses were used to map 4181 A to the short arm of chromosome 11 and to two P1s from the physical map (118A10, 18B12). These data suggested that 4181A was part of a potassium channel gene on chromosome 11p15.5.

Two different cDNA library screening methods were used to determine if trapped exon 4181A was part of a gene. Traditional plaque filter hybridization with an adult human cardiac cDNA library led to the identification of a single positive clone. A variation of cDNA selection was used to screen a second cardiac cDNA library (the GENETRAPPER^{™} cDNA Positive Selection System, Life Technologies, Inc.), and twelve independent clones were recovered. DNA sequence analyses revealed complete alignment with sequences derived from 4181A and the other trapped exons described above. The composite sequence of these cDNA clones is shown in Figure 3A. The longest open reading frame spans 1654 base pairs. Two consensus polyadenylation signals were identified upstream of the poly(A) tail in the 3' untranslated region. The identity of the initiation codon is not yet certain.

This cDNA predicted a protein with structural characteristics of potassium channels. Hydropathy analyses suggested a topology of six major hydrophobic regions that may represent membrane-spanning α-helices. These regions share sequence similarity with potassium channel transmembrane domains S1-S6. A comparison of the predicted amino acid sequence derived from the identified gene and the Shaker (SHA) potassium channel (Pongs et al., 1988) is shown in Figure 3B. In the region containing S1-S6, the amino acid sequence identity was 30% and similarity was 59%. The sequence located 3' of S1-S6 did not have significant similarity to any known protein. Because this gene has high similarity to voltage-gated potassium channel genes and became a strong candidate for *LQT1*, it was named *KVLQT1*.

Northern blot analyses were used to determine the tissue distribution of *KVLQT1* mRNA. *KVLQT1* cDNA probes detected a 3.2 kb transcript in human heart, kidney, lung, and placenta, but not in skeletal muscle, liver, or brain (Figure 4). The heart showed highest levels of

*KVLQT1* mRNA.

### EXAMPLE 9

### Characterization of the Complete KVLQT1 cDNA

The studies described above resulted in the cloning and characterization of an incomplete cDNA for *KVLQT1*. The sequence of this incomplete cDNA predicted a protein with six hydrophobic membrane-spanning α-helices (S1-S6) and a typical K+ channel pore signature sequence (Heginbotham et al., 1994). However, this cDNA appeared to be missing the amino terminal domain and did not functionally express. To define the complete sequence of *KVLQT1*, several cDNA libraries were screened and a new clone was isolated. The screening was performed by radiolabeling a partial *KVLQT1* cDNA with ³²P and screening several cDNA libraries obtained from Clontech. A 1.2 kb clone was isolated from a pancreatic library and subcloned into pBluescript II and sequenced. This clone included a putative translational start site and an ATG sequence in-frame with the original *KVLQT1* clones. This new sequence data was combined with the earlier sequence data to yield the cDNA sequence encoding the complete protein. This cDNA sequence as well as 5' and 3' untranslated regions is shown in Figures 12A-12D. The new cDNA sequence predicts a 581 amino acid protein with a complete S1 domain and a 27 amino acid N-terminal region. This is shown in Figure 8A. To ensure that this new sequence was part of the chromosome 11p15.5-linked *KVLQT1* gene, a 135 base pair *Xho*I restriction fragment from this region was used in hybridization experiments with DNA from a somatic cell hybrid panel. The new 5' end mapped to the short arm of chromosome 11. Northern analysis using the new *KVLQT1* sequence indicated hybridization with a single messenger RNA of 3.2 kb in human pancreas, heart, kidney, lung and placenta, but not in brain, liver or skeletal muscle (Figure 8B). The Northern analyses were performed using a multiple tissue Northern filter (Human MTN blot 1, Clontech) as described by Curran et al., 1995.

### EXAMPLE 10

### Characterization of KVLQT1 Function

To define the function of KVLQT1, Chinese hamster ovary (CHO) cells were transfected with the complete cDNA described above in Example 9. The *KVLQT1* cDNA was subcloned into pCEP4 (InVitrogen). CHO cells were cultured in Ham's F-12 medium and transiently transfected using Lipofectamine (Gibco BRL). Cells were transfected for 18 hours in 35 mm dishes containing 6 µL lipofectamine, 0.5 µg green fluorescent protein (pGreen Lantern-1, Gibco BRL), and 1.5 µg of *KVLQT1* in pCEP4. Fluorescent cells were voltage-clamped using an Axopatch 200 patch clamp amplifier (Axon Instruments) 48 to 78 hours after transfection. The bathing solution contained, in mM: 142 NaCl, 2 KCl, 1.2 MgCl₂, 1.8 CaCl₂, 11.1 glucose, 5.5 HEPES buffer (pH 7.4, 22-25°C). The pipette solution contained, in mM: 110 potassium glutamate, 20 KCl, 1.0 MgCl₂, 5 EGTA, 5 K₂ATP, 10 HEPES (pH 7.3). Data acquisition and analyses were done using pCLAMP6 (Axon Instruments). The voltage dependence of current activation was determined by fitting the relationship between tail currents (determine by extrapolation of deactivating phase of current to the end of the test pulse) and test potential with a Boltzmann function. Tail currents were normalized relative to the largest value for each oocyte.

A voltage-dependent, outward K⁺ current was observed after membrane depolarization to potentials above -60 mV (Figure 9A). This current reached a steady state within 1 second at +40 mV. Activation of the current was preceded by a brief delay, and repolarization to -70 mV elicited a tail current with an initial increase in amplitude (a hook) before deactivation. Similar tail current hooks were previously observed for HERG K⁺ channels, and were attributed to recovery of channels from inactivation at a rate faster than deactivation (Sanguinetti et al., 1995; Smith et al., 1996; Spector et al., 1996). The activation curve for KVLQT1 current was half-maximal (V_{½}) at -11.6 ± 0.6 mV, and had a slope factor of 12.6 ± 0.5 mV (n = 6; Figure 9B).

The biophysical properties of KVLQT1 were unlike other known cardiac K⁺ currents. It was hypothesized that KVLQT1 might coassemble with another subunit to form a known cardiac channel. The slowly activating delayed rectifier K⁺ current, I_{Ks}, modulates repolarization of cardiac action potentials. Despite intensive study, the molecular structure of the I_{Ks} channel is not understood. Physiological data suggest that one component of the I_{Ks} channel is minK (Goldstein and Miller, 1991; Hausdorff et al., 1991; Takumi et al., 1991; Busch et al., 1992; Wang and Goldstein, 1995; Wang et al., 1996), a 130 amino acid protein with a single putative transmembrane domain (Takumi et al., 1988). The size and structure of this protein, however, have led to doubt that minK alone forms functional channels (Attali et al., 1993; Lesage et al., 1993).

To test this hypothesis, CHO cells were cotransfected with *KVLQT1* and human *minK* (*hminK*) cDNAs. An *hminK* cDNA was subcloned in pCEP4 (InVitrogen) and transfection was performed as described above for *KVLQT1* alone. For the cotransfection of *KVLQT1* and *hminK*, 0.75 µg of each cDNA was used. As reported previously (Lesage et al., 1993), transfection of CHO cells with *hminK* alone did not induce detectable current (n = 10, Figure 9C). Cotransfection of *hminK* with *KVLQT1* induced a slowly activating delayed-rectifier current that was much larger than the current in cells transfected with *KVLQT1* alone (Figures 9D and 9E). The slow activation of current in cotransfected CHO cells was preceded by a delay that lasted several hundred msec, indicating that no significant homomeric KVLQT1 channel current was present. Current did not saturate during long depolarizing pulses, and required a three-exponential function to best describe the initial delay and two phases of current activation. During a 30 sec depolarizing pulse to +40 mV, current was activated with time constants of 0.68 ± 0.18, 1.48 ± 0.16, and 8.0 ± 0.6 sec (n = 4). The isochronal (7.5 sec) activation curve for current had a V_{½} of 7.5 ± 0.9 mV, and a slope factor of 16.5 ± 0.8 mV (n = 7; Figure 9B). By comparison, the V_{½} and slope of the activation curve for human cardiac I_{Ks} are 9.4 mV and 11.8 mV (Li et al., 1996). Like KVLQT1 and hminK coexpressed in CHO cells, activation of cardiac I_{Ks} is extremely slow and was best described by a three-exponential function (Balser et al., 1990; Sanguinetti and Jurkiewicz, 1990). Quinidine (50 µM) blocked tail currents in cotransfected CHO cells by 30 ± 8% (n = 5), similar to its effect (40-50% block) on I_{Ks} in isolated myocytes (Balser et al, 1991). Thus, coexpression of KVLQT1 and hminK in CHO cells induced a K⁺ current with biophysical properties nearly identical to cardiac I_{Ks}.

To characterize the properties of hminK and KVLQT1 further, these channels were expressed separately and together in *Xenopus* oocytes. *Xenopus laevis* oocytes were isolated and injected with cRNA as described by Sanguinetti et al., 1995. *KVLQT1* cDNA was subcloned into pSP64 (Promega). *HminK* cDNA was a gift from R. Swanson. Roughly equimolar concentrations of *KVLQT1* cRNA (5.8 ng per oocyte) and *hminK* (1 ng per oocyte) cRNA were used for the co-injection experiments. The bathing solution contained, in mM: 98 NaCl, 2 KCl, 2 MgCl₂, 0.1 CaCl₂, and 5 HEPES (pH 7.6, 22-25°C). For reversal-potential experiments, osmolarity was maintained by equimolar substitution of external NaCl for KCl. Currents were recorded using standard two-microelectrode voltage clamp techniques 3 days after injection of oocytes with cRNA (Sanguinetti et al., 1995). Currents were filtered at 0.5 kHz and digitized at 2 kHz. Data are presented as mean ± s.e.m.

Oocytes injected with *KVLQT1* complementary RNA expressed a rapidly activating outward K⁺ current with a voltage dependence of activation nearly identical to CHO cells transfected with *KVLQT1* cDNA (Figures 10A and 10B). The K⁺ selectivity of KVLQT1 channels was determined by measuring the reversal potential (Eᵣₑᵥ) of tail currents in different concentrations of extracellular K ([K⁺]ₑ). The slope of the relationship between Eᵣₑᵥ and log[K⁺]ₑ was 49.9 ± 0.4 mV (n = 7; Figure 10C), significantly less than predicted by the Nernst equation (58 mV) for a perfectly selective K⁺ channel. Co-injection of oocytes with *KVLQT1* and *hminK* cRNA induced a current similar to I_{Ks} (Figure 11C). The slope of the relationship between Eᵣₑᵥ and log[K⁺]ₑ for co-injected oocytes was 49.9 ± 4 mV (n = 6), similar to KVLQT1 alone and to guinea pig cardiac I_{Ks} (49 mV) (Matsuura et al., 1987). The isochronal (7.5 sec) activation curve for co-injected oocytes had a V_{½} of 6.2 mV and a slope of 12.3 mV (Figure 11E), similar to cardiac I_{Ks}.

### EXAMPLE 11

### Identification of a KVLQT1 Gene in Xenopus

By contrast with CHO cells, *hminK* was able to undergo functional expression in *Xenopus* oocytes (Figure 11B). The induced current (I_{sK}) was smaller than the current induced in co-injected oocytes, but the kinetics and voltage dependence of activation were similar (Figures 11 A-E). Two observations have led to the hypothesis that I_{sK} in *Xenopus* oocytes results from channels formed by coassembly of minK with an unidentified, constitutively expressed subunit. First, the magnitude of I_{sK} saturates after injection of very small amounts of *minK* cRNA (Figure 11D), suggesting that an endogenous component of limited quantity is required for functional expression ((Wang and Goldstein, 1995; Cui et al., 1994). Second, heterologous expression of minK in mammalian cells does not induce detectable current (Lesage et al., 1993) (Figure 9C), suggesting that minK is not sufficient to form functional channels. It was hypothesized that this unidentified subunit might be a homologue of KVLQT1. To test this hypothesis, a *Xenopus* oocyte cDNA library (Clontech) was screened with a *KVLQT1* cDNA clone spanning the S3-S5 domains. A 1.6 kb partial clone (*XKVLQT1*, Figure 8A) was isolated. *XKYLQT1* is 88% identical at the amino acid level with the corresponding region of *KVLQT1* (Figure 8A). These data suggest that I_{sK} results from the coassembly of the XKVLQT1 and minK proteins.

It was concluded that KVLQT1 and hminK coassemble to form the cardiac I_{Ks} channel. Two delayed-rectifier K⁺ currents, I_{Kr} and I_{Ks}, modulate action-potential duration in cardiac myocytes (Li et al., 1996; Sanguinetti and Jurkiewicz, 1990). Previous studies have implicated dysfunction of I_{Kr} channels in long QT syndrome (Sanguinetti et al., 1995; Curran et al., 1995; Sanguinetti et al., 1996). The observation that *KVLQT1* mutations also cause this disorder (Wang et al., 1996), and the discovery that KVLQT1 forms part of the I_{Ks} channel, indicate that dysfunction of both cardiac delayed-rectifier K⁺ channels contribute to risk of sudden death from cardiac arrhythmia.

### EXAMPLE 12

### Cosegregation of KVLQT1 Missense Mutations with LQT in Six Large Families

To test the hypothesis that *KVLQT1* is *LQT1,* single-strand conformational polymorphism (SSCP) analyses were used to screen for functional mutations in affected members of K1532, the largest LQT family that showed linkage to chromosome 11. SSCP was carried out as previously described (Wang et al., 1995a; Wang et al., 1995b). Normal and aberrant SSCP products were isolated and sequenced directly as described (Wang and Keating, 1994) or subcloned into pBluescript (SK+) (Stratagene) using the T-vector method (Marchuk et al., 1990). When the latter method was used, several clones were sequenced by the dideoxy chain termination method using Sequenase^{™} Version 2.0 (United States Biochemicals, Inc.). Analyses were focused on the region between S2 and S6 since these regions might be important for KVLQT1 function. We designed oligonucleotide primers based on cDNA sequences and used these primers for cycle sequencing reactions with the *KVLQT1*-containing P1, 18B12 (Wang and Keating, 1994). These experiments defined intronic sequences flanking exons encoding S2-S6. Additional primers were then generated from these intronic sequences and used for SSCP analyses (Table 2).

SSCP analyses identified an anomalous conformer in the 70 affected members of K1532 (Figure 5). This aberrant conformer was not observed in the 147 unaffected members of this kindred or in genomic DNA from more than 200 unrelated control individuals (Q. Wang, unpublished results). The two-point LOD score for linkage between this anomaly and LQT was 14.19 at a recombination fraction of 0 (Table 1). No recombination was observed between *KVLQT1* and *LQT1*, indicating that these loci are completely linked. DNA sequence analyses of the normal and aberrant SSCP conformers revealed a single base substitution, a G to A transition, at the first nucleotide of codon Val-125 (Figure 5 and Table 3). This mutation results in a valine to methionine substitution in the predicted intracellular domain between S4 and S5.

To further test the hypothesis that mutations in *KVLQT1* cause LQT, DNA samples from affected members of five additional large LQT kindreds were studied. Linkage analyses with polymorphic markers from this region had shown that the disease phenotype was linked to chromosome 11 in these families (Q. Wang, unpublished results). Aberrant SSCP conformers were identified in affected members of K1723, K2605, K1807 (Figure 5), K161 and K162 (Q. Wang, unpublished results). The SSCP anomalies identified in K161 and K162 were identical to that observed in K1807 (Q. Wang, unpublished results). The aberrant SSCP conformer was not seen in unaffected members of these kindreds or in DNA samples from more than 200 unrelated control individuals (Figure 5; Q. Wang, unpublished results). The normal and aberrant conformers identified in each family were sequenced. The nucleotide change, coding effect, and location of each mutation are summarized in Table 3.

**Table 2. PCR primers used to define KVLQT1 mutations.**

| Primer | Sequence | Region amplified | SEQ.ID NO. |
|---|---|---|---|
| 1 | GAGATCGTGCTGGTGGTGTTCT | S2-S3 | 3 |
| 2 | CTTCCTGGTCTGGAAACCTGG | | 4 |
| | | | |
| 3 | CTCTTCCCTGGGGCCCTGGC | S3-S4 | 5 |
| 4 | TGCGGGGGAGCTTGTGGCACAG | | 6 |
| | | | |
| 5 | GGGCATCCGCTTCCTGCAGA | S4 | 7 |
| 6 | CTGGGCCCCTACCCTAACCC | | 8 |
| | | | |
| 7 | TCCTGGAGCCCGAACTGTGTGT | S5-Pore | 9 |
| 8 | TGTCCTGCCCACTCCTCAGCCT | | 10 |
| | | | |
| 9 | CCCCAGGACCCCAGCTGTCCAA | Pore-S6 | 11 |
| 10 | AGGCTGACCACTGTCCCTCT | | 12 |
| | | | |
| 11 | GCTGGCAGTGGCCTGTGTGGA | S6 | 13 |
| 12 | AACAGTGACCAAAATGACAGTGAC | | 14 |

**Table 3 Summary of KVLQT1 mutations**

| Codon | Nucleotide change | Coding effect | Mutation | Region | Kindred | Number of affected |
|---|---|---|---|---|---|---|
| 38-39 | ΔTCG | Deletion | F38W/G39Δ | S2 | K13216 | 1 |
| 49 | GCC to CCC | Missense | A49P | S2-S3 | K13119 | 1 |
| 60 | GGG to AGG | Missense | G60R | S2-S3 | K2557 | 3 |
| 61 | CGG to CAG | Missense | R61Q | S2-S3 | K15019 | 2 |
| 125 | GTG to ATG | Missense | V125M | S4-S5 | K1532 | 70 |
| 144 | CTC to TTC | Missense | L144F | S5 | K1777 | 2 |
| 177 | GGG to AGG | Missense | G177R | Pore | K20926 | 1 |
| 183 | ACC to AIC | Missense | T1831 | Pore | K20925 | 1 |
| 212 | GCG to GAG | Missense | A212E | S6 | K1723 | 6 |
| 212 | GCG to GAG | Missense | A212E | S6 | K2050 | 2 |
| 212 | GCG to GTG | Missense | A212V | S6 | K1807 | 6 |
| 212 | GCG to GTG | Missense | A212V | S6 | K161 | 18 |
| 212 | GCG to GTG | Missense | A212V | S6 | K162 | 18 |
| 212 | GCG to GTG | Missense | A212V | S6 | K163 | 3 |
| 212 | GCG to GTG | Missense | A212V | S6 | K164 | 2 |
| 216 | GGG to GAG | Missense | G216E | S6 | K2605 | 11 |

### EXAMPLE 13

### A KVLQT1 Intragenic Deletion and Nine Missense Mutations Associated with LQT in Small Families and Sporadic Cases

To identify additional LQT-associated mutations in *KVLQT1,* further SSCP analyses were performed for small kindreds and sporadic cases. SSCP revealed an aberrant conformer in kindred 13216 (Figure 6). Analyses of more than 200 unrelated control individuals failed to show this anomaly (Q. Wang, unpublished results). This aberrant conformer was cloned and sequenced, revealing a three base pair deletion encompassing codons 38 and 39. This mutation results in a phenylalanine to tryptophan substitution and deletion of a glycine in the putative S2 domain (Table 3).

Aberrant SSCP conformers were identified in affected members of nine additional kindreds: K1777, K20925, K13119, K20926, K15019 (Figure 6), K2050, K163 and K164 (Q. Wang, unpublished results). An aberrant SSCP conformer identified in K2050 was identical to that in K1723, and aberrant conformers identified in K163 and K164 were identical to that observed in K 1807. None of the aberrant conformers was identified in DNA samples from more than 200 control individuals (Q. Wang, unpublished results). In each case, the normal and aberrant conformers were sequenced. These data are shown in Figure 6 and summarized in Table 3. In total, *KVLQT1* mutations associated with LQT in 16 families or sporadic cases (Figure 7) were identified, providing strong molecular genetic evidence that mutations in *KVLQT1* cause the chromosome 11-linked form of LQT.

### EXAMPLE 14

### Generation of Polyclonal Antibody against KVLQT1

Segments of *KVLQT1* coding sequence are expressed as fusion protein in *E. coli.* The overexpressed protein is purified by gel elution and used to immunize rabbits and mice using a procedure similar to the one described by Harlow and Lane, 1988. This procedure has been shown to generate Abs against various other proteins (for example, see Kraemer et al., 1993).

Briefly, a stretch of *KVLQT1* coding sequence is cloned as a fusion protein in plasmid PET5A (Novagen, Inc., Madison, WI). After induction with IPTG, the overexpression of a fusion protein with the expected molecular weight is verified by SDS/PAGE. Fusion protein is purified from the gel by electroelution. Identification of the protein as the *KVLQT1* fusion product is verified by protein sequencing at the N-terminus. Next, the purified protein is used as immunogen in rabbits. Rabbits are immunized with 100 µg of the protein in complete Freund's adjuvant and boosted twice in 3 week intervals, first with 100 µg of immunogen in incomplete Freund's adjuvant followed by 100 µg of immunogen in PBS. Antibody containing serum is collected two weeks thereafter.

This procedure is repeated to generate antibodies against the mutant forms of the *KVLQT1* gene. These antibodies, in conjunction with antibodies to wild type *KVLQT1,* are used to detect the presence and the relative level of the mutant forms in various tissues and biological fluids.

### EXAMPLE 15

### Generation of Monoclonal Antibodies Specific for KVLQT1

Monoclonal antibodies are generated according to the following protocol. Mice are immunized with immunogen comprising intact *KVLQT1* or *KVLQT1* peptides (wild type or mutant) conjugated to keyhole limpet hemocyanin using glutaraldehyde or EDC as is well known.

The immunogen is mixed with an adjuvant. Each mouse receives four injections of 10 to 100 µg of immunogen and after the fourth injection blood samples are taken from the mice to determine if the serum contains antibody to the immunogen. Serum titer is determined by ELISA or RIA. Mice with sera indicating the presence of antibody to the immunogen are selected for hybridoma production.

Spleens are removed from immune mice and a single cell suspension is prepared (see Harlow and Lane, 1988). Cell fusions are performed essentially as described by Kohler and Milstein, 1975. Briefly, P3.65.3 myeloma cells (American Type Culture Collection, Rockville, MD) are fused with immune spleen cells using polyethylene glycol as described by Harlow and Lane, 1988. Cells are plated at a density of 2x10⁵ cells/well in 96 well tissue culture plates. Individual wells are examined for growth and the supernatants of wells with growth are tested for the presence of *KVLQT1* specific antibodies by ELISA or RIA using wild type or mutant *KVLQT1* target protein. Cells in positive wells are expanded and subcloned to establish and confirm monoclonality.

Clones with the desired specificities are expanded and grown as ascites in mice or in a hollow fiber system to produce sufficient quantities of antibody for characterization and assay development.

### EXAMPLE 16

### Sandwich Assay for KVLQT1

Monoclonal antibody is attached to a solid surface such as a plate, tube, bead or particle. Preferably, the antibody is attached to the well surface of a 96-well ELISA plate. 100 µl sample (e.g., serum, urine, tissue cytosol) containing the *KVLQT1* peptide/protein (wild-type or mutants) is added to the solid phase antibody. The sample is incubated for 2 hrs at room temperature. Next the sample fluid is decanted, and the solid phase is washed with buffer to remove unbound material. 100 µl of a second monoclonal antibody (to a different determinant on the *KVLQT1* peptide/protein) is added to the solid phase. This antibody is labeled with a detector molecule (e.g., 125-I, enzyme, fluorophore, or a chromophore) and the solid phase with the second antibody is incubated for two hrs at room temperature. The second antibody is decanted and the solid phase is washed with buffer to remove unbound material.

The amount of bound label, which is proportional to the amount of *KVLQT1* peptide/protein present in the sample, is quantitated. Separate assays are performed using monoclonal antibodies which are specific for the wild-type *KVLQT1* as well as monoclonal antibodies specific for each of the mutations identified in *KVLQT1.*

### EXAMPLE 17

### Assay to Screen Drugs Affecting the KVLQT1 and minK K⁺ Channel

With the knowledge that KVLQT1 and minK coassemble to form a cardiac I_{Ks} potassium channel, it is now possible to devise an assay to screen for drugs which will have an effect on this channel. The two genes, *KVLQT1* and *minK*, are cotransfected into oocytes or mammalian cells and coexpressed as described above. The cotransfection is performed using any combination of wild-type or specifically mutated *KVLQT1* and *minK*. When one of the genes used for cotransfection contains a mutation which causes LQT a change in the induced current is seen as compared to cotransfection with wild-type genes only. A drug candidate is added to the bathing solution of the transfected cells to test the effects of the drug candidates upon the induced current. A drug candidate which alters the induced current such that it is closer to the current seen with cells cotransfected with wild-type *KVLQT1* and *minK* is useful for treating LQT.

### LIST OF REFERENCES

Anderson, et al. (1980). Proc. Natl. Acad. Sci. USA 77,5399-5403.
Anderson, M.A. and Gusella, J.K. (1984). Use of cyclosporin A in establishing Epstein-Barr virus-transformed human lymphoblastoid cell lines. In Vitro 20, 856-858.
Antzelevitch, C. and Sicouri, S. (1994). Clinical relevance of cardiac arrhythmias generated by after depolarizations: Role of M cells in the generation of U waves, triggered activity and torsade de pointes. J. Am. Col. Card. 23, 259-277.
Attali, B., et al. (1993). Nature 365, 850-852.
Attwell, D. et al. (1979). Steady-state TTX-sensitive ("window") current in cardiac Purkinje fibres. Pflugers Arch. 379, 137-142.
Ausubel, F.M., et al. (1992). Current Protocols in Molecular Biology, (J. Wiley and Sons, N.Y.)
Balser, J.R. et al. (1991). Circ. Res. 69, 519-529.
Balser, J.R. et al. (1990). J. Gen. Physiol. 96, 835-863.
Bazett, H. C. (1920). An analysis of the time-relationships of electrocardiograms. Heart 7, 353-370.
Beaucage & Carruthers (1981). Tetra. Letts. 22, 1859-1862.
Benhorin, J. et al. (1993). Evidence of genetic heterogeneity in the long QT syndrome, Science 260, 1960-1962.
Berkner, et al. (1988). BioTechniques 6, 616-629.
Berkner (1992). Curr. Top. Microbiol. Immunol. 158, 39-61.
Bonner, T. I. et al. (1987). Identification of a family of muscarinic acetylcholine receptor genes. Science 237, 527-532.
Brandyopadhyay and Temin (1984). Mol. Cell. Biol. 4, 749-754.
Breakfield and Geller (1987). Mol. Neurobiol. 1, 337-371.
Brinster, et al. (1981). Cell 27, 223-231.
Bruggeman, A. et al. (1993). Ether-α-go-go encodes a voltage-gated channel permeable to K+ and Ca2+ and modulated by cAMP. Nature 365, 445-448.
Buchschacher and Panganiban (1992). J. Virol. 66, 2731-2739.
Buckler, A.J., et al. (1991). Exon amplification: a strategy to isolate mammalian genes based on RNA splicing. Proc. Natl. Acad. Sci. USA 88, 4005-4009.
Bum, T.C. et al. (1995). Increased exon trapping efficiencies through modifications to the pSPL3 splicing vector. Gene 161, 183-187.
Busch, A.E. et al. (1992). Science 255, 1705-1707.
Capecchi, M.R. (1989). Science 244, 1288.
Cariello (1988). Human Genetics 42, 726.
Chin, H., et al. (1991). A brain L-type calcium channel α1 subunit gene (CCHL1A2) maps to mouse chromosome 14 and human chromosome 3. Genomics 11, 914-919.
Church, D.M., et al. (1994). Isolation of genes from complex sources of mammalian genomic DNA using exon amplification. Nature Genet. 6, 98-104.
Conner, B.J., et al. (1983). Proc. Natl. Acad. Sci. USA 80, 278-282.
Constantini and Lacy (1981). Nature 294, 92-94.
Cotten, M. et al. (1990). Transferrin-polycation-mediated introduction of DNA into human leukemic cells: stimulation by agents that affect the survival of transfected DNA or modulate transferrin receptor levels. Proc. Natl. Acad. Sci. USA 87, 4033-4037.
Cotton, R.G. et al. (1988). Reactivity of cytosine and thymine in single-base-pair mismatches with hydroxylamine and osmium tetroxide and its application to the study of mutations. Proc. Natl. Acad. Sci. USA 85, 4397-4401.
Covarrubias, M. et al. (1991). Shaker, shal, shab, and shaw express independent K+ current systems. Neuron 7, 763-773.
Cui, J. et al. (1994). J. Gen. Physiol. 104, 87-105.
Curiel, et al. (1991a). Hum. Gene Ther. 3, 147-154.
Curiel, et al. (1991b). Proc. Natl. Acad. Sci. USA 88, 8850-8854.
Curran, M. E. et al. (1993). Locus heterogeneity of autosomal dominant long QT syndrome. J. Clin. Invest. 92, 799-803.
Curran, M.E. et al. (1995). A Molecular Basis for Cardiac Arrhythmia: HERG Mutations Cause Long QT Syndrome. Cell 80, 795-803.
Donehower, L.A., et al. (1992). Nature 356, 215.
Enhancers and Eukaryotic Gene Expression, Cold Spring Harbor Press, Cold Spring Harbor, New York (1983).
Felgner, et al. (1987). Proc. Natl. Acad. Sci. USA 84, 7413-7417.
Fiers, et al. (1978). Nature 273, 113.
Fink, et al. (1992). Hum. Gene Ther. 3, 11-19.
Finkelstein, J., et al. (1990). Genomics 7, 167-172.
Freese, et al. (1990). Biochem. Pharmacol. 40, 2189-2199.
Friedman, T. (1991). In Therapy for Genetic Diseases, T. Friedman, ed., Oxford University Press, pp. 105-121.
Gellens, M. et al. (1992). Primary structure and functional expression of the human cardiac tetrodotoxin-insensitive voltage-dependent sodium channel. Proc. Natl. Acad. Sci. USA 89, 554-558.
George, A.L. et al. (1995). Assignment of the human heart tetrodotoxin-resistant voltage-gated Na+ channel α-subunit gene (SCN5A) to band 3p21. Cytogenet. Cell. Genet. 68, 67-70.
Goldstein, S.A.N. and Miller, C. (1991). Neuron 7,403-408.
Gordon, et al. (1980). Proc. Natl. Acad. Sci. USA 77, 7380-7384.
Gorziglia and Kapikian (1992). J. Virol. 66, 4407-4412.
Graham and van der Eb (1973). Virology 52, 456-467.
Green, E.D. and Olson, M.V. (1990). Systematic screening of yeast artificial-chromosome libraries by use of the polymerase chain reaction. Proc. Natl. Acad. Sci. USA 87, 1213-1217.
Grompe, M., (1993). Nature Genetics 5, 111-117.
Grompe, M., et al., (1989). Proc. Natl. Acad. Sci. USA 86, 5855-5892.
Gyapay, G. et al. (1994). The 1993-94 Genethon human genetic linkage map. Nat. Genet. 7, 246-339.
Harlow & Lane (1988). Antibodies: A Laboratory Manual (Cold Spring Harbor Laboratory, Cold Spring Harbor, N.Y.).
Hasty, P., K., et al. (1991). Nature 350, 243.
Hausdorff, S.F. et al. (1991). Biochem. 30,3341-3346.
Heginbotham, L. et al. (1994). Biophys. J. 66, 1061-1067.
Helseth, et al. (1990). J. Virol. 64, 2416-2420.
Jablonski, E., et al. (1986). Nucl. Acids Res. 14, 6115-6128.
Jakoby, W.B. and Pastan, I.H. (eds.) (1979). Cell Culture. Methods in Enzymology, volume 58 (Academic Press, Inc., Harcourt Brace Jovanovich (New York)).
January, C. T. and Riddle, J. M. (1989). Early after depolarizations: Mechanism of induction and block. Circ. Res. 64, 977-990.
Jervell, A. and Lange-Nielson, F. (1957). Congenital deaf mutism, functional heart disease with prolongation of the QT interval, and sudden death. Am. Heart J. 54, 59-78.
Jiang, C. et al. (1994). Two long QT syndrome loci map to chromosomes 3 and 7 with evidence for further heterogeneity. Nat. Genet. 8, 141-147.
Johnson, et al. (1992). J. Virol. 66, 2952-2965.
Kaneda, et al. (1989). J. Biol. Chem. 264, 12126-12129.
Kanehisa (1984). Nucl. Acids Res. 12, 203-213.
Kannel, W. B. et al. (1987). Sudden death risk in overt coronary heart diseases: The Framingham study. Am. Heart J. 113, 799-804.
Keating, M. T. et al. (1991a). Linkage of a cardiac arrhythmia, the long QT syndrome, and the Harvey ras-1 gene. Science 252, 704-706.
Keating, M. T. et al. (1991b). Consistent linkage of the long QT syndrome to the Harvey ras-1 locus on chromosome 11. Am. J. Hum. Genet. 49, 1335-1339.
Kinszler, K.W., et al. (1991). Science 251, 1366-1370.
Koch, M. C. et al. (1992). The skeletal muscle chloride channel in dominant and recessive human myotonia. Science 257, 797-800.
Kohler, G. and Milstein, C. (1975). Nature 256, 495-497.
Kraemer, F.B. et al. (1993). J. Lipid Res. 34, 663-672.
Kubo, T., et al. (1988). FEBS Letts. 241, 119.
Kwiatowski, T.J. et al. (1990). Rapid identification of yeast artificial chromosome clones by matrix pooling and crude lysate PCR. Nucl. Acids Res. 17, 7191-7192.
Landegren, et al. (1988). Science 242, 229.
Lathrop, G. M. et al. (1985). Multilocus linkage analysis in humans: detection of linkage and estimation of recombination. Am. J. Hum. Genet. 37, 482-498.
Lesage, F., et al. (1993). Receptors and Channels 1, 143-152.
Li, G.-R. et al. (1996). Circ. Res. 78, 689-696.
Lim, et al. (1992). Circulation 83, 2007-2011.
MacKinnon, R. (1991). Determination of the subunit stoichiometry of a voltage-activated potassium channel. Nature 350, 232-235.
MacKinnon, R. et al. (1993). Functional stoichiometry of shaker potassium channel inactivation. Science 262, 757-759.
Madzak, et al. (1992). J. Gen. Virol. 73,1533-1536.
Magovcevic, I. et al. (1992). Regional localization of the human G protein ai2 (GNAI2) gene: Assignment to 3021 and a related sequence (GNAI2L) to 12p12-p13. Genomics 12, 125-129.
Maniatis, T. et al. (1982). Molecular Cloning: A Laboratory Manual (Cold Spring Harbor Laboratory, Cold Spring Harbor, N.Y.).
Mann and Baltimore (1985). J. Virol. 54, 401-407.
Marchuk, D. et al. (1990). Construction of T-vectors, a rapid and general system for direct cloning of unmodified PCR products. Nucl. Acids Res. 19, 1154.
Margolskee (1992). Curr. Top. Microbiol. Immunol. 158, 67-90.
Martin, R., et al. (1990). BioTechniques 9, 762-768.
Matsuura, H. et al. (1987). Pflugers Arch. 410, 596-603.
Matteucci, M.D. and Caruthers, M.H. (1981). J. Am. Chem. Soc. 103, 3185.
Matthews & Kricka (1988). Anal. Biochem. 169, 1.
Metzger, et al. (1988). Nature 334, 31-36.
Miller (1992). Curr. Top. Microbiol. Immunol. 158, 1-24.
Miller, et al. (1985). Mol. Cell. Biol. 5, 431-437.
Miller, et al. (1988). J. Virol. 62, 4337-4345.
Modrich, P. (1991). Ann. Rev. Genet. 25, 229-253.
Mombaerts, P., et al. (1992). Cell 68, 869.
Moss, A.J. and McDonald, J. (1970). Unilateral cervicothoracic sympathetic ganglionectomy for the treatment of long QT interval syndrome. N. Engl. J. Med. 285, 903-904.
Moss, A.J. et al. (1991). The long QT syndrome: prospective longitudinal study of 328 families. Circulation 84, 1136-1144.
Moss (1992). Curr. Top. Microbiol. Immunol. 158, 25-38.
Muzyczka (1992). Curr. Top. Microbiol. Immunol. 158, 97-123.
Nabel, et al. (1990). Science 249, 1285-1288.
Nabel (1992). Hum. Gene Ther. 3, 399-410.
"The Cardiac Arrhythmia Suppression Trial II Investigators. Effect of the antiarrhythmic agent moricizine on survival after myocardial infarction." (1992). N. Engl. J. Med. 327, 227.
Newton, C.R. et al. (1989). Nucl. Acids Res. 17, 2503-2516.
Nguyen, Q., et al. (1992). BioTechniques 13, 116-123.
Novack, et al. (1986). Proc. Natl. Acad. Sci. USA 83, 586.
Ochman, H. et al. (1988). Genetic application of an inverse polymerase chain reaction. Genetics 120,621-623.
Ohi, et al. (1990). Gene 89, 279-282.
Orita, M. et al. (1989). Detection of polymorphisms of human DNA by gel electrophoresis as single strand conformation polymorphisms. Proc. Natl. Acad. Sci. USA 86, 2766-2770.
Page, et al. (1990). J. Virol. 64, 5370-5276.
Pellicer, et al. (1980). Science 209, 1414-1422.
Petropoulos, et al. (1992). J. Virol. 66,3391-3397.
Philpott, K.L., et al. (1992). Science 256, 1448.
Pongs, O., et al. (1988). Shaker encodes a family of putative potassium channel proteins in the nervous system of Drosophila. EMBO J. 7, 1087-1095.
Quantin, et al. (1992). Proc. Natl. Acad. Sci. USA 89, 2581-2584.
Rano & Kidd (1989). Nucl. Acids Res. 17, 8392.
Rigby, P.W.J., et al. (1977). J. Mol. Biol. 113, 237-251.
Romano, C. (1965). Congenital cardiac arrhythmia. Lancet 1, 658-659.
Rosenfeld, et al. (1992). Cell 68, 143-155.
Roy, N. et al. (1994). Exclusion of HRAS from long QT locus. Nat. Genet. 8, 113-114.
Russell, M.W., et al. (1995). Localization of Romano-Ward long QT syndrome gene, LQT1, to the interval between tyrosine hydroxylase (TH) and D11S1349. Am. J. Hum. Genet. 57, 503-507.
Sambrook, J., et al. (1989). Molecular Cloning: A Laboratory Manual, 2nd Ed. (Cold Spring Harbor Laboratory, Cold Spring Harbor, N.Y.).
Sanguinetti, M.C. et al. (1996). Proc. Natl. Acad. Sci. USA 93, 2208-2212.
Sanguinetti, M.C. et al. (1995). Cell 81, 299-307.
Sanguinetti, M.C. and Jurkiewicz, N.K. (1990). J. Gen. Physiol. 96, 195-215.
Schott, J., et al. (1995). Mapping of a gene for long QT syndrome to chromosome 4q25-27. Am. J. Hum. Genet. 57, 1114-1122.
Schwartz, P. J. et al. (1975). The long QT syndrome. Am. Heart J. 109, 378-390.
Schwartz, P. J. et al. (1994). The long QT syndrome. In Cardiac Electrophysiology: from cell to bedside. D. P. Zipes and J. Jalife eds. (W.B. Sanders Company) pp.788-811.
Seino, S. et al. (1992). Assignment of the gene encoding the α1 subunit of the neuroendocrine/brain-type calcium channel (CACNL1A2) to human chromosome 3, band p14.3. Genomics 13, 1375-1377.
Sheffield, V.C., et al. (1989). Proc. Natl. Acad. Sci. USA 86, 232-236.
Sheffield, V.C., et al., (1991). Am. J. Hum. Genet. 49, 699-706.
Shenk, T.E. et al. (1975). Biochemical method for mapping mutational alterations in DNA with S 1 nuclease; the location of deletions and temperature-sensitive mutations in simian virus 40. Proc. Natl. Acad. Sci. USA 72,989-993.
Shimada, et al. (1991). J. Clin. Invest. 88, 1043-1047.
Shinkai, Y., et al. (1992). Cell 68, 855.
Smith, P.L. et al. (1996). Nature 379, 833-836.
Snouwaert, J.N., et al. (1992). Science 257, 1083.
Sorge, et al. (1984). Mol. Cell. Biol. 4, 1730-1737.
Spector, P.S. et al. (1996). J. Gen. Physiol. 107, 611-619.
Sternberg, N. (1990). Bacteriophage P1 cloning system for the isolation, amplification, and recovery of DNA fragments as large as 100 kilobase pairs. Proc. Natl. Acad. Sci. USA 87, 103-107.
Stewart, et al. (1992). Hum. Gene Ther. 3,267-275.
Stratford-Perricaudet, et al. (1990). Hum. Gene Ther. 1, 241-256.
Surawicz, B. (1989). Electrophysiologic substrate of torsade de pointes: Dispersion of repolarization or early after depolarizations? J. Am. Coll. Cardiol. 14, 172-184.
Takumi, T. et al. (1988). Science 242, 1042-1045.
Takumi, T., et al. (1991). J. Biol. Chem. 266,22192-22198.
Tanigami, A., et al. (1992). Mapping of 262 DNA markers into 24 intervals on human chromosome 11. Am. J. Hum. Genet. 50, 56-64.
Tokino, T., et al. (1991). Isolation and mapping of 62 new RFLP markers on human chromosome 11. Am. J. Hum. Genet. 48, 258-268.
Towbin, J. A. et al. (1994). Evidence of genetic heterogeneity in Romano-Ward long QT syndrome (LQTs): Analysis of 23 families. Circulation 90, 2635-2644.
Valancius, V. & Smithies, O. (1991). Mol. Cell Biol. 11, 1402.
Vincent, G. M. et al. (1992). The spectrum of symptoms and QT intervals in carriers of the gene for the long QT syndrome. N. Engl. J. Med. 327, 846-852.
Wagner, et al. (1991). Proc. Natl. Acad. Sci. USA 88, 4255-4259.
Wagner, et al. (1990). Proc. Natl. Acad. Sci. USA 87, 3410-3414.
Wang and Huang (1989). Biochemistry 28, 9508-9514.
Wang, K.-W. and Goldstein, S.A.N. (1995). Neuron 14, 1303-1309.
Wang, K., Tai, K. and Goldstein, S.A.N. (1996). Neuron 16, 571-577.
Wang, Q. and Keating, M. T. (1994). Isolation of P1 insert ends by direct sequencing. BioTechniques 17, 282-284.
Wang, Q. et al. (1995a). SCN5A Mutations Associated with an Inherited Cardiac Arrhythmia, Long QT Syndrome. Cell 80, 805-811.
Wang, Q., et al. (1995b). Cardiac sodium channel mutations in patients with long QT syndrome, an inherited cardiac arrhythmia. Hum. Mol. Genet. 4, 1603-1607.
Wang, Q., et al. (1996). Nature Genetics 12, 17-23.
Ward, O. C. (1964). A new familial cardiac syndrome in children. J. Ir. Med. Assoc. 54, 103-106.
Warmke, J. E. and Ganetzky, B. (1994). A family of potassium channel genes related to eag in Drosophila and mammals. Proc. Natl. Acad. Sci. 91, 3438-3442.
Wartell, R.M., et al. (1990). Nucl. Acids Res. 18, 2699-2705.
Weinstein, L.S. et al. (1988). Cloning and characterization of the human gene for the α-subunit of Gi2, a GTP-binding signal transduction protein. FEBS Letters 232, 333-340.
Wetmur & Davidson (1968). J. Mol. Biol. 31, 349-370.
White, M.B., et al. (1992). Genomics 12, 301-306.
Wilkinson, et al. (1992). Nucleic Acids Res. 20, 2233-2239.
Willich, S. N. et al. (1987). Circadian variation in the incidence of sudden cardiac death in the Framingham heart study population. Am. J. Cardiol. 60, 801-806.
Wolff, et al. (1990). Science 247, 1465-1468.
Wolff, et al. (1991). BioTechniques 11, 474-485.
Wu, et al. (1989). J. Biol. Chem. 264, 16985-16987.
Wu, et al. (1991). J. Biol. Chem. 266, 4338-14342.
Wymore, R.S., et al. (1994). Genomic organization, nucleotide sequence, biophysical properties, and localization of the voltage-gated K+ channel gene KCN4A/Kv1.4 to mouse chromosome 2/human 11p14 and mapping of KCNC1/Kv3.1 to mouse 7/human 11p14.3-p15.2 and KCNA1/Kv1.1 to human 12p13. Genomics 20, 191202.
Zenke, et al. (1990). Proc. Natl. Acad. Sci. USA 87, 3655-3659.
Zipes, D.P. (1987). Proarrhythmic effects of antiarrhythmic drugs. Am. J. Cardiol. 59, 26E-31E.
European Patent Application Publication No. 0332435.
EPO Publication No. 225,807.
Hitzeman et al., EP 73,675A.

### Patents and Patent Applications:

PCT published application WO 93/07282.
U.S. Patent 4,376,110.
U.S. Patent 4,486,530.
U.S. Patent 4,868,105.
U.S. Patent 5,252,479.

## Claims

1. A method of assessing a risk in a human subject for long QT syndrome which comprises screening said subject for a mutation in a KVLQT1 gene by comparing the sequence of the KVLQT1 gene or its expression products isolated from a tissue sample of said subject with a wild-type KVLQT1 gene or its expression products, wherein the wild-type KVLQT1 gene has the sequence of SEQ ID NO:15, and wherein a mutation in the sequence of the subject is indicative of a risk for long QT syndrome.

2. The method of claim 1 wherein said expression product is selected from the group consisting of mRNA of the KVLQT1 gene and a KVLQT1 polypeptide encoded by the KVLQT1 gene.

3. The method of any one of claims 1 to 2 wherein the DNA or RNA encoding amino acids 38-39 of the KVQLT1 polypeptide of SEQ ID NO:16 or amino acids 38-39 of the KVLQT1 polypeptide of SEQ ID NO:16 are compared.

4. The method of any one of claims 1 to 3 wherein the DNA or RNA is compared for the mutations shown in Table 3.

## Patentansprüche

1. Verfahren zur Risikobewertung bei einem menschlichen Subjekt für das Long-QT-Syndrom, welches das Screening des Subjekts auf eine Mutation eines KVLQT1-Gens umfasst, durch den Vergleich der Sequenz des KVLQT1-Gens oder seiner Expressionsprodukte, die aus einer Gewebeprobe des Subjekts isoliert wurden, mit einem Wild-Typ-KVLQT1-Gen oder seinen Expressionsprodukten, wobei das Wild-Typ-KVLQT1-Gen die Sequenz der SEQ ID NO:15 aufweist, und wobei eine Mutation in der Sequenz des Subjekts auf ein Risiko für das Long-QT-Syndrom hinweist.

2. Verfahren nach Anspruch 1, wobei das Expressionsprodukt ausgewählt ist aus der Gruppe, bestehend aus mRNA des KVLQT1-Gens und einem KVLQT1-Polypeptid, das durch das KVLQT1-Gen codiert wird.

3. Verfahren nach einem der Ansprüche 1 bis 2, wobei die DNA oder RNA, die für die Aminosäuren 38-39 des KVLQT1-Polypeptids der SEQ ID NO:16 kodiert, bzw. die Aminosäuren 38-39 des KVLQT1-Polypeptids der SEQ ID NO:16 verglichen werden.

4. Verfahren nach einem der Ansprüche 1 bis 3, wobei die DNA oder RNA bezüglich der in Tabelle 3 aufgeführten Mutationen verglichen wird.

## Revendications

1. Méthode pour l'évaluation d'un risque de présence du syndrome du QT long dans un sujet humain, comprenant le criblage dudit sujet à la recherche d'une mutation dans un gène KVLQT1 par comparaison de la séquence du gène KVLQT1 ou ses produits d'expression isolés d'un échantillon de tissu dudit sujet avec un gène KVLQT1 de type sauvage ou ses produits d'expression, dans laquelle le gène KVLQT1 de type sauvage a la séquence de SEQ ID NO:15, et dans laquelle une mutation dans la séquence du sujet est un indice de risque de présence du syndrome du QT long.

2. Méthode selon la revendication 1, dans laquelle ledit produit d'expression est sélectionné dans le groupe se composant de mRNA du gène KVLQT1 et d'un polypeptide de KVLQT1 encodé par le gène KVLQT1.

3. Méthode selon l'une quelconque des revendications 1 à 2, dans laquelle les aminoacides 38-39 du polypeptide de KVQLT1 de SEQ ID NO:16 ou les aminoacides 38-39 du polypeptide de KVLQT1 de SEQ ID NO:16 qui encodent l'ADN ou l'ARN sont comparés.

4. Méthode selon l'une quelconque des revendications 1 à 3, dans laquelle l'ADN ou l'ARN est comparé pour ce qui est des mutations indiquées à la Table 3.


### SEQUENCE LISTING

(1) GENERAL INFORMATION:
   (i) APPLICANT: University of Utah Research Foundation
   (ii) TITLE OF INVENTION: KVLQT1 - A LONG QT SYNDROME GENE WHICH ENCODES KVLQT1 WHICH COASSEMBLES WITH minK TO FORM CARDIAC I_{Ks} POTASSIUM CHANNELS
   (iii) NUMBER OF SEQUENCES: 26
   (iv) CORRESPONDENCE ADDRESS:
      (A) ADDRESSEE: Venable, Baetjer, Howard & Civiletti, LLP
      (B) STREET: 1201 New York Avenue, N.W., Suite 1000
      (C) CITY: Washington
      (D) STATE: DC
      (E) COUNTRY: U.S.A.
      (F) ZIP: 20005
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Word for Windows 6.0
   (vi) CURRENT APPLICATION DATA:
      (A) APPLICATION NUMBER: WO
      (B) FILING DATE: 20-DEC-1996
      (C) CLASSIFICATION:
   (viii) ATTORNEY/AGENT INFORMATION:
      (A) NAME: Ihnen, Jeffrey L.
      (B) REGISTRATION NUMBER: 28,957
      (C) REFERENCE/DOCKET NUMBER: 19780-116871-WO
   (ix) TELECOMMUNICATION INFORMATION:
      (A) TELEPHONE: 202-962-4800
      (B) TELEFAX: 202-962-8300
(2) INFORMATION FOR SEQ ID NO:1:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic oligomer"
   (xi) SEQUENCE DESCRIPTION: SEQ ID NO:1:
      CAGATCCTGA GGATGCT 17
(2) INFORMATION FOR SEQ ID NO:2:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic oligomer"
   (xi) SEQUENCE DESCRIPTION: SEQ ID NO:2:
      GTACCTGGCT GAGAAGG 17
(2) INFORMATION FOR SEQ ID NO:3:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:3:
      GAGATCGTGC TGGTGGTGTT CT 22
(2) INFORMATION FOR SEQ ID NO:4:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:4:
      CTTCCTGGTC TGGAAACCTG G 21
(2) INFORMATION FOR SEQ ID NO:5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:5:
      CTCTTCCCTG GGGCCCTGGC 20
(2) INFORMATION FOR SEQ ID NO:6:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:6:
      TGCGGGGGAG CTTGTGGCAC AG 22
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:7:
      GGGCATCCGC TTCCTGCAGA 20
(2) INFORMATION FOR SEQ ID NO:8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:8:
      CTGGGCCCCT ACCCTAACCC 20
(2) INFORMATION FOR SEQ ID NO:9:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:9:
      TCCTGGAGCC CGAACTGTGT GT 22
(2) INFORMATION FOR SEQ ID NO:10:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:10:
      TGTCCTGCCC ACTCCTCAGC CT 22
(2) INFORMATION FOR SEQ ID NO:11:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:11:
      CCCCAGGACC CCAGCTGTCC AA 22
(2) INFORMATION FOR SEQ ID NO:12:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:12:
      AGGCTGACCA CTGTCCCTCT 20
(2) INFORMATION FOR SEQ ID NO:13:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:13:
      GCTGGCAGTG GCCTGTGTGG A 21
(2) INFORMATION FOR SEQ ID NO:14:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer"
   (xi) SEQUENCE DESCRIPTION: SEQ ID NO:14:
      AACAGTGACC AAAATGACAG TGAC 24
(2) INFORMATION FOR SEQ ID NO:15:
   (i) SEQUENCE CHARACTERISTICS:
      (A) LENGTH: 2633 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION: 2..1642
   (xi) SEQUENCE DESCRIPTION: SEQ ID NO:15:
   C TTC CTC ATC GTC CTG GTC TGC CTC ATC TTC AGC GTG CTG TCC ACC        46
     Phe Leu Ile Val Leu Val Cys Leu Ile Phe Ser Val Leu Ser Thr
      1               5                  10                  15
   ATC GAG CAG TAT GCC GCC CTG GCC ACG GGG ACT CTC TTC TGG ATG GAG       94
   Ile Glu Gln Tyr Ala Ala Leu Ala Thr Gly Thr Leu Phe Trp Met Glu
                   20                  25                  30
   ATC GTG CTG GTG GTG TTC TTC GGG ACG GAG TAC GTG GTC CGC CTC TGG      142
   Ile Val Leu Val Val Phe Phe Gly Thr Glu Tyr Val Val Arg Leu Trp
               35                  40                  45
   TCC GCC GGC TGC CGC AGC AAG TAC GTG GGC CTC TGG GGG CGG CTG CGC      190
   Ser Ala Gly Cys Arg Ser Lys Tyr Val Gly Leu Trp Gly Arg Leu Arg
           50                  55                  60
   TTT GCC CGG AAG CCC ATT TCC ATC ATC GAC CTC ATC GTG GTC GTG GCC      238
   Phe Ala Arg Lys Pro Ile Ser Ile Ile Asp Leu Ile Val Val Val Ala
       65                  70                  75
   TCC ATG GTG GTC CTC TGC GTG GGC TCC AAG GGG CAG GTG TTT GCC ACG      286
   Ser Met Val Val Leu Cys Val Gly Ser Lys Gly Gln Val Phe Ala Thr
   80                  85                  90                  95
   TCG GCC ATC AGG GGC ATC CGC TTC CTG CAG ATC CTG AGG ATG CTA CAC      334
   Ser Ala Ile Arg Gly Ile Arg Phe Leu Gln Ile Leu Arg Met Leu His
                   100                 105                 110
   GTC GAC CGC CAG GGA GGC ACC TGG AGG CTC CTG GGC TCC GTG GTC TTC      382
   Val Asp Arg Gln Gly Gly Thr Trp Arg Leu Leu Gly Ser Val Val Phe
               115                 120                 125
   ATC CAC CGC CAG GAG CTG ATA ACC ACC CTG TAC ATC GGC TTC CTG GGC      430
   Ile His Arg Gln Glu Leu Ile Thr Thr Leu Tyr Ile Gly Phe Leu Gly
           130                 135                 140
   CTC ATC TTC TCC TCG TAC TTT GTG TAC CTG GCT GAG AAG GAC GCG GTG      478
   Leu Ile Phe Ser Ser Tyr Phe Val Tyr Leu Ala Glu Lys Asp Ala Val
       145                 150                 155
   AAC GAG TCA GGC CGC GTG GAG TTC GGC AGC TAC GCA GAT GCG CTG TGG      526
   Asn Glu Ser Gly Arg Val Glu Phe Gly Ser Tyr Ala Asp Ala Leu Trp
   160                 165                 170                 175
   TGG GGG GTG GTC ACA GTC ACC ACC ATC GGC TAT GGG GAC AAG GTG CCC      574
   Trp Gly Val Val Thr Val Thr Thr Ile Gly Tyr Gly Asp Lys Val Pro
                   180                 185                 190
   CAG ACG TGG GTC GGG AAG ACC ATC GCC TCC TGC TTC TCT GTC TTT GCC      622
   Gln Thr Trp Val Gly Lys Thr Ile Ala Ser Cys Phe Ser Val Phe Ala
               195                 200                 205
   ATC TCC TTC TTT GCG CTC CCA GCG GGG ATT CTT GGC TCG GGG TTT GCC      670
   Ile Ser Phe Phe Ala Leu Pro Ala Gly Ile Leu Gly Ser Gly Phe Ala
           210                 215                 220
   CTG AAG GTG CAG CAG AAG CAG AGG CAG AAG CAC TTC AAC CGG CAG ATC      718
   Leu Lys Val Gln Gln Lys Gln Arg Gln Lys His Phe Asn Arg Gln Ile
       225                 230                 235
   CCG GCG GCA GCC TCA CTC ATT CAG ACC GCA TGG AGG TGC TAT GCT GCC      766
   Pro Ala Ala Ala Ser Leu Ile Gln Thr Ala Trp Arg Cys Tyr Ala Ala
   240                 245                 250                 255
   GAG AAC CCC GAC TCC TCC ACC TGG AAG ATC TAC ATC CGG AAG GCC CCC        814
   Glu Asn Pro Asp Ser Ser Thr Trp Lys Ile Tyr Ile Arg Lys Ala Pro
                   260                 265                 270
   CGG AGC CAC ACT CTG CTG TCA CCC AGC CCC AAA CCC AAG AAG TCT GTG        862
   Arg Ser His Thr Leu Leu Ser Pro Ser Pro Lys Pro Lys Lys Ser Val
               275                 280                 285
   GTG GTA AAG AAA AAA AAG TTC AAG CTG GAC AAA GAC AAT GGG GTG ACT        910
   Val Val Lys Lys Lys Lys Phe Lys Leu Asp Lys Asp Asn Gly Val Thr
           290                 295                 300
   CCT GGA GAG AAG ATG CTC ACA GTC CCC CAT ATC ACG TGC GAC CCC CCA        958
   Pro Gly Glu Lys Met Leu Thr Val Pro His Ile Thr Cys Asp Pro Pro
       305                 310                 315
   GAA GAG CGG CGG CTG GAC CAC TTC TCT GTC GAC GGC TAT GAC AGT TCT       1006
   Glu Glu Arg Arg Leu Asp His Phe Ser Val Asp Gly Tyr Asp Ser Ser
   320                 325                 330                 335
   GTA AGG AAG AGC CCA ACA CTG CTG GAA GTG AGC ATG CCC CAT TTC ATG       1054
   Val Arg Lys Ser Pro Thr Leu Leu Glu Val Ser Met Pro His Phe Met
                   340                 345                 350
   AGA ACC AAC AGC TTC GCC GAG GAC CTG GAC CTG GAA GGG GAG ACT CTG       1102
   Arg Thr Asn Ser Phe Ala Glu Asp Leu Asp Leu Glu Gly Glu Thr Leu
               355                 360                 365
   CTG ACA CCC ATC ACC CAC ATC TCA CAG CTG CGG GAA CAC CAT CGG GCC       1150
   Leu Thr Pro Ile Thr His Ile Ser Gln Leu Arg Glu His His Arg Ala
           370                 375                 380
   ACC ATT AAG GTC ATT CGA CGC ATG CAG TAC TTT GTG GCC AAG AAG AAA       1198
   Thr Ile Lys Val Ile Arg Arg Met Gln Tyr Phe Val Ala Lys Lys Lys
       385                 390                 395
   TTC CAG CAA GCG CGG AAG CCT TAC GAT GTG CGG GAC GTC ATT GAG CAG       1246
   Phe Gln Gln Ala Arg Lys Pro Tyr Asp Val Arg Asp Val Ile Glu Gln
   400                 405                 410                 415
   TAC TCG CAG GGC CAC CTC AAC CTC ATG GTG CGC ATC AAG GAG CTG CAG       1294
   Tyr Ser Gln Gly His Leu Asn Leu Met Val Arg Ile Lys Glu Leu Gln
                   420                 425                 430
   AGG AGG CTG GAC CAG TCC ATT GGG AAG CCC TCA CTG TTC ATC TCC GTC       1342
   Arg Arg Leu Asp Gln Ser Ile Gly Lys Pro Ser Leu Phe Ile Ser Val
               435                 440                 445
   TCA GAA AAG AGC AAG GAT CGC GGC AGC AAC ACG ATC GGC GCC CGC CTG       1390
   Ser Glu Lys Ser Lys Asp Arg Gly Ser Asn Thr Ile Gly Ala Arg Leu
           450                 455                 460
   AAC CGA GTA GAA GAC AAG GTG ACG CAG CTG GAC CAG AGG CTG GCA CTC       1438
   Asn Arg Val Glu Asp Lys Val Thr Gln Leu Asp Gln Arg Leu Ala Leu
       465                 470                 475
   ATC ACC GAC ATG CTT CAC CAG CTG CTC TCC TTG CAC GGT GGC AGC ACC       1486
   Ile Thr Asp Met Leu His Gln Leu Leu Ser Leu His Gly Gly Ser Thr
   480                 485                 490                 495
   CCC GGC AGC GGC GGC CCC CCC AGA GAG GGC GGG GCC CAC ATC ACC CAG        1534
   Pro Gly Ser Gly Gly Pro Pro Arg Glu Gly Gly Ala His Ile Thr Gln
                   500                     505                     510
   CCC TGC GGC AGT GGC GGC TCC GTC GAC CCT GAG CTC TTC CTG CCC AGC        1582
   Pro Cys Gly Ser Gly Gly Ser Val Asp Pro Glu Leu Phe Leu Pro Ser
               515                     520                     525
   AAC ACC CTG CCC ACC TAC GAG CAG CTG ACC GTG CCC AGG AGG GGC CCC        1630
   Asn Thr Leu Pro Thr Tyr Glu Gln Leu Thr Val Pro Arg Arg Gly Pro
           530                     535                     540
   GAT GAG GGG TCC TGAGGAGGGG ATGGGGCTGG GGGATGGGCC TGAGTGAGAG            1682
   Asp Glu Gly Ser
       545
   GGGAGGCCAA GAGTGGCCCC ACCTGGCCCT CTCTGAAGGA GGCCACCTCC TAAAAGGCCC      1742
   AGAGAGAAGA GCCCCACTCT CAGAGGCCCC AATACCCCAT GGACCATGCT GTCTGGCACA      1802
   GCCTGCACTT GGGGGCTCAG CAAGGCCACC TCTTCCTGGC CGGTGTGGGG GCCCCGTCTC      1862
   AGGTCTGAGT TGTTACCCCA AGCGCCCTGG CCCCCACATG GTGATGTTGA CATCACTGGC      1922
   ATGGTGGTTG GGACCCAGTG GCAGGGCACA GGGCCTGGCC CATGTATGGC CAGGAAGTAG      1982
   CACAGGCTGA GTGCAGGCCC ACCCTGCTTG GCCCAGGGGG CTTCCTGAGG GGAGACAGAG      2042
   CAACCCCTGG ACCCCAGCCT CAAATCCAGG ACCCTGCCAG GCACAGGCAG GGCAGGACCA      2102
   GCCCACGCTG ACTACAGGGC CACCGGCAAT AAAAGCCCAG GAGCCCATTT GGAGGGCCTG      2162
   GGCCTGGCTC CCTCACTCTC AGGAAATGCT GACCCATGGG CAGGAGACTG TGGAGACTGC      2222
   TCCTGAGCCC CCAGCTTCCA GCAGGAGGGA CAGTCTCACC ATTTCCCCAG GGCACGTGGT      2282
   TGAGTGGGGG GAACGCCCAC TTCCCTGGGT TAGACTGCCA GCTCTTCCTA GCTGGAGAGG      2342
   AGCCCTGCCT CTCCGCCCCT GAGCCCACTG TGCGTGGGGC TCCCGCCTCC AACCCCTCGC      2402
   CCAGTCCCAG CAGCCAGCCA AACACACAGA AGGGGACTGC CACCTCCCCT TGCCAGCTGC      2462
   TGAGCCGCAG AGAAGTGACG GTTCCTACAC AGGACAGGGG TTCCTTCTGG GCATTACATC      2522
   GCATAGAAAT CAATAATTTG TGGTGATTTG GATCTGTGTT TTAATGAGTT TCACAGTGTG      2582
   ATTTTGATTA TTAATTGTGC AAGCTTTTCC TAATAAACGT GGAGAATCAC A               2633
(2) INFORMATION FOR SEQ ID NO:16:
   (i) SEQUENCE CHARACTERISTICS:
      (A) LENGTH: 547 amino acids
      (B) TYPE: amino acid
      (D) TOPOLOGY: linear
   (ii) MOLECULE TYPE: protein
   (xi) SEQUENCE DESCRIPTION: SEQ ID NO:16:
   Phe Leu Ile Val Leu Val Cys Leu Ile Phe Ser Val Leu Ser Thr Ile
    1               5                  10                  15
   Glu Gln Tyr Ala Ala Leu Ala Thr Gly Thr Leu Phe Trp Met Glu Ile
               20                  25                  30
   Val Leu Val Val Phe Phe Gly Thr Glu Tyr Val Val Arg Leu Trp Ser
           35                  40                  45
   Ala Gly Cys Arg Ser Lys Tyr Val Gly Leu Trp Gly Arg Leu Arg Phe
       50                  55                  60
   Ala Arg Lys Pro Ile Ser Ile Ile Asp Leu Ile Val Val Val Ala Ser
   65                  70                  75                  80
   Met Val Val Leu Cys Val Gly Ser Lys Gly Gln Val Phe Ala Thr Ser
                   85                  90                  95
   Ala Ile Arg Gly Ile Arg Phe Leu Gln Ile Leu Arg Met Leu His Val
               100                 105                 110
   Asp Arg Gln Gly Gly Thr Trp Arg Leu Leu Gly Ser Val Val Phe Ile
           115                 120                 125
   His Arg Gln Glu Leu Ile Thr Thr Leu Tyr Ile Gly Phe Leu Gly Leu
       130                 135                 140
   Ile Phe Ser Ser Tyr Phe Val Tyr Leu Ala Glu Lys Asp Ala Val Asn
   145                 150                 155                 160
   Glu Ser Gly Arg Val Glu Phe Gly Ser Tyr Ala Asp Ala Leu Trp Trp
                   165                 170                 175
   Gly Val Val Thr Val Thr Thr Ile Gly Tyr Gly Asp Lys Val Pro Gln
               180                 185                 190
   Thr Trp Val Gly Lys Thr Ile Ala Ser Cys Phe Ser Val Phe Ala Ile
           195                 200                 205
   Ser Phe Phe Ala Leu Pro Ala Gly Ile Leu Gly Ser Gly Phe Ala Leu
       210                 215                 220
   Lys Val Gln Gln Lys Gln Arg Gln Lys His Phe Asn Arg Gln Ile Pro
   225                 230                 235                 240
   Ala Ala Ala Ser Leu Ile Gln Thr Ala Trp Arg Cys Tyr Ala Ala Glu
                   245                 250                 255
   Asn Pro Asp Ser Ser Thr Trp Lys Ile Tyr Ile Arg Lys Ala Pro Arg
               260                 265                 270
   Ser His Thr Leu Leu Ser Pro Ser Pro Lys Pro Lys Lys Ser Val Val
           275                 280                 285
   Val Lys Lys Lys Lys Phe Lys Leu Asp Lys Asp Asn Gly Val Thr Pro
       290                 295                 300
   Gly Glu Lys Met Leu Thr Val Pro His Ile Thr Cys Asp Pro Pro Glu
   305                 310                 315                 320
   Glu Arg Arg Leu Asp His Phe Ser Val Asp Gly Tyr Asp Ser Ser Val
                   325                 330                 335
   Arg Lys Ser Pro Thr Leu Leu Glu Val Ser Met Pro His Phe Met Arg
               340                 345                 350
   Thr Asn Ser Phe Ala Glu Asp Leu Asp Leu Glu Gly Glu Thr Leu Leu
           355                 360                 365
   Thr Pro Ile Thr His Ile Ser Gln Leu Arg Glu His His Arg Ala Thr
       370                 375                 380
   Ile Lys Val Ile Arg Arg Met Gln Tyr Phe Val Ala Lys Lys Lys Phe
   385                 390                 395                 400
   Gln Gln Ala Arg Lys Pro Tyr Asp Val Arg Asp Val Ile Glu Gln Tyr
                   405                 410                 415
   Ser Gln Gly His Leu Asn Leu Met Val Arg Ile Lys Glu Leu Gln Arg
               420                 425                 430
   Arg Leu Asp Gln Ser Ile Gly Lys Pro Ser Leu Phe Ile Ser Val Ser
           435                 440                 445
   Glu Lys Ser Lys Asp Arg Gly Ser Asn Thr Ile Gly Ala Arg Leu Asn
       450                 455                 460
   Arg Val Glu Asp Lys Val Thr Gln Leu Asp Gln Arg Leu Ala Leu Ile
   465                 470                 475                 480
   Thr Asp Met Leu His Gln Leu Leu Ser Leu His Gly Gly Ser Thr Pro
                   485                 490                 495
   Gly Ser Gly Gly Pro Pro Arg Glu Gly Gly Ala His Ile Thr Gln Pro
               500                 505                 510
   Cys Gly Ser Gly Gly Ser Val Asp Pro Glu Leu Phe Leu Pro Ser Asn
           515                 520                 525
   Thr Leu Pro Thr Tyr Glu Gln Leu Thr Val Pro Arg Arg Gly Pro Asp
       530                 535                 540
   Glu Gly Ser
   545
(2) INFORMATION FOR SEQ ID NO:17:
   (i) SEQUENCE CHARACTERISTICS:
      (A) LENGTH: 26 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (xi) SEQUENCE DESCRIPTION: SEQ ID NO:17:
   Phe Leu Ile Val Leu Val Cys Leu Ile Phe Ser Val Leu Ser Thr Ile
   1               5                   10                  15
   Glu Gln Tyr Ala Ala Leu Ala Thr Gly Thr
               20                  25
(2) INFORMATION FOR SEQ ID NO:18:
   (i) SEQUENCE CHARACTERISTICS:
      (A) LENGTH: 61 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (xi) SEQUENCE DESCRIPTION: SEQ ID NO:18:
   Leu Phe Trp Met Glu Ile Val Leu Val Val Phe Phe Gly Thr Glu Tyr
   1               5                   10                  15
   Val Val Arg Leu Trp Ser Ala Gly Cys Arg Ser Lys Tyr Val Gly Leu
               20                  25                  30
   Trp Gly Arg Leu Arg Phe Ala Arg Lys Pro Ile Ser Ile Ile Asp Leu
           35                  40                  45
   Ile Val Val Val Ala Ser Met Val Val Leu Cys Val Gly
       50                  55                  60
(2) INFORMATION FOR SEQ ID NO:19:
   (i) SEQUENCE CHARACTERISTICS:
      (A) LENGTH: 137 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (xi) SEQUENCE DESCRIPTION: SEQ ID NO:19:
   Ser Lys Gly Gln Val Phe Ala Thr Ser Ala Ile Arg Gly Ile Arg Phe
   1               5                   10                  15
   Leu Gln Ile Leu Arg Met Leu His Val Asp Arg Gln Gly Gly Thr Trp
               20                  25                  30
   Arg Leu Leu Gly Ser Val Val Phe Ile His Arg Gln Glu Leu Ile Thr
           35                  40                  45
   Thr Leu Tyr Ile Gly Phe Leu Gly Leu Ile Phe Ser Ser Tyr Phe Val
       50                  55                  60
   Tyr Leu Ala Glu Lys Asp Ala Val Asn Glu Ser Gly Arg Val Glu Phe
   65                  70                  75                  80
   Gly Ser Tyr Ala Asp Ala Leu Trp Trp Gly Val Val Thr Val Thr Thr
                   85                  90                  95
   Ile Gly Tyr Gly Asp Lys Val Pro Gln Thr Trp Val Gly Lys Thr Ile
               100                 105                 110
   Ala Ser Cys Phe Ser Val Phe Ala Ile Ser Phe Phe Ala Leu Pro Ala
           115                 120                 125
   Gly Ile Leu Gly Ser Gly Phe Ala Leu
       130                 135
(2) INFORMATION FOR SEQ ID NO:20:
   (i) SEQUENCE CHARACTERISTICS:
      (A) LENGTH: 66 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (xi) SEQUENCE DESCRIPTION: SEQ ID NO:20:
   Ile Leu Leu Ser Ile Val Ile Phe Cys Leu Glu Thr Leu Pro Glu Phe
   1               5                   10                  15
   Lys His Tyr Lys Val Phe Asn Thr Thr Thr Asn Gly Thr Lys Ile Glu
               20                  25                  30
   Glu Asp Glu Val Pro Asp Ile Thr Asp Pro Phe Phe Leu Ile Glu Thr
           35                  40                  45
   Leu Cys Ile Ile Trp Phe Thr Phe Glu Leu Thr Val Arg Phe Leu Ala
       50                  55                  60
   Cys Pro
   65
(2) INFORMATION FOR SEQ ID NO:21:
   (i) SEQUENCE CHARACTERISTICS:
      (A) LENGTH: 123 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (xi) SEQUENCE DESCRIPTION: SEQ ID NO:21:
   Asn Lys Leu Asn Phe Cys Arg Asp Val Met Asn Val Ile Asp Ile Ile
   1               5                   10                  15
   Ala Ile Ile Pro Tyr Phe Ile Thr Leu Ala Thr Val Val Ala Glu Glu
               20                  25                  30
   Glu Asp Thr Leu Asn Leu Pro Lys Ala Pro Val Ser Pro Gln Asp Lys
           35                  40                  45
   Ser Ser Asn Gln Ala Met Ser Leu Ala Ile Leu Arg Val Ile Arg Leu
       50                  55                  60
   Val Arg Val Phe Arg Ile Phe Lys Leu Ser Arg His Ser Lys Gly Leu
   65                  70                  75                  80
   Gln Ile Leu Gly Arg Thr Leu Lys Ala Ser Met Arg Glu Leu Gly Leu
                   85                  90                  95
   Leu Ile Phe Phe Leu Phe Ile Gly Val Val Leu Phe Ser Ser Ala Val
               100                 105                 110
   Tyr Phe Ala Glu Ala Gly Ser Glu Asn Ser Phe
           115                 120
(2) INFORMATION FOR SEQ ID NO:22:
   (i) SEQUENCE CHARACTERISTICS:
      (A) LENGTH: 58 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (xi) SEQUENCE DESCRIPTION: SEQ ID NO:22:
   Phe Lys Ser Ile Pro Asp Ala Phe Trp Trp Ala Val Val Thr Met Thr
   1               5                   10                  15
   Thr Val Gly Tyr Gly Asp Met Thr Pro Val Gly Phe Trp Gly Lys Ile
               20                  25                  30
   Val Gly Ser Leu Cys Val Val Ala Gly Val Leu Thr Ile Ala Leu Pro
           35                  40                  45
   Val Pro Val Ile Val Ser Asn Phe Asn Tyr
       50                  55
(2) INFORMATION FOR SEQ ID NO:23:
   (i) SEQUENCE CHARACTERISTICS:
      (A) LENGTH: 376 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (vi) ORIGINAL SOURCE:
      (A) ORGANISM: Xenopus
   (xi) SEQUENCE DESCRIPTION: SEQ ID NO:23:
   Met Asn Glu Asn Ala Ile Asn Ser Leu Tyr Glu Ala Ile Pro Leu Pro
   1 5 10 15
   Gln Asp Gly Ser Ser Asn Gly Gln Arg Gln Glu Asp Arg Gln Ala Asn
   20 25 30
   Ser Phe Glu Leu Lys Arg Glu Thr Leu Val Ala Thr Asp Pro Pro Arg
   35 40 45
   Pro Thr Ile Asn Leu Asp Pro Arg Val Ser Ile Tyr Ser Gly Arg Arg
   50 55 60
   Pro Leu Phe Ser Arg Thr Asn Ile Gln Gly Arg Val Tyr Asn Phe Leu
   65 70 75 80
   Glu Arg Pro Thr Gly Trp Lys Cys Phe Val Tyr His Phe Thr Val Phe
   85 90 95
   Leu Ile Val Leu Ile Cys Leu Ile Phe Ser Val Leu Ser Thr Ile Gln
   100 105 110
   Gln Tyr Asn Asn Leu Ala Thr Glu Thr Leu Phe Trp Met Glu Ile Val
   115 120 125
   Leu Val Val Phe Phe Gly Ala Glu Tyr Val Val Arg Leu Trp Ser Ala
   130 135 140
   Gly Cys Arg Ser Lys Tyr Val Gly Val Trp Gly Arg Leu Arg Phe Ala
   145 150 155 160
   Arg Lys Pro Ile Ser Val Ile Asp Leu Ile Val Val Val Ala Ser Val
   165 170 175
   Ile Val Leu Cys Val Gly Ser Asn Gly Gln Val Phe Ala Thr Ser Ala
   180 185 190
   Ile Arg Gly Ile Arg Phe Leu Gln Ile Leu Arg Met Leu His Val Asp
   195 200 205
   Arg Gln Gly Gly Thr Trp Arg Leu Leu Gly Ser Val Val Phe Ile His
   210 215 220
   Arg Gln Glu Leu Ile Thr Thr Leu Tyr Ile Gly Phe Leu Gly Leu Ile
   225                 230                 235                 240
   Phe Ser Ser Tyr Phe Val Tyr Leu Ala Glu Lys Asp Ala Ile Asp Ser
                   245                 250                 255
   Ser Gly Glu Tyr Gln Phe Gly Ser Tyr Ala Asp Ala Leu Trp Trp Gly
               260                 265                 270
   Val Val Thr Val Thr Thr Ile Gly Tyr Gly Asp Lys Val Pro Gln Thr
           275                 280                 285
   Trp Ile Gly Lys Thr Ile Ala Ser Cys Phe Ser Val Phe Ala Ile Ser
       290                 295                 300
   Phe Phe Ala Leu Pro Ala Gly Ile Leu Gly Ser Gly Phe Ala Leu Lys
   305                 310                 315                 320
   Val Gln Gln Lys Gln Arg Gln Lys His Phe Asn Arg Gln Ile Pro Ala
                   325                 330                 335
   Ala Ala Ser Leu Ile Gln Thr Ala Trp Arg Cys Tyr Ala Ala Glu Asn
               340                 345                 350
   Pro Asp Ser Ala Thr Trp Lys Ile Tyr Ile Arg Lys Gln Ser Arg Asn
           355                 360                 365
   His His Ile Met Ser Pro Ser Pro
       370                 375
(2) INFORMATION FOR SEQ ID NO:24:
   (i) SEQUENCE CHARACTERISTICS:
      (A) LENGTH: 581 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (vi) ORIGINAL SOURCE:
      (A) ORGANISM: Homo sapiens
   (xi) SEQUENCE DESCRIPTION: SEQ ID NO:24:
   Met Glu Thr Arg Gly Ser Arg Leu Thr Gly Gly Gln Gly Arg Val Tyr
   1               5                   10                  15
   Asn Phe Leu Glu Arg Pro Thr Gly Trp Lys Cys Phe Val Tyr His Phe
               20                  25                  30
   Ala Val Phe Leu Ile Val Leu Val Cys Leu Ile Phe Ser Val Leu Ser
           35                  40                  45
   Thr Ile Glu Gln Tyr Ala Ala Leu Ala Thr Gly Thr Leu Phe Trp Met
       50                  55                  60
   Glu Ile Val Leu Val Val Phe Phe Gly Thr Glu Tyr Val Val Arg Leu
   65                  70                  75                  80
   Trp Ser Ala Gly Cys Arg Ser Lys Tyr Val Gly Leu Trp Gly Arg Leu
                   85                  90                  95
   Arg Phe Ala Arg Lys Pro Ile Ser Ile Ile Asp Leu Ile Val Val Val
               100                 105                 110
   Ala Ser Met Val Val Leu Cys Val Gly Ser Lys Gly Gln Val Phe Ala
           115                 120                 125
   Thr Ser Ala Ile Arg Gly Ile Arg Phe Leu Gln Ile Leu Arg Met Leu
       130                 135                 140
   His Val Asp Arg Gln Gly Gly Thr Trp Arg Leu Leu Gly Ser Val Val
   145                 150                 155                 160
   Phe Ile His Arg Gln Glu Leu Ile Thr Thr Leu Tyr Ile Gly Phe Leu
                   165                 170                 175
   Gly Leu Ile Phe Ser Ser Tyr Phe Val Tyr Leu Ala Glu Lys Asp Ala
               180                 185                 190
   Val Asn Glu Ser Gly Arg Val Glu Phe Gly Ser Tyr Ala Asp Ala Leu
           195                 200                 205
   Trp Trp Gly Val Val Thr Val Thr Thr Ile Gly Tyr Gly Asp Lys Val
       210                 215                 220
   Pro Gln Thr Trp Val Gly Lys Thr Ile Ala Ser Cys Phe Ser Val Phe
   225                 230                 235                 240
   Ala Ile Ser Phe Phe Ala Leu Pro Ala Gly Ile Leu Gly Ser Gly Phe
                   245                 250                 255
   Ala Leu Lys Val Gln Gln Lys Gln Arg Gln Lys His Phe Asn Arg Gln
               260                 265                 270
   Ile Pro Ala Ala Ala Ser Leu Ile Gln Thr Ala Trp Arg Cys Tyr Ala
           275                 280                 285
   Ala Glu Asn Pro Asp Ser Ser Thr Trp Lys Ile Tyr Ile Arg Lys Ala
       290                 295                 300
   Pro Arg Ser His Thr Leu Leu Ser Pro Ser Pro Lys Pro Lys Lys Ser
   305                 310                 315                 320
   Val Val Val Lys Lys Lys Lys Phe Lys Leu Asp Lys Asp Asn Gly Val
                   325                 330                 335
   Thr Pro Gly Glu Lys Met Leu Thr Val Pro His Ile Thr Cys Asp Pro
               340                 345                 350
   Pro Glu Glu Arg Arg Leu Asp His Phe Ser Val Asp Gly Tyr Asp Ser
           355                 360                 365
   Ser Val Arg Lys Ser Pro Thr Leu Leu Glu Val Ser Met Pro His Phe
       370                 375                 380
   Met Arg Thr Asn Ser Phe Ala Glu Asp Leu Asp Leu Glu Gly Glu Thr
   385                 390                 395                 400
   Leu Leu Thr Pro Ile Thr His Ile Ser Gln Leu Arg Glu His His Arg
                   405                 410                 415
   Ala Thr Ile Lys Val Ile Arg Arg Met Gln Tyr Phe Val Ala Lys Lys
               420                 425                 430
   Lys Phe Gln Gln Ala Arg Lys Pro Tyr Asp Val Arg Asp Val Ile Glu
           435                 440                 445
   Gln Tyr Ser Gln Gly His Leu Asn Leu Met Arg Val Ile Lys Glu Leu
       450                 455                 460
   Gln Arg Arg Leu Asp Gln Ser Ile Gly Lys Pro Ser Leu Phe Ile Ser
   465                 470                 475                 480
   Val Ser Glu Lys Ser Lys Asp Arg Gly Ser Asn Thr Ile Gly Ala Arg
                   485                 490                 495
   Leu Asn Arg Val Glu Asp Lys Val Thr Gln Leu Asp Gln Arg Leu Ala
               500                 505                 510
   Leu Ile Thr Asp Met Leu His Gln Leu Leu Ser Leu His Gly Gly Ser
           515                 520                 525
   Thr Pro Gly Ser Gly Gly Pro Pro Arg Glu Gly Gly Ala His Ile Thr
       530                 535                 540
   Gln Pro Cys Gly Ser Gly Gly Ser Val Asp Pro Glu Leu Phe Leu Pro
   545                 550                 555                 560
   Ser Asn Thr Leu Pro Thr Tyr Glu Gln Leu Thr Val Pro Arg Arg Gly
                   565                 570                 575
   Pro Asp Glu Gly Ser
               580
(2) INFORMATION FOR SEQ ID NO:25:
   (i) SEQUENCE CHARACTERISTICS:
      (A) LENGTH: 2821 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (ix) FEATURE:
      (A) NAME/KEY: CDS
      (B) LOCATION: 88..1830
   (xi) SEQUENCE DESCRIPTION: SEQ ID NO:25:
   GGCTTCCTCG AGCGTCCCAC CGGCTGGAAG TTGTAGACGC GGCCCTGGAC GTGGGTGCGC      60
   GCCAACACCG GGCGGCGCGT GCTGTAG ATG GAG ACG CGC GGG TCT AGG CTC         111
                                 Met Glu Thr Arg Gly Ser Arg Leu
                                   1               5
   ACC GGC GGC CAG GGC CGC GTC TAC AAC TTC CTC GAG CGT CCC ACC GGC       159
   Thr Gly Gly Gln Gly Arg Val Tyr Asn Phe Leu Glu Arg Pro Thr Gly
        10                  15                  20
   TGG AAA TGC TTC GTT TAC CAC TTC GCC GTC TTC CTC ATC GTC CTG GTC       207
   Trp Lys Cys Phe Val Tyr His Phe Ala Val Phe Leu Ile Val Leu Val
    25                  30                  35                  40
   TGC CTC ATC TTC AGC GTG CTG TCC ACC ATC GAG CAG TAT GCC GCC CTG       255
   Cys Leu Ile Phe Ser Val Leu Ser Thr Ile Glu Gln Tyr Ala Ala Leu
                    45                  50                  55
   GCC ACG GGG ACT CTC TTC TGG ATG GAG ATC GTG CTG GTG GTG TTC TTC       303
   Ala Thr Gly Thr Leu Phe Trp Met Glu Ile Val Leu Val Val Phe Phe
                60                  65                  70
   GGG ACG GAG TAC GTG GTC CGC CTC TGG TCC GCC GGC TGC CGC AGC AAG       351
   Gly Thr Glu Tyr Val Val Arg Leu Trp Ser Ala Gly Cys Arg Ser Lys
            75                  80                  85
   TAC GTG GGC CTC TGG GGG CGG CTG CGC TTT GCC CGG AAG CCC ATT TCC       399
   Tyr Val Gly Leu Trp Gly Arg Leu Arg Phe Ala Arg Lys Pro Ile Ser
        90                  95                 100
   ATC ATC GAC CTC ATC GTG GTC GTG GCC TCC ATG GTG GTC CTC TGC GTG       447
   Ile Ile Asp Leu Ile Val Val Val Ala Ser Met Val Val Leu Cys Val
   105                 110                 115                 120
   GGC TCC AAG GGG CAG GTG TTT GCC ACG TCG GCC ATC AGG GGC ATC CGC       495
   Gly Ser Lys Gly Gln Val Phe Ala Thr Ser Ala Ile Arg Gly Ile Arg
                   125                 130                 135
   TTC CTG CAG ATC CTG AGG ATG CTA CAC GTC GAC CGC CAG GGA GGC ACC       543
   Phe Leu Gln Ile Leu Arg Met Leu His Val Asp Arg Gln Gly Gly Thr
               140                 145                 150
   TGG AGG CTC CTG GGC TCC GTG GTC TTC ATC CAC CGC CAG GAG CTG ATA       591
   Trp Arg Leu Leu Gly Ser Val Val Phe Ile His Arg Gln Glu Leu Ile
           155                 160                 165
   ACC ACC CTG TAC ATC GGC TTC CTG GGC CTC ATC TTC TCC TCG TAC TTT       639
   Thr Thr Leu Tyr Ile Gly Phe Leu Gly Leu Ile Phe Ser Ser Tyr Phe
       170                 175                 180
   GTG TAC CTG GCT GAG AAG GAC GCG GTG AAC GAG TCA GGC CGC GTG GAG       687
   Val Tyr Leu Ala Glu Lys Asp Ala Val Asn Glu Ser Gly Arg Val Glu
   185                 190                 195                 200
   TTC GGC AGC TAC GCA GAT GCG CTG TGG TGG GGG GTG GTC ACA GTC ACC      735
   Phe Gly Ser Tyr Ala Asp Ala Leu Trp Trp Gly Val Val Thr Val Thr
                   205                 210                 215
   ACC ATC GGC TAT GGG GAC AAG GTG CCC CAG ACG TGG GTC GGG AAG ACC      783
   Thr Ile Gly Tyr Gly Asp Lys Val Pro Gln Thr Trp Val Gly Lys Thr
               220                 225                 230
   ATC GCC TCC TGC TTC TCT GTC TTT GCC ATC TCC TTC TTT GCG CTC CCA      831
   Ile Ala Ser Cys Phe Ser Val Phe Ala Ile Ser Phe Phe Ala Leu Pro
           235                 240                 245
   GCG GGG ATT CTT GGC TCG GGG TTT GCC CTG AAG GTG CAG CAG AAG CAG      879
   Ala Gly Ile Leu Gly Ser Gly Phe Ala Leu Lys Val Gln Gln Lys Gln
       250                 255                 260
   AGG CAG AAG CAC TTC AAC CGG CAG ATC CCG GCG GCA GCC TCA CTC ATT      927
   Arg Gln Lys His Phe Asn Arg Gln Ile Pro Ala Ala Ala Ser Leu Ile
   265                 270                 275                 280
   CAG ACC GCA TGG AGG TGC TAT GCT GCC GAG AAC CCC GAC TCC TCC ACC      975
   Gln Thr Ala Trp Arg Cys Tyr Ala Ala Glu Asn Pro Asp Ser Ser Thr
                   285                 290                 295
   TGG AAG ATC TAC ATC CGG AAG GCC CCC CGG AGC CAC ACT CTG CTG TCA     1023
   Trp Lys Ile Tyr Ile Arg Lys Ala Pro Arg Ser His Thr Leu Leu Ser
               300                 305                 310
   CCC AGC CCC AAA CCC AAG AAG TCT GTG GTG GTA AAG AAA AAA AAG TTC     1071
   Pro Ser Pro Lys Pro Lys Lys Ser Val Val Val Lys Lys Lys Lys Phe
           315                 320                 325
   AAG CTG GAC AAA GAC AAT GGG GTG ACT CCT GGA GAG AAG ATG CTC ACA     1119
   Lys Leu Asp Lys Asp Asn Gly Val Thr Pro Gly Glu Lys Met Leu Thr
       330                 335                 340
   GTC CCC CAT ATC ACG TGC GAC CCC CCA GAA GAG CGG CGG CTG GAC CAC     1167
   Val Pro His Ile Thr Cys Asp Pro Pro Glu Glu Arg Arg Leu Asp His
   345                 350                 355                 360
   TTC TCT GTC GAC GGC TAT GAC AGT TCT GTA AGG AAG AGC CCA ACA CTG     1215
   Phe Ser Val Asp Gly Tyr Asp Ser Ser Val Arg Lys Ser Pro Thr Leu
                   365                 370                 375
   CTG GAA GTG AGC ATG CCC CAT TTC ATG AGA ACC AAC AGC TTC GCC GAG     1263
   Leu Glu Val Ser Met Pro His Phe Met Arg Thr Asn Ser Phe Ala Glu
               380                 385                 390
   GAC CTG GAC CTG GAA GGG GAG ACT CTG CTG ACA CCC ATC ACC CAC ATC     1311
   Asp Leu Asp Leu Glu Gly Glu Thr Leu Leu Thr Pro Ile Thr His Ile
           395                 400                 405
   TCA CAG CTG CGG GAA CAC CAT CGG GCC ACC ATT AAG GTC ATT CGA CGC     1359
   Ser Gln Leu Arg Glu His His Arg Ala Thr Ile Lys Val Ile Arg Arg
       410                 415                 420
   ATG CAG TAC TTT GTG GCC AAG AAG AAA TTC CAG CAA GCG CGG AAG CCT     1407
   Met Gln Tyr Phe Val Ala Lys Lys Lys Phe Gln Gln Ala Arg Lys Pro
   425                 430                 435                 440
   TAC GAT GTG CGG GAC GTC ATT GAG CAG TAC TCG CAG GGC CAC CTC AAC      1455
   Tyr Asp Val Arg Asp Val Ile Glu Gln Tyr Ser Gln Gly His Leu Asn
                   445                 450                 455
   CTC ATG GTG CGC ATC AAG GAG CTG CAG AGG AGG CTG GAC CAG TCC ATT      1503
   Leu Met Val Arg Ile Lys Glu Leu Gln Arg Arg Leu Asp Gln Ser Ile
               460                 465                 470
   GGG AAG CCC TCA CTG TTC ATC TCC GTC TCA GAA AAG AGC AAG GAT CGC      1551
   Gly Lys Pro Ser Leu Phe Ile Ser Val Ser Glu Lys Ser Lys Asp Arg
           475                 480                 485
   GGC AGC AAC ACG ATC GGC GCC CGC CTG AAC CGA GTA GAA GAC AAG GTG      1599
   Gly Ser Asn Thr Ile Gly Ala Arg Leu Asn Arg Val Glu Asp Lys Val
       490                 495                 500
   ACG CAG CTG GAC CAG AGG CTG GCA CTC ATC ACC GAC ATG CTT CAC CAG      1647
   Thr Gln Leu Asp Gln Arg Leu Ala Leu Ile Thr Asp Met Leu His Gln
   505                 510                 515                 520
   CTG CTC TCC TTG CAC GGT GGC AGC ACC CCC GGC AGC GGC GGC CCC CCC      1695
   Leu Leu Ser Leu His Gly Gly Ser Thr Pro Gly Ser Gly Gly Pro Pro
                   525                 530                 535
   AGA GAG GGC GGG GCC CAC ATC ACC CAG CCC TGC GGC AGT GGC GGC TCC      1743
   Arg Glu Gly Gly Ala His Ile Thr Gln Pro Cys Gly Ser Gly Gly Ser
               540                 545                 550
   GTC GAC CCT GAG CTC TTC CTG CCC AGC AAC ACC CTG CCC ACC TAC GAG      1791
   Val Asp Pro Glu Leu Phe Leu Pro Ser Asn Thr Leu Pro Thr Tyr Glu
           555                 560                 565
   CAG CTG ACC GTG CCC AGG AGG GGC CCC GAT GAG GGG TCC TGAGGAGGGG       1840
   Gln Leu Thr Val Pro Arg Arg Gly Pro Asp Glu Gly Ser
       570                 575                 580
   ATGGGGCTGG GGGATGGGCC TGAGTGAGAG GGGAGGCCAA GAGTGGCCCC ACCTGGCCCT    1900
   CTCTGAAGGA GGCCACCTCC TAAAAGGCCC AGAGAGAAGA GCCCCACTCT CAGAGGCCCC    1960
   AATACCCCAT GGACCATGCT GTCTGGCACA GCCTGCACTT GGGGGCTCAG CAAGGCCACC    2020
   TCTTCCTGGC CGGTGTGGGG GCCCCGTCTC AGGTCTGAGT TGTTACCCCA AGCGCCCTGG    2080
   CCCCCACATG GTGATGTTGA CATCACTGGC ATGGTGGTTG GGACCCAGTG GCAGGGCACA    2140
   GGGCCTGGCC CATGTATGGC CAGGAAGTAG CACAGGCTGA GTGCAGGCCC ACCCTGCTTG    2200
   GCCCAGGGGG CTTCCTGAGG GGAGACAGAG CAACCCCTGG ACCCCAGCCT CAAATCCAGG    2260
   ACCCTGCCAG GCACAGGCAG GGCAGGACCA GCCCACGCTG ACTACAGGGC CACCGGCAAT    2320
   AAAAGCCCAG GAGCCCATTT GGAGGGCCTG GGCCTGGCTC CCTCACTCTC AGGAAATGCT    2380
   GACCCATGGG CAGGAGACTG TGGAGACTGC TCCTGAGCCC CCAGCTTCCA GCAGGAGGGA    2440
   CAGTCTCACC ATTTCCCCAG GGCACGTGGT TGAGTGGGGG GAACGCCCAC TTCCCTGGGT    2500
   TAGACTGCCA GCTCTTCCTA GCTGGAGAGG AGCCCTGCCT CTCCGCCCCT GAGCCCACTG    2560
   TGCGTGGGGC TCCCGCCTCC AACCCCTCGC CCAGTCCCAG CAGCCAGCCA AACACACAGA 2620
   AGGGGACTGC CACCTCCCCT TGCCAGCTGC TGAGCCGCAG AGAAGTGACG GTTCCTACAC 2680
   AGGACAGGGG TTCCTTCTGG GCATTACATC GCATAGAAAT CAATAATTTG TGGTGATTTG 2740
   GATCTGTGTT TTAATGAGTT TCACAGTGTG ATTTTGATTA TTAATTGTGC AAGCTTTTCC 2800
   TAATAAACGT GGAGAATCAC A 2821
(2) INFORMATION FOR SEQ ID NO:26:
   (i) SEQUENCE CHARACTERISTICS:
      (A) LENGTH: 581 amino acids
      (B) TYPE: amino acid
      (D) TOPOLOGY: linear
   (ii) MOLECULE TYPE: protein
   (xi) SEQUENCE DESCRIPTION: SEQ ID NO:26:
   Met Glu Thr Arg Gly Ser Arg Leu Thr Gly Gly Gln Gly Arg Val Tyr
     1               5                  10                  15
   Asn Phe Leu Glu Arg Pro Thr Gly Trp Lys Cys Phe Val Tyr His Phe
                20                  25                  30
   Ala Val Phe Leu Ile Val Leu Val Cys Leu Ile Phe Ser Val Leu Ser
            35                  40                  45
   Thr Ile Glu Gln Tyr Ala Ala Leu Ala Thr Gly Thr Leu Phe Trp Met
        50                  55                  60
   Glu Ile Val Leu Val Val Phe Phe Gly Thr Glu Tyr Val Val Arg Leu
    65                  70                  75                  80
   Trp Ser Ala Gly Cys Arg Ser Lys Tyr Val Gly Leu Trp Gly Arg Leu
                    85                  90                  95
   Arg Phe Ala Arg Lys Pro Ile Ser Ile Ile Asp Leu Ile Val Val Val
               100                 105                 110
   Ala Ser Met Val Val Leu Cys Val Gly Ser Lys Gly Gln Val Phe Ala
           115                 120                 125
   Thr Ser Ala Ile Arg Gly Ile Arg Phe Leu Gln Ile Leu Arg Met Leu
       130                 135                 140
   His Val Asp Arg Gln Gly Gly Thr Trp Arg Leu Leu Gly Ser Val Val
   145                 150                 155                 160
   Phe Ile His Arg Gln Glu Leu Ile Thr Thr Leu Tyr Ile Gly Phe Leu
                   165                 170                 175
   Gly Leu Ile Phe Ser Ser Tyr Phe Val Tyr Leu Ala Glu Lys Asp Ala
               180                 185                 190
   Val Asn Glu Ser Gly Arg Val Glu Phe Gly Ser Tyr Ala Asp Ala Leu
   195 200 205
   Trp Trp Gly Val Val Thr Val Thr Thr Ile Gly Tyr Gly Asp Lys Val
   210 215 220
   Pro Gln Thr Trp Val Gly Lys Thr Ile Ala Ser Cys Phe Ser Val Phe
   225 230 235 240
   Ala Ile Ser Phe Phe Ala Leu Pro Ala Gly Ile Leu Gly Ser Gly Phe
   245 250 255
   Ala Leu Lys Val Gln Gln Lys Gln Arg Gln Lys His Phe Asn Arg Gln
   260 265 270
   Ile Pro Ala Ala Ala Ser Leu Ile Gln Thr Ala Trp Arg Cys Tyr Ala
   275 280 285
   Ala Glu Asn Pro Asp Ser Ser Thr Trp Lys Ile Tyr Ile Arg Lys Ala
   290 295 300
   Pro Arg Ser His Thr Leu Leu Ser Pro Ser Pro Lys Pro Lys Lys Ser
   305 310 315 320
   Val Val Val Lys Lys Lys Lys Phe Lys Leu Asp Lys Asp Asn Gly Val
   325 330 335
   Thr Pro Gly Glu Lys Met Leu Thr Val Pro His Ile Thr Cys Asp Pro
   340 345 350
   Pro Glu Glu Arg Arg Leu Asp His Phe Ser Val Asp Gly Tyr Asp Ser
   355 360 365
   Ser Val Arg Lys Ser Pro Thr Leu Leu Glu Val Ser Met Pro His Phe
   370 375 380
   Met Arg Thr Asn Ser Phe Ala Glu Asp Leu Asp Leu Glu Gly Glu Thr
   385 390 395 400
   Leu Leu Thr Pro Ile Thr His Ile Ser Gln Leu Arg Glu His His Arg
   405 410 415
   Ala Thr Ile Lys Val Ile Arg Arg Met Gln Tyr Phe Val Ala Lys Lys
   420 425 430
   Lys Phe Gln Gln Ala Arg Lys Pro Tyr Asp Val Arg Asp Val Ile Glu
   435 440 445
   Gln Tyr Ser Gln Gly His Leu Asn Leu Met Val Arg Ile Lys Glu Leu
   450 455 460
   Gln Arg Arg Leu Asp Gln Ser Ile Gly Lys Pro Ser Leu Phe Ile Ser
   465 470 475 480
   Val Ser Glu Lys Ser Lys Asp Arg Gly Ser Asn Thr Ile Gly Ala Arg
   485 490 495
   Leu Asn Arg Val Glu Asp Lys Val Thr Gln Leu Asp Gln Arg Leu Ala
   500 505 510
   Leu Ile Thr Asp Met Leu His Gln Leu Leu Ser Leu His Gly Gly Ser
           515                 520                 525
   Thr Pro Gly Ser Gly Gly Pro Pro Arg Glu Gly Gly Ala His Ile Thr
       530                 535                 540
   Gln Pro Cys Gly Ser Gly Gly Ser Val Asp Pro Glu Leu Phe Leu Pro
   545                 550                 555                 560
   Ser Asn Thr Leu Pro Thr Tyr Glu Gln Leu Thr Val Pro Arg Arg Gly
                   565                 570                 575
   Pro Asp Glu Gly Ser
               580